(19) Europäisches Patentamt
European Patent Office
Office européen des brevets

(11) **EP 4 613 279 A1**

(12) **EUROPEAN PATENT APPLICATION**
published in accordance with Art. 153(4) EPC

(43) Date of publication:
**10.09.2025 Bulletin 2025/37**

(21) Application number: **23885155.4**

(22) Date of filing: **14.08.2023**

(51) International Patent Classification (IPC):
**A61K 36/00** (2006.01)    **A23L 33/00** (2016.01)

(52) Cooperative Patent Classification (CPC):
**A23L 33/00; A61K 36/00**

(86) International application number:
**PCT/CU2023/050003**

(87) International publication number:
**WO 2024/094232 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 CU 20220063**

(71) Applicant: **Centro Nacional de Biopreparados Bejucal 32600 (CU)**

(72) Inventors:
• **RODRÍGUEZ MARTÍNEZ, Claudio**
  **Bejucal 32600 (CU)**
• **ESQUIVEL CRESPO, Nashelly**
  **La Habana 32600 (CU)**
• **GARCÍA HERNÁNDEZ, Yenela**
  **Quivicán 33500 (CU)**
• **GARCÍA MARICHAL, José Miguel**
  **Bejucal 32600 (CU)**
• **LÓPEZ RICARDO, Yudisleidy**
  **Bejucal 32600 (CU)**
• **RÁBAGO CAPIRO, Rachel**
  **La Habana 11500 (CU)**
• **VIERA ORAMAS, Diana Rosa**
  **Bejucal 32600 (CU)**
• **VALIENTE CHÁVEZ, Yubel**
  **San Antonio de los Baños 32500 (CU)**

(74) Representative: **Capitán García, Maria Nuria**
**ARS Privilegium, S.L.**
**Felipe IV no. 10, bajo iz.**
**28014 Madrid (ES)**

(54) **NUTRACEUTICAL TO PREVENT AND TREAT COGNITIVE IMPAIRMENT**

(57)    The present invention consists of a solid nutraceutical composition for preventing and treating cognitive impairment, composed of a mixture of an emulsion and a mass of seeds and oil whose components are of natural origin. The emulsion contains *Plukenetia volubilis* oil, extracts of vegetable origin such as *Curcuma longa, Piper nigrum* or *Theobroma cacao,* emulsifiers selected among lecithin, *Saponaria* or *Agavaceae* extract, or egg white, edulcorants selected among fructooligosaccharide, *Stevia rebaudiana* or *Agavaceae* extract, *Apis mellifera,* sucrose, and also contains air. The mass of seeds and oil includes *Cucurbita* seeds and *Plukenetia volubilis* oil. Other components selected from *Theobroma cocoa* powder, *Cocos nucifera* oil, fruit or seed mass, natural juices and proteins are added. Thus, the nutraceutical is composed of 3 to 5 sources of fatty acids, phytosterols and triacylglycerols, 2 to 8 sources of antioxidants, up to 3 sources of hypoglycemic agents, up to 4 sources of anticholesterol and anti-inflammatory compounds. It also contains proteins, vitamins, macro- and microelements, and substances that enable easy chewing and swallowing suitable for subjects and patients suffering from cognitive deficits. The nutraceutical is appropriate for the prevention and treatment of Alzheimer's disease, mental, behavioral and developmental disorders, including depressive disorders and disorders of the nervous, digestive, immune, endocrine and circulatory systems, cardiovascular, cerebrovascular, infectious, parasitic, oncological and blood diseases, tiredness, fatigue and malnutrition.

EP 4 613 279 A1

**Description**

## DESCRIPTION

The invention relates to the field of natural products directed to exert some beneficial physiological effect on the human organism, said beneficial effect being preventive to avoid the occurrence of a disease or disorder or as an adjuvant in the treatment of a disease or disorder or for its treatment. More specifically, the invention relates, although not in a limited manner, to the prevention or treatment of cognitive deficits and neurological disorders and in particular to Alzheimer's Disease.

Cognitive impairment is a characteristic phenomenon of aging as a natural process. Cognitive impairment is also associated with neurological diseases and disorders and is considered to be caused by vascular accidents and high consumption of alcohol and drugs, among other factors. Cognitive impairment is present in Parkinson's disease, Alzheimer's disease (C. Patterson, MD, FRCPC. Cognitive Impairment in the Elderly: Case Finding in Family Practice. Can. Fam. Physician. 1989 Mar; 35: 621-624) and in other diseases.

The onset of cognitive impairment and Alzheimer's disease has been associated with other causes of different nature: advanced age, family history pointing to genetic factors, metabolic disorders, cranial trauma, pathological changes in the brain, disorders in the cholinergic system and in the noradrenergic and serotonergic neurotransmitter systems (Larson EB, Kukull WA, Katzman RL. Cognitive impairment: dementia and Alzheimer's disease. Annu Rev Public Health. 1992;13:431-49; Larson EB, Kukull WA, Katzman RL. Alzheimer's Disease and Mild Cognitive Impairment. Neurol. Clin. 2007 August; 25(3): 577-v).

Although different hypotheses have been proposed to help understand its complex neurodegenerative process, the causes that promote its development are still unknown (Haass, C.; Kaether, C.; Thinakaran, G. S. (2012). Sisodia Trafficking and proteolytic processing of APP. Cold Spring Harb Perspect Med., 2 p.; Mucke, L.; SelkoeD.J. (2012). Neurotoxicity of amyloid β-protein: Synaptic and network dysfunction. Cold Spring Harb Perspect Med., 2 p.). Most experts agree that Alzheimer's Disease develops as a result of the combination of multiple modifiable and non-modifiable risk factors (age, gender, family and genetic history, environment and, in addition, lifestyle) rather than a single cause (Castello, M.A.; Soriano, S. (2014). On the origin of Alzheimer's disease. Trials and tribulations of the amyloid hypothesis. Ageing Res Rev, 13 pp. 10-12. On the origin of Alzheimer's disease. Trials and tribulations of the amyloid hypothesis. Ageing Res Rev, 13 pp. 10-12; Drachman, D. A. (2014). The amyloid hypothesis, time to move on: Amyloid is the downstream result, not cause, of Alzheimer's disease. Alzheimers Dement, 10 pp. 372-380).

Currently, the two proposed etiological hypotheses most widely accepted by the scientific community are tau protein hyperphosphorylation and the amyloid cascade. In a neuropathological situation, the amyloid precursor protein (APP) is metabolized by the amyloidogenic pathway where the enzyme β-secretase produces a soluble N-terminal fragment, known as APPSβ; furthermore, such cleavage produces the C99 fragment. Subsequently, the γ-secretase enzyme cleaves the fragment at the C-terminal end, giving rise to the Aβ40/42 fragments. The Aβ42 peptide is both more aggregation prone and neurotoxic than Aβ40 and has been hypothesized to represent pathogenic Aβ species. Thus, Aβ42 oligomerizes and accumulates in the form of senile plaques in the limbic system and associative cortex, thereby exerting toxic effects on neuronal synapses (Hong-Qi, Y.; Zhi-Kun, S.; Sheng-Di, C. (2012). Current advances in the treatment of Alzheimer's disease: focused on considerations targeting Aβ and tau. Trans. Neurodeg.; 1: 1-21; Selkoe, D. J.; Hardy, J. (2016). The amyloid hypothesis of Alzheimer's disease at 25 years. EMBO molecular medicine 8: 595-608).

The toxic effect of senile plaques triggers a glial response by activating astrocytes and surrounding microglia. This action causes the release of cytokines or components of the complement system, leading to inflammatory responses. Oxidative stress is also induced in the neuron, which causes an alteration in calcium ion homeostasis by hyperactivation of protein kinases and inactivation of phosphatases. For this reason, the tau protein is hyperphosphorylated and forms neurofibrillary tangles, which accumulate in the synapses and neuronal bodies, causing neuronal death by apoptosis and a deficit of neurotransmitters. This cascade of processes leads to the onset of dementia. (Selkoe, D. J.; Hardy, J. (2016). The amyloid hypothesis of Alzheimer's disease at 25 years. EMBO molecular medicine 8: 595-608).

One theory that has become very popular is related to disorders at the metabolic level, specifically those linked to insulin resistance at the cerebral level. This pathology is characterized by a diminished capacity of insulin to carry out its normal physiological functions. (Serrano M.; Cascales, M. (2015). Resistencia a la insulina, inflamación y obesidad, 1:377-399). It has been observed that there are receptors for this hormone mainly at the level of the hippocampus, which when activated allow the utilization of glucose by neurons. It seems that a series of enzymes are activated that have a detoxifying effect related to the toxic action of amyloid plaques, which has been observed only in animal models (Jagua, A.; Avila, A. (2007). Insulina y enfermedad de Alzheimer: ¿una diabetes tipo 3?. Rev Med Univ Nac Colomb. 55(1): p. 6-70.).

Therefore, a therapeutic option could be to incorporate into the diet some nutrient that leads to obtaining alternative energy through a metabolic pathway other than glycolysis, to counteract neuronal death due to lack of glucose and improve the patient at any stage of the disease. In this context, ketone bodies, which are produced from medium chain triglycerides (MCTG), constitute an alternative source of energy in the absence of glucose for the cells. (Hu, I.; Ortí, J. E. R.; Sabater, P.

S.; Castillo S. S.; Rochina, M. J. (2015). Aceite de coco: tratamiento alternativo no farmacológico frente a la enfermedad de Alzheimer. Universidad Católica de Valencia. España. Nutr Hosp. 32(6):2822-2827 ISSN 0212-1611).

Some of the alternative hypotheses currently proposed are: altered mitochondrial activity and neuroinflammation (Felice F. D., Lourenco M, Ferreira S. (2014). How does brain insulin resistance develop in Alzheimer's disease? Alzheimers Dement 10:S26-32). Finally, the dendritic hypothesis of Alzheimer's disease has been proposed. All this confirms the complexity of this disease, in addition to the fact that the mechanism of neuronal death by apoptosis is not yet fully understood. (Boada, M.; Ramos-Fernández, E.; Guivernau, B.; Muñoz, F. J.; Costa, M.; Ortiz, A. M. (2016). Tratamiento de la enfermedad de Alzheimer mediante terapia combinada de aféresis terapéutica y hemoféresis con albúmina e inmunoglobulina intravenosa: fundamentos y aproximación terapéutica al estudio AMBAR (Alzheimer Management by Albumin Replacement). Sociedad Española de Neurología. 31(7): 473-481).

Pereoxidation reactions cause impairment of mitochondrial functions and can lead to cell death. Impairment due to this cause is considered one of the most common in the onset of cognitive impairment. (Warren E.B., Morrow E.M. Mitochondrial Function in 22q11 Deletion Syndrome. Neuron. Volume 102, Issue 6, 19 June 2019, 1089-1091; Jara C., Torres A.K., Olesen M.A. Tapia-Rojas C. Mitochondrial Dysfunction as a Key Event during Aging: From Synaptic Failure to Memory Loss. Consultado el 28 FEB 202 en https://www.intechopen.com/predownload/68488; Mancuso M., Calsolaro V., Orsucci D., Carlesi C., Choub A., Piazza S., and Siciliano G. Mitochondria, Cognitive Impairment, and Alzheimer's Disease. Int J Alzheimers Dis. 2009; 2009: 951548. Published online 2009 Jul 6).

On the other hand, the influence of medium-chain fatty acids in inflammatory processes in general and especially in neuroinflammation has been described.

In subjects with Alzheimer's disease the neurons of the brain are unable to metabolize glucose due to insulin resistance located at the level of the brain and not in the rest of the organism. This is why the body does not synthesize the ketone bodies necessary to provide the alternative energy required by the neurons to survive. Ketone bodies can originate from medium-chain triglycerides that are easily ionized at the pH of human blood, facilitating their greater solubility and better emulsification with smaller droplets that are more easily processed by lipases than those of larger chains. All this leads, in addition, to their better oxidation in the liver and therefore to the greater formation of ketone bodies. It is proposed that this process ultimately leads to improved blood flow and cognitive improvement in normal subjects (Hasselbalch S. G., Madsen P. L., Hageman L. P., Olsen K. S., Justesen N., Holm S., Paulson O. B. Changes in cerebral blood flow and carbohydrate metabolism during acute hyperketonemia. American Journal of Physiology-Endocrinology and Metabolism, Vol. 270, No. 5. ; Veneman T., Mitrakou A., Cryer P, Gerich J. Effect of Hyperketonemia and Hyperlacticacidemia on Symptoms, Cognitive Dysfunction, and Counterregulatory Hormone Responses During Hypoglycemia in Normal Humans. Diabetes 1994 Nov; 43(11): 1311-1317.

According to Sloan E., the components of nutraceuticals include fatty acids, vitamins, minerals, antioxidants and other substances with biological activity on the human organism, which are contained in foods that can influence the physiology of the brain and the state of neurons and their vitality. Sloan affirms that some of these substances intervene in the synthesis of proteins, carbohydrates and lipids that form the structures of the brain and in the synthesis of neurotransmitters and guarantee the synapse and neuronal functions, making possible the normal cognitive function (Sloan, E. The top 10 trends to watch and work on. Food Technol. 50(7): p. 55-71, 1996.

U.S. Patent No. 6,914,071 describes a diet and method that inhibits the onset of mental capacity deterioration in companion pets. The diet comprises an anti-oxidant or a mixture of antioxidants. The invention shows no applicability to humans.

US Patent No. 6426362 details a composition that ameliorates the effect of stress on energy metabolism through the use of tocopherol and/or its derivatives and other compounds such as flavonoids and lactoferrin or derivatives of these compounds. The compounds contained in these substances are not described in depth, nor is the neuroprotective role.

The authors of patent application DE 4303214A1 claim the use of a wide range of saponins and sapogenins for viral diseases that have no proven relationship with neurological diseases or cognitive impairment.

The authors of patent ES2209539T3 from Spain disclose the use of plant extracts with anti-radical action and cosmetic or dermopharmaceutical composition containing such extracts. Among the vegetable raw material described is *Clidemia hirta.* The invention does not claim any action on cognitive impairment or its prevention or as a palliative. Nor are the mechanisms of action applicable to an indication for such purposes.

In U.S. Invention No. 5,985,936 the authors claim a method for preventing or delaying the onset of Alzheimer's Disease comprising a composition of phytosterols (beta-sitosterol, campesterol and stigmastarol) that possess antioxidant activity, protecting the brain from oxidative stress. However, the invention does not reverse any of the other causes attributed to the onset of the disease.

Invention application No 20170065660, also from the USA, shows the conformation of a liquid composition with alkaline pH by combining orange and vanilla extract, honey, agave syrup, apple cider vinegar, olive and coconut oil, multivitamins, *Aloe vera* juice, among other components, and is aimed at supplying energy and reducing inflammation. This invention does not describe the use of substances with considerable antioxidant potential or that act on other mechanisms that trigger cognitive impairment and Alzheimer's disease.

The use of medium chain triglycerides for the treatment and prevention of Alzheimer's disease is described in Spanish patent 2 323 940. The product is presented in the form of a food bar in which medium chain fatty acids are emulsified with L-carnitine, glycerin, corn syrup solids, cocoa and coating. It comprises orally administering a single dose of medium chain triglyceride to a patient such that the blood level of D-beta-hydroxybutyrate in said patient rises to 1-10 mM, or the urinary excretion of D-beta-hydroxybutyrate is in the range of 5 mg/dl to 160 mg/dl, causing hyperketonemia in the patient, which results in the utilization of ketone bodies as an energy source in the brain in the presence of glucose. The invention has as a limitation, among others, that it is necessary the measurement of parameters that the patient or subject must perform in specialized laboratories. As in previously described solutions, it does not correct or prevent the deficit in the metabolism of other substances that cause cognitive impairment, it does not show antioxidant, hypocholesterolemic effect, nor triglyceride lowering capacity. Nor does it show any effect on the intestinal microbiota.

Other patents describe the use of supplements containing vitamins, carotenoids, omega-3 fatty acids and other nutrients, which theoretically possess antioxidant capacity and supplement energy for the brain, but none of them demonstrate beneficial effects on the Nervous System, the brain or its functions and even less on cognitive impairment and Alzheimer's Disease (U.S. Patent Nos. 6,579,544 and 6,140,304).

Curcumin is used in several inventions for the prevention and treatment of neurological diseases. Thus, U.S. Patent No. 5834044 describes a method of obtaining a food consisting of a curcumin-containing turmeric extract, which is combined with a lipid phase of medium-chain or long-chain saturated fatty acids. They are also combined with vitamin B. The structure of the product presents difficulties for assimilation by older adults, since it has a coating and its structure is not homogeneous. In addition, the coating is necessary to avoid the degradation of several compounds, since it has no natural substances to prevent such degradation. It also does not prevent the appearance of undesirable or unacceptable colors, so coating is required. The product is effective only if combined with physical exercise. Finally, no beneficial activity for cognitive impairment or Alzheimer's Disease is described.

On the other hand, the poor bioavailability of curcuminoids is well known, of which only micrograms are absorbed when consuming tens of grams of these compounds daily in the form of extracts or the powdered rhizome. Patent WO2019018445 describes the use of Cathelicidin together with curcumin dissolved in coconut oil as components of a drug. It also describes the use of the fatty acids that make up the oil, specifically docosahexaenoic, caprylic, capric and lauric acids. The method has the disadvantage that it is necessary to previously determine the concentration of Cathelicidin LL-37 and β-amyloid peptide in the subject's tissues, which, in addition to representing an invasive action on the organism, is not suitable for prevention in subjects with normal levels of both parameters. The disadvantage of the low bioavailability of curcumin and other curcuminoids in this form of consumption is also observed.

An example of the use of plant-derived compounds for prevention in neurological diseases appears in the US patent application 20180325842 A1. A tablet is formed from a turmeric extract that is combined with mannitol in a ratio of 1:1 to 1:3. A system with a particle size of 100 to 250 nm is formed. Soy lecithin is employed as a lipid phase to solubilize the extract and increase bioavailability. It is mixed with other components, including piperine, silitol and *Stevia* extract. The tablet contains very little curcuminoids, insufficient to achieve an effective effect. Lecithin is not used as a foaming agent or emulsifier, only as a solubilizer. Tablets are not the most suitable means of supplying substances to elderly people and even less to those who have difficulty feeding themselves independently, as in the case of those suffering from advanced neurological diseases.

U.S. Patent No. 4451488 describes an intermediate moisture food in bar form with easy chewability and high stability that combines at least two purified alcohols (sorbitol and glycerol). Its components include coconut, cocoa and antioxidant. However, the paper does not present any beneficial effect on cognitive status, neurological health or general human health status. The nature of the antioxidant is not described.

International application WO 2006/053379 A1 discloses a series of compositions of plant origin containing fatty acids, carotenoids and other compounds that may prevent Alzheimer's disease or produce therapeutic effects. The source of fatty acids is palm oil. The forms of presentation do not include solid forms that are appropriate for elderly people with difficulties in assimilating nutrients. The forms include liquids, tablets, among others, and it is not described how these forms enable a high bioavailability of its components. The therapeutic or preventive effect of Alzheimer's Disease in animal models or in humans is not demonstrated either.

Patent application WO 2006/121985 A1 "Antioxidant food composition and methods and uses thereof" describes a composition useful for slowing or preventing cognitive decline, using it alone or in combination with physical exercise or cognitive training. The food, among other forms, is a bar and contains at least one antioxidant in its original matrix and at least one ingredient in its processed form. Vitamin E is combined with at least one complex phyto-antioxidant in its natural matrix and optionally with other key nutrients useful for neuroprotection in synthetic or natural form. Other compounds may be added, such as omega-3 fatty acids, phosphatidyl serine and indole-3 propionic acid. The composition is combined with physical exercises for the prevention of cognitive decline and Alzheimer's Disease. This invention can be considered as the closest solution to the present invention.

The invention does not provide or describe a food that complies with organoleptic characteristics (chewiness, ease of swallowing, hardness) that meet the needs of elderly people (who are the most common sufferers of cognitive disorders and especially Alzheimer's disease) such as easy chewability and swallowability. Nor does it include components that may affect other mechanisms that trigger the disease, since it is based on the use of antioxidants and omega 3 fatty acids. It does not foresee a marked anti-inflammatory effect, which is both a cause and a consequence of Alzheimer's disease. It also provides for the use of synthetically obtained substances. The product does not intervene in triggering mechanisms of Alzheimer's disease and other cognitive disorders and Central Nervous System, such as anti-aggregation activity of beta-amyloid peptide, anti-degradation of nurofibrillary tangles, regulation of arterial pressure or protective of cardiovascular function, nor does it possess angiogenic or activating activity of neuronal synapses. Neither does it have antibacterial or vermifuge activity.

So far there is no drug, nutraceutical, medicinal food or effective food to prevent, stop, eliminate or reverse cognitive impairment or associated neurodegenerative diseases or complex metabolic disorders.

By way of summary it can be stated that, so far, no food, supplement, nutraceutical or drug, groups in its composition substances or compounds that possess in unison all the activities against cognitive deficit, Alzheimer's Disease or other diseases and neuronal disorders and in general of the Central Nervous System, such as: antioxidant, anti-inflammatory, antihypertensive, contributor of medium chain fatty acids and omega 3, 6, 9 for the formation of ketone bodies, antimicrobial, antiviral, antitoxic, promoter of neuronal synapse, hypoglycemic, cholesterol-lowering, anti-platelet aggregation, anti-formation of beta-amyloid peptide and anti-neurofibrillary tangle formation and cardiovascular protection, as well as provider of fatty acids of different composition, vitamins, minerals, proteins, carbohydrates and fibers.

From the state of the art it can be summarized that, up to now, the solutions described present other deficiencies such as:

- the emulsions fail to completely mask the undesirable color or flavor of plant-derived components, so that flavors and colorants, in many cases artificial ones, have to be introduced;
- in general, the greatest bioavailability of the components of nutraceuticals and supplements is not achieved, since the total solubility in the interface is not reached, phase separation occurs and a fast and complete absorption in the organism is not achieved, since it depends on the lipolysis process;
- in a considerable number of solutions high levels of foaming capacity are reported, but very rarely 100 % and even less for emulsion stability. This is largely due to the fact that the inventions describe in priority the use of medium chain fatty acids for their benefit for the prevention of Alzheimer's Disease, without taking into account the possible combinations with other fatty acids of larger chains, suitable for the formation of emulsions, especially for the formation of foams within the emulsions;
- many of the products obtained decompose at room temperature or are sensitive to microbial contamination and require coatings that make digestion difficult;
- as mentioned above, no product so far disclosed as a nutraceutical or supplement contains components that intervene in all the processes attributed to the origin of Alzheimer's disease and other neurological conditions, such as anti-inflammatory, hypoglycemic, cholesterol-lowering, antioxidants, alternative energy sources to counteract insulin resistance at the neuronal level, antibacterial and antifungal, vermifuge, anti-aggregation of beta-amyloid peptide, anti neurofibrillary tangle formation and that promote neuronal synapses, as well as components with antihypertensive, antiviral, vermifuge, antitoxic, antiplatelet aggregation and cardiovascular protective activity;
- in general, the nutraceuticals and associated products described contain only one source of fatty acids, antioxidants, anti-inflammatories, or, when they contain more than one, these are of the same synthetic chemical nature, and therefore the metabolic or physiological pathways in which they act are the same;
- the nutraceuticals found in the scientific literature, especially those presented in the form of capsules, tablets and oral liquids, only contain biologically active substances that act on the Central Nervous System and are specifically related to Alzheimer's Disease, and do not provide other components that contribute to the vitality of the subjects to whom the products are supplied, or in very few cases contain only one class of these substances, for example, additionally contain some vitamin, amino acid or protein, or scarce macro- and micro-elements;
- there are no nutraceuticals that not only act on Alzheimer's disease and degenerative disorders, but also have an effect against other disorders of old age or age-related pathologies, such as inflammatory processes at other levels, for example, at the prostate level, urinary frequency disorders, frequent among other comorbidities present in these age groups;
- no natural nutraceuticals containing medium chain fatty acids as the only source of energy for neurons, combined with other antioxidant and anti-inflammatory substances, were found in the available bibliography;
- it is common to find nutraceuticals and supplements for the diseases addressed by the invention that contain compounds obtained by chemical synthesis, recombinantly, or obtained from animal sources;
- most of the nutraceuticals described in the scientific and patent literature and in commercial catalogs are generally presented as tablets, as other types of tablets, liquids, capsules, which are not suitable for subjects or patients with neurological disorders with difficulties for their independent feeding or totally unable for this purpose and for patients with difficulties for chewing or swallowing;

- the form of presentation of the previously developed products is limited to the form of presentation and technological or packaging capacity, such as tablets, rigid or soft capsules, among others, so that in order to provide high doses of active components to combat Alzheimer's Disease it would be necessary to administer the product several times. It should be noted that, in a considerable number of solutions, no examples of tests performed on experimental animals are shown to demonstrate that such doses are effective;
- in other cases, for the more advanced stages of the disease, the finished forms described in the previous paragraph are insufficient to meet the needs of the subjects, even with more frequent administration;
- it has been shown that the presentation of supplements in the form of traditional drugs induces the subjects who receive them to perceive them as drugs and reinforces the feeling of being ill, which in many cases causes depressive feelings and does not reflect wellbeing;
- many of the components of nutraceuticals are obtained with complex methods, expensive equipment, from very scarce sources and available in certain locations on the planet, which limits their sustainability;
- finally, we did not find in the literature any composition that in a short period of time demonstrated effective and significant cognitive improvement; in general, no results of reversion of cognitive deterioration are shown, only affirmations based on the demonstration of effects in very few examples, but for some of its components, or in very few solutions, results of experiments in experimental animals involving months of treatment to achieve a slight effect are claimed;
- for the first time, less oxidative damage to lipids in brain homogenate was evidenced in the group of aged mice treated with the nutraceutical than that found in young animals;
- surprisingly, a statistically significant improvement in antioxidant activity was demonstrated in the group of mice treated with the nutraceutical with respect to the untreated aged mice. This has not been previously described in prior art solutions for aged animal models using a product containing the components described in the present invention;
- with the technical solution proposed in the present invention, it is demonstrated, for the first time, that the cognitive status of the experimental animals treated with the nutraceutical not only exceeded that of the aged animals, but did not show significant differences with respect to that of the young animals;
- the reduction in blood glucose levels with respect to both young and aged animals that did not receive treatment has not been reported in other previous solutions using the analytical methodology described.

Nutraceutical, for the purposes of the present invention, is a set of active chemical or biological substances that can be found as natural components of food or added to the same and that, administered in doses equal to or higher than those existing in the food containing it, presume a favorable effect on health greater than that of normal food and have the capacity to strengthen healthy conditions, serving as an aid in the care and maintenance of health, as well as in the prevention and treatment of diseases and in the improvement of the physiological functions of the organism.

Cognitive deficit or impairment in the present invention relates to, but is not limited to, the occurrence of one of the following symptoms: impairment of short, medium or long-term memory; impairment of learning, attention and concentration.

The ratios between the different components in the present invention are expressed as mass:mass, (m/m) and in a particular case as volume:volume (v/v) and all guarantee the correct execution of the different variants.

The following is a detailed description of the invention.

Preparation of the emulsion components:

To prepare the emulsion, first the aqueous liquid phase is selected, which may consist of water, aqueous solutions of *Piper nigrum* extract or *Theobroma cacao* extract or combinations thereof, which are in ratios of 3 to 30 % m/m with respect to the total mass of the nutraceutical.

*Piper nigrum* extract is purchased from a commercial supplier or prepared from commercial powder by extraction from Cuban-grown black pepper (Fiallo, V. R. F., & Castro, L. P. L. (2000). Apuntes para la flora económica de Cuba III. Plantas condimenticias. Revista del Jardín Botánico Nacional, 21(1), 47-70. http://www.jstor.org/stable/42597069), (Jorge A. Pino, Juan Agüero & Victor Fuentes (2003) Chemical Composition of the Aerial Parts of Piper nigrum L. from Cuba, Journal of Essential Oil Research, 15:3, 209-210, DOI: 10.1080/10412905.2003.9712116).

*Theobroma cacao* extract is obtained from a commercial supplier or from cocoa powder grown in Cuba on an industrial scale (Damaris de la C. Salgado, Max Monan, J. G. D. P. I. G. R. S. E. G. (2018). Pharmacognostic and Physicochemical Studies of Theobroma cacao bean husk in Cuba. International Invention of Scientific Journal, 2(07). Retrieved from http://iisj.in/index.php/iisj/article/view/54), (Fajardo Rosabal, L., Figueredo Delgado, Y., Rosabal Cordoví, U., Guardia Puebla, Y., Rodriguez Pérez, S., Silva-Pupo, J., & Viera Tamayo, Y. (2020). Contenido de polifenoles totales en callos de Theobroma cacao L. clon 'UF-650'. Biotecnologia Vegetal, 20(1), 63-72. Recuperado de https://revista.ibp.co.cu/index.php/BV/article/view/656).

If it is desired to prepare the aqueous phase with an added solid phase, this solid phase is selected to be composed of soluble or water-suspendable solids, among them:

- a fructooligosaccharide sweetener in an amount of 1 to 30 % m/m with respect to the mass of the nutraceutical, developed by the Center of Genetic Engineering and Biotechnology of Cuba and commercialized by the Research Institute of Sugar Cane and its Derivatives of Cuba. (Pérez-Cruz, E. R., Martinez-Garcia, D., Rodriguez-Rico, I., Sobrino-Legón, A., & Hernández, L. (2011). Escalado de la reacción de biosíntesis de fructooligosacáridos, a partir de sacarosa, en biorreactores tipo tanque agitado. Tecnologia Química, XXXI(2), 19-25.), (Centro de Ingenieria Genética y Biotecnología. Enzimas Industriales. https://www.cigb.edu.cu/ research/_enzimas-industriales-2/),

- *Stevia rebaudiana* extract from plants grown in Cuba (Rodriguez González, Horacio, Acosta de la Luz, Lérida L, Hechevarría Sosa, Isabel, Rivera Amita, Maria Magdalena, Rodriguez Ferradá, Carlos Alberto, Sánchez Govín, Ester, & Milanés Figueredo, Masgloiris. (2007). Comportamiento del cultivo de Stevia rebaudiana (Bertoni) Bertoni en Cuba. Revista Cubana de Plantas Medicinales, 12(4) Recuperado en 21 de marzo de 2023, de http://scielo.sld.cu/ scielo. php?script=sci_arttext&pid=S 1028-47962007000400004&lng=es&tlng=es), (Ulloa Trujillo L. (2021). Proyecto avileño de investigación valida uso de la Stevia como edulcorante. Agencia Cubana de Noticias. 20 Diciembre. http://www.acn.cu/medio-ambiente/88821-proyecto-avileno-de-investigacion-valida-uso-de-la-stevia-como-edulcorante-fotos) en cantidades de 1 a 30 % m/m,

- *Apis mellifera* honey (honey from commercial suppliers), in quantities of 15 to 30 % m/m with respect to the total mass of the nutraceutical,

- sucrose (sugar of commercial quality) in a quantity of 1 % to 30 % m/m with respect to the total mass of the nutraceutical,

- liquor of the *Agave* fruit, preferably *Agave tequilana* (Paneque Escalona, T.; Polanco Arias, M.; Jiménez Nogueras, C.; Piquera Palomino, Y. (2019). Estudio etnofarmacológico de algunas especies endémicas de agave utilizados en la medicina tradicional. Revista Científica y Tecnológica UPSE, 6 (2) pág. 57-66. DOI: 10.26423/rctu.v6i2.471 ISER), (Maidelys et al. Effect of diet supplementation with meal of Agave tequilana stems on hematological indicators and blood biochemistry of fattening rabbits. Cuban Journal of Agricultural Science, [S.l.], v. 53, n. 4, dec. 2019. ISSN 2079-3480. Available at: http://www.cjascience.com/index.php/CJAS/article/view/920/913).

In the present invention these substances, in general, play the role of sweeteners, although they may participate in other biological functions.

In certain embodiments, the substances, preferably bee honey with the sweetening fructooligosaccharide in equal parts, or fructooligosaccharide alone, are mixed. If a sugar (sucrose) is selected, a syrup of the sugar in water is prepared beforehand, for which the mixture is heated in a sugar:water ratio of 0.2:1 to 2:1, without stirring, at a temperature of 60 °C to 100 °C, preferably 80 °C to 100 °C, until complete dissolution of the sugar; it is then cooled to a temperature of 40 °C to 60 °C. This procedure and the ratio of sugar to the aqueous phase ensure that the mixture does not crystallize or increase in hardness and, on the contrary, maintains the plasticity of the mixture obtained. Citric acid can be added (from 0.3 % to 0.7 % by mass). In embodiments where fructooligosaccharide is used, it is added to water and is not heated.

To form the emulsion, an aqueous, alcoholic or hydroalcoholic extract of *Curcuma longa* cultivated in Cuba is added in an amount of 0.1 to 10 % m/m with respect to the mass of the nutraceutical (Espinosa Reyes, Á., Silva Pupo, J., Borges Garcia, M., Fajardo Rosabal, L., Pérez Pérez, J., González Paneque, O., & Sariego Frómeta, S. (2009). Establecimiento y multiplicación in vitro de Curcuma longa L.. Biotecnologia Vegetal, 9(1). Recuperado de https:// revista.ibp.co.cu/index.php/BV/article/view/308), (Fernández Izquierdo A. Cúrcuma: una reina, para Cuba y el mundo. (2021). El artemiseño 16 junio, 2021. http://artemisadiario.cu/2021/06/curcuma-una-reina-para-cuba-y-el-mundo/) en cantidad de 0,1 a 10 % m/m con respecto a la masa total del nutracéutico.

In some embodiments, the emulsifying and/or foaming agent is selected from:

- soy lecithin and egg lecithin from commercial suppliers, such as that marketed by Procesadora de Soya de Santiago de Cuba, in a ratio of 0.1 to 1 % m/m with respect to the total mass of the nutraceutical. (Beatón Ruiz B. (2020) Trabajadores. 24 abril. https://www.trabajadores.cu/20200424/procesadora-de-soya-de-santiago-de-cuba-estable-en-su-quehacer-productivo/), (Lemus Rodriguez, Martha Zoe, Chong Quesada, Amaury, & Bosch Escobar, Jorge. (2017). Tableta masticable de lecitina de soya: de subproducto a producto farmacéutico. MEDISAN, 21(5), 556-563. Recuperado en 21 de marzo de 2023, de http://scielo.sld.cu/scielo.php?script=sci_arttext& pid=S1029-30192017000500007&lng=es&tlng=es);

- extract of *Saponaria* species, specifically *Saponaria officinalis* or *Saponaria ocymoides,* from commercial suppliers or obtained by extraction from the root of the plant, in a ratio of 0.1 to 10 % m/m with respect to the total mass of the nutraceutical *(Saponaria officinalis.* Ecured. https://www.ecured.cu/Saponaria), (Saponaria: propiedades medicinales, mucho más que para hacer jabón. Eco agricultor. https://www.ecoagricultor.com/saponaria-jabon/).

- egg white in a ratio of 3 % to 35 % with respect to the total mass of the nutraceutical.

- plant extract of the genus Agave, specifically extract of Agave americana, Agave tequilana, or Agave fourcroydes, in quantities of 0.1 to 10 % m/m with respect to the mass of the nutraceutical. (de Zayas, A. Á. (1984). Los agaves de Cuba Occidental. Revista Del Jardín Botánico Nacional, 5(3), 3-16. http://www.jstor.org/stable/42596752) (Acevedo-Rodríguez, P. & Strong, M.T. (2012). Catalogue of seed plants of the West Indies. Smithsonian Contributions to Botany 98: 1-1192.) (Vinent Serrano E. y Fajardo Gutiérrez O. Estrategia para el mejoramiento genético de Agaves en Cuba. Instituto de Investigaciones Horticolas "Liliana Dimitrova" (IIHLD). La Habana, Cuba. https: //www.utm.mx/edi_anteriores/temas037/N3.pdf) (Valdivia, A., Rubio Fontanills, Y., Hernández Álvarez, L., Jiménez Rabelo, J., Pérez Hernández, Y., & Portilla Tundidor, Y. (2018). Propiedades fitoquímicas y antibacterianas de los extractos de las hojas de Agave fourcroydes Lem. (henequén). Revista Cubana de Plantas Medicinales, 23(2). Recuperado de https:/lr-evplantasmedicinales.sld.cu/index.php/pla/article/view/452/302).

Agave extracts are obtained from companies that commercialize them or by extraction in water of the plant material at a temperature of 60 °C to 70 °C for 10 min to 30 min; the material is crushed, washed in water and mixed for 5 h to 8 h to obtain a decoction, decanted and filtered. This process is repeated several times while maintaining a level equal to or greater than 3 % of the active component in the extractions (can be measured by the solids). Upon completion, the extract is heated to boiling and concentrated to about 5 % to 15 % total solids, to ensure that the content of the emulsifying and foaming substances contained in the extract form the oil:water:air:suspended or dissolved solids emulsion. The content of these compounds (saponins) in the final product should not exceed 0.03 % in order not to cause damage to health, so the amount of extract to be added to the nutraceutical mass is calculated, always within the indicated limits.

In some embodiments, according to the present invention, when employing the *Agave* and *Saponaria* extracts, these are mixed with the sugar or honey at slower speeds, from 100 1/min to 150 1/min, for a period of 10 min to 30 min at temperature of 105 °C to 110 °C.

Subsequently, the oily phase of the emulsion or the oily phase with a solid phase soluble in the oily phase or with a solid phase suspended in the oily phase is added. The oily phase is composed of *Plukenetia* volubilis oil prepared by the Cuban Research Center for Protein Plants and Natural Bioproducts from plants cultivated in Cuba (González de la Torre, L, Rodriguez Leyes E.A., Vicente Murillo R.,. González Canavaciolo V.L, Diaz Rivera Y. Yurisley. Caracterización preliminar del aceite de Plukenetia volubilis L. (sacha inchi) cultivada en Cuba. Revista Cubana de Plantas Medicinales. (2022);27(1):e1227. file:///C:/Users/claudio/Downloads/1227-6625-1-PB.pdf) in a ratio of 5 to 20 % with respect to the total mass of the nutraceutical..

Under certain circumstances, substances that are not solubilized but suspended in the oil can be added to the oily phase, as in the case of some of the concentrated ethanolic or hydroalcoholic extracts of *Curcuma longa*.

The components are then stirred and mixed at stirring speeds of 100 1/min to 13,000 1/min to obtain the emulsion with larger droplet size between 201 nm and 10,000 nm; for obtaining the microemulsion with droplet size from 101 nm to 200 nm speeds of 6,000 1/min to 15,000 1/min are used and for the nanoemulsion with droplet size from 60 nm to 100 nm the speeds are from 12,000 1/min to 45,000 1/min. In other embodiments other methods of obtaining micro- and nano-emulsion droplets may be used, employing ultrasound and high pressures in combination with agitation.

When these components are agitated, the gaseous phase (air) is incorporated, which represents between 0.2 % v/v and 50 % v/v with respect to the volume of the emulsion. To obtain the highest volumes of air (10 % v/v - 50 % v/v), preferably egg white, extracts of *Saponaria officinalis, Saponaria ocymoides* or *agavaceae* are used. In certain embodiments the air is supplied at a pressure of up to 300,000 Pa.

In certain embodiments, for this emulsion preparation phase the temperature is maintained between 20 °C and 70 °C.

In certain embodiments, additives that improve the stability of the emulsion, such as pectin, gelatin or potato starch, can be used in amounts of 1 % with respect to the mass of the emulsion.

The final composition of the emulsions mentioned above contains between 5 and 7 components of different nature.

In some circumstances, a component performs several biological or functional functions, in other embodiments it is introduced for a single purpose.

In some embodiments for this emulsion preparation phase the temperature is maintained between 20 °C and 70 °C. In other embodiments the entire process is run in said temperature range (between 20 °C and 70 °C, preferably from 25 °C to 50 °C).

In this way, depending on the embodiments to be executed, 10 variants of 3- to 4-phase emulsions are obtained, including: aqueous:oily:gaseous; aqueous:oily:gaseous:solids suspended in the oily; aqueous:oily:gaseous:solubilized solids in the aqueous; aqueous:oily:gaseous:solubilized solids in the aqueous and solubilized solids in the oily; aqueous:oily:gaseous:solubilized solids in the aqueous and solubilized solids in the oily; aqueous:oily:gaseous: solids solubilized in aqueous and solids suspended in aqueous; aqueous:oily:solids solubilized in aqueous; aqueous:oily:solids suspended in aqueous, aqueous:oily:solubilized solids in aqueous; aqueous:oily:solubilized solids in aqueous and solubilized solids in aqueous; aqueous:oily:solids suspended in aqueous and solubilized solids in aqueous.

In parallel, or later, the mass is prepared from seeds selected from *Cucurbita moschata* or *Cucurbita pepo* cultivated in Cuba (Características agronómicas del cultivo de la calabaza e importancia nutricional y medicinal. Monographs. https://www.monografias.com/trabajos55/calabaza-cubana/calabaza-cubana). Seeds can be processed in one of the following ways: whole and ground; shelled and ground; whole, dried and ground; whole, ground and roasted; whole, dried, ground and roasted; whole, dried, ground and roasted; shelled, ground and roasted; shlled, ground, processed in salted water, washed, separated and roasted; shelled, dried, ground and roasted.

In some embodiments, the seeds are pre-soaked in hot water for 30 min to 50 min, allowed to stand for 2 h to 6 h and then deshelled. In others, to obtain a somewhat salty taste or to aid the shelling process, the seeds are immersed in 4 % - 6 % brine (NaCl) at a ratio of 6 % to 10 % seed mass to brine volume. On occasions when seed drying is foreseen as final processing, either in shell or for shelling, it is carried out at temperatures of 80 °C to 115 °C for 30 min to 60 min. Roasting of whole or shelled seeds is carried out at temperatures between 80 °C to 130 °C.

The grinding of the seeds is executed by any of the known methods until a particle size of 10 μm to 70 μm is achieved, preferably from 10 μm to 30 μm.

To obtain the final nutraceutical composition, a food paste-like macro-emulsion is formed by mixing the emulsion with the seed and oil mass. For this purpose, *Plukenetia volubilis* oil is added to the seed mass (40 % m/m to 65 % m/m with respect to the total mass of the nutraceutical) in quantities of 1 % m/m to 20 % m/m with respect to the final mass of the nutraceutical, thus forming a seed and oil mass that represents 40 % m/m to 70 % m/m of the final mass of the nutraceutical.

The emulsion is added to the mass of seeds and oil in an amount of 20 % m/m to 60 % m/m with respect to the total mass of the nutraceutical. The addition is carried out under constant agitation at constant or variable speeds, manually, mechanized or automated, with different mixer propeller constructions to ensure homogeneous mixing and the least loss of trapped air. As already mentioned, the mixture is set up such that the raw or roasted, shelled or whole seeds of *Cucurbita moschata* or *Cucurbita pepo* are in concentration of 40 % m/m to 65 % m/m with respect to the total mass of the nutraceutical.

In certain embodiments, to this mixture composed of emulsion and the mass of seeds and oil is added *Theobroma cacao* powder in amount of 0.5 % m/m to 9 % m/m with respect to the total mass of the nutraceutical.

Sometimes, *Cocos nucifera* oil is added to the emulsion mixture with the mass of seeds and oil in quantities of 5 % m/m to 20 % m/m with respect to the total mass of the nutraceutical (Alvarado-Ruffo K., Blanco-Imbert A., M. de la Noval-Pons B, Martin-Alonso G.M. (2018). Nursery propagation of Cocos nucifera L. Case study: Baracoa. Tropical Crops. 39(4). https://ediciones.inca.edu.cu/index.php/ediciones/article/view/1487/html) from commercial suppliers, as in the case of the Baracoa Oil Production Plant, Cuba, or obtained personally from fruit collected in the fields.

In other embodiments, in addition to the aforementioned seeds, emulsion and cocoa powder, a mass of fruits and seeds from commercial sources or directly harvested from crops may be added in amounts of 1 % m/m to 10 % m/m with respect to the total mass of the nutraceutical selected from among the fruit of:

- *Cocus nucifera* (preferably);
- *Prunus dulcis* nuts harvested in Cuba (Ecured. Pronus dulcis. https://www.ecured.cu/Almendra), (Fiallo, V. R. F. (2003). Apuntes para la flora económica de Cuba VII. Especies Frutales. Revista Del Jardín Botánico Nacional, 24(1/2), 177-217. http://www.istor.org/stable/42597201);
- fruits of Corylus avellana grown by small producers in organoponic gardens in Cuba (Ecured. Anexo:Especies de frutales cultivadas en Cuba en la agricultura urbana. https://www.ecured.cu/Anexo:Especies_de_frutales_cultiva das_en_Cuba_en_la_agricul tura_urbana).

In some cases, natural juices or fruit concentrates from commercial suppliers or obtained from fruits, are added to the mixture of the emulsion with the mass of seeds and oil in quantities of 1 % m/m to 5 % m/m with respect to the total mass of the nutraceutical, preferably natural grape juice concentrate.

In other cases, protein sources in the form of isolates, concentrates, extracts or integral powders, preferably from *Moringa oleifera,* are added to the emulsion mixture with the mass of seeds and oil (MARTÍN, C. et al. Potential applications of Moringa oleifera. A critical review. Pastures and Forages, [S.I.], v. 36, n. 2, Sep. 2013. ISSN 2078-8452. https://payfo. ihatuey.cu/index.php?journal=pasto&page=article&op=view&path%5B%5D =1539) or whey in amounts of 1 % to 10 % m/m with respect to the total mass of the nutraceutical.

The mixing of the emulsion with the rest of the components to form a food emulsion type macroemulsion, with all the components of the final nutraceutical formulation, is carried out, in some cases, at speeds of 10 1/min to 200 1/min. In other cases it is manually stirred until a macroemulsion of the nutraceutical is obtained in the form of a dense paste or it is manually mixed with paddles of different materials and applying enveloping movements.

Subsequently, the macroemulsion of the nutraceutical obtained with all the components is cooled, preferably at temperatures of 2 °C to 10 °C. In certain embodiments this step is carried out at room temperature, for an interval of 5 h to 24 h. In other circumstances, the cooling is executed on an automatic production line with ventilation tunnel with forced cold air circulation or without tunnel; the cooling occurs in a shorter time interval. Once the dough is cooled, it is cut and portioned into bars or portions.

The nutraceutical of solid consistency obtained is packaged in the form of bars or nougat, or individual pieces, with a mass from 20 g to 80 g, preferably from 20 g to 40 g and more preferably from 20 g to 30 g, of various shapes, preferably covered with waterproof paper or placed in containers impermeable to grease, in PVC for example. The nutraceutical is stored at temperatures from 5 °C to 40 °C, preferably from 4 °C to 25 °C.

As described above, different formulations of the nutraceutical can be obtained by combining the components to guarantee the expected biological effect on the different therapeutic targets involved in the triggering of Alzheimer's disease, other diseases, disorders or physiological states. A single component may exhibit several favorable biological actions to prevent, halt or ameliorate the course of the disease or metabolic disorder, or to improve the general state of health.

In various embodiments the nutraceutical contains 3 to 5 different sources of fatty acids, phytosterols and triacylglycerols, among them are the seeds of *Cucurbita moschata, Cucurbita pepo,* or combinations thereof; oil present in the emulsion or in the seed and oil mass *(Plukenetia volubilis)* or *Cocos nucifera* oil added to the seed mass; soy lecithin or egg lecithin as part of the emulsion; *Prunus dulcis* and *Corylus avellana* nuts; *Theobroma cacao* powder, added to the seed mass once the emulsion is introduced.

Thus, in all embodiments are present compounds belonging to the groups of fatty acids from 6 to 12 carbons, such as hexanoic acid, octanoic acid, decanoic acid and docecanoic acid; other fatty acids such as octadecatrienoic acid, eicosapentaenoic acid and docosahexaenoic acid, fatty acids with a higher number of carbon atoms such as 9,12-octadecadecadienoic acid, 11,14-eicosadienoic acid, 5,8,11,14-eicosatetraenoic acid, 13,16-docosadienoic acid, 7,10,13,16-docosatetraenoic acid, 4,7,10, 13,16-docosapentaenoic acid and 8e,10e,12z-octadecatrienoic acid, 9-octadecenoic acid, 11-eicosenoic acid, 5,8,11-eicosatrienoic acid, 13-docosenoic acid and 15-tetracosenoic acid, tetradecanoic acid, hexadecanoic acid, delta-9-cis-hexadecenoic acid, octadecanoic acid, nonadecanoic acid, heptadecanoic acid; in addition the phytosterols β-sitosterol, campesterol, stigmasterol, stigmastenol and the triacylglycerols ECN 36 (LnLnLn), ECN 38 (LnLnL), ECN 40 (LnLLL + LnLnP+LnLnO), ECN 42 (LLL+OLLn+PLLn), ECN 44 (LLO + OOLn +LLP+ POLn), ECN 46 (OOL+ LnPP+ PLO), ECN 48 (OOO+ POO), ECN 50 (SSO).

*Cucurbita spp.* seeds provide the nutraceutical with Ca, Mg, Fe, P, K, Na and Zn, while those of *Pronus dulcis* provide P at significant levels. *Theobroma cacao* powder provides K, P, Fe, Na, Mg, Ca, Cu, Zn and Se.

In various embodiments, the final nutraceutical product contains from 2 to 8 components with antioxidant activity, including *Curcuma longa* or *Piper nigrum* extracts or combinations thereof; *Plukenetia volubilis* as part of the emulsion; *Theobroma cacao* powder added to the seed mass; *Agave tequilana* juice, *Agave fourcroydes* juice, *Saponaria officinalis* juice, *Saponaria ocymoides* juice or *Stevia rebaudiana* extract as part of the emulsion. In some embodiments, extracts of *Curcuma longa* or *Piper nigrum* are combined with one or more of the extracts or juices of *Agave tequilana, Agave fourcroydes, Saponaria officinalis,* or *Saponaria ocymoides* as part of the emulsion. In other embodiments, one or more components of each of the aforementioned groups are combined with *Theobroma cacao* powder, which is added the seed mass or in the emulsion. In other embodiments, one or more of the components of each of the above three groups are combined with *Stevia rebaudiana* extract, forming part of the emulsion.

Included in the final nutraceutical composition are 1 to 3 sources of hypoglycemic compounds selected from seeds of *Cucurbita moschata, Cucurbita pepo,* or combinations thereof; seeds of *Cocos nucifera* which is added to the seed mass; one of the following components: *Agave cajalbanensis* juice, *Agave tequilana* juice, *Agave fourcroydes* juice, *Saponaria officinalis* juice, *Saponaria ocymoides* juice forming part of the emulsion or *Stevia rebaudiana* extract.

In some embodiments *Cucurbita moschata* seeds are used, in others *Cucurbita pepo* seeds are used. In other formulations of the nutraceutical, *Cucurbita moschata* or *Cucurbita pepo* are combined. In certain other formulations these seeds are combined with an oil selected from *Cocos nucifera.*

In all embodiments, 1 to 4 different sources of anticholesterolemic compounds are used, selected from one of the oils of *Plukenetia volubilis L.* or *Cocos nucifera* oil, added to the seed mass; *Theobroma cacao* powder, added to the seed mass; soy lecithin or egg lecithin, preferably soy lecithin and/or *Agave fourcroydes* fruit liquor or *Agave tequilana* fruit liquor, all as part of the emulsion.

Specifically, in certain compositions of the nutraceutical, *Plukenetia volubilis L.* oil is combined with *Cocos nucifera* oil.

In other compositions, one of each of the two aforementioned sources is combined with *Theobroma cacao* powder and soy or egg lecithin, preferably soy lecithin and/or with *Agave fourcroydes* fruit liquor or *Agave tequilana* fruit liquor. In other embodiments, the above mentioned components are combined, with the exception of *Theobroma cacao.*

To make up the nutraceutical, 1 to 4 sources of anti-inflammatory compounds selected from *Stevia rebaudiana* extract forming part of the emulsion; *Cucurbita moschata* or *Cucurbita pepo* seeds, or combinations thereof, are used; *Theobroma cacao* powder added to the seed mass; *Piper nigrum* extract forming part of the emulsion; *Agave fourcroydes* fruit liquor or *Agave tequilana* fruit liquor also forming part of the emulsion.

In all the compositions are used protein sources of different origins, preferably of vegetable origin, selected among the seeds of *Cucurbita moschata, Cucurbita pepo* or their combinations, the egg white as part of the emulsion or combinations of the subgroups of the seeds with the egg white.

In some embodiments, other vegetable protein sources may be incorporated into the seed mass in amounts of 1 % to 10 % with respect to the nutraceutical mass, specifically isolates, concentrates, extracts or integral powders in the form of soybean, peanut, canola, pea meals, preferably from *Moringa oleifera.*

In other embodiments, the added proteins are of animal origin, specifically, whey proteins.

All nutraceutical formulations contain components that provide vitamins in sufficient quantities to supply different vitamin deficiencies, or to maintain the necessary physiological level; among these components are some of those already mentioned above, which, due to the percentage they represent in the final composition, are distinguished by this contribution. The *Cucurbita* seeds that provide vitamins A, K, E, folic acid, soy lecithin that provides choline; *Pronus dulcis* that provides B1, B2, B3, B6, C and E stand out. *Agave fourcroydes* fruit liqueur or *Agave tequilana* fruit liqueur provides vitamins A, C, E, B1, B2, B3, B6, B12, folic acid and choline. *Theobroma cacao* powder provides vitamins A, B and E.

The formulations contain components that provide important minerals for biological activity, including *Theobroma cacao* powder that provides Mg, Fe, K, Zn, P, Na, Ca, Cu, Se; ethanolic extract of *Curcuma longa* that provides Na, K, Mg, Ca, Fe, Zn.

The seeds of *Cucurbita spp.* provide the nutraceutical with Ca, Mg, Fe, P, K, Na and Zn. While those of *Pronus dulcis* P in significant levels. *Theobroma cacao* powder provides K, P, Fe, Na, Mg, Ca, Cu, Zn and Se.

The nutraceutical composition contains substances that provide organoleptic characteristics, such as easy chewability (without effort) and easy swallowing (does not adhere to the upper digestive tract, but slides down it) for the subjects and patients who consume it. The components that guarantee these properties also have biological functions already described.

In certain embodiments, *Theobroma cacao* powder masks the flavor of the *Curcuma longa* extract and the intense yellow color characteristic of the extract; in other embodiments, the oils of *Plukenetia volubilis* and *Cocos nucifera,* in addition to masking the flavor of the *Curcuma longa* extract, give the nutraceutical a smooth consistency, increase adhesiveness, make the nutraceutical easier to swallow and facilitate chewing; honey from *Apis mellifera* gives a sweet and characteristic flavor, accepted by most people and increases adhesiveness. On the other hand, *Stevia rebaudiana* extract provides sweetness to the composition, which eliminates or diminishes the characteristic flavor of *Curcuma longa* extract.

In other circumstances, natural juices, concentrates or fruit extracts are added to the seeds in substitution or in combination with the *Theobroma cacao* powder, among them natural commercial products of grapes, citrus, mint, anise, anon, preferably grapes.

The final formulation includes components containing three or more fatty acids selected from among the 6 to 12 carbon fatty acids. These fatty acids are present in components that cover other biological functions within the final formulation, but define the role they play in neurodegenerative diseases, particularly Alzheimer's Disease. Among these components, *Cocos nucifera* and *Plukenetia volubilis* oils stand out.

The nutraceutical composition obtained, according to the present invention, is used for the prevention or treatment of the following diseases, disorders or syndromes, but not limited to: cognitive deficit, Alzheimer's disease, mental, behavioral and developmental disorders, including depressive disorders; of the nervous system such as sclerosis, ataxias, Parkinson's; of the digestive system such as constipation, diarrhea, heartburn; of the immune system such as Diabetes mellitus, celiac disease, Crohn's disease; of the endocrine system, in particular diabetes mellitus and prediabetes; of the circulatory system such as venous insufficiency; cardiovascular diseases including ischemic heart disease, coronary artery disease, peripheral vascular disease and endocarditis; cerebrovascular diseases such as ischemic cerebrovascular disease; infectious and parasitic diseases; oncological and blood diseases; as well as tiredness, fatigue and malnutrition.

In certain embodiments, the product can be consumed in the regular diet of persons without any apparent impairment, disorder or disease.

The nutraceutical is administered or consumed with a frequency of 1 to 3 times a day in portions of 20 g to 80 g, with daily frequency, every other day, every three days, for a period of one month, rest from 3 days to 30 days, repeating the cycle of administration.

In the following, the role of the main components of the present invention acting in an original and very dissimilar manner is described. In particular, activities are emphasized which have not been described in any of the prior inventions reviewed during the extensive background search. These components are presented with all the purposes and effects in which they are described in the present invention, as well as those components which can be employed for the same purpose, either because of their related chemical composition, or because of their common biological properties: *Curcuma longa* extract rich in curcuminoids:

- functional role: during experiments it was surprisingly determined that this extract masks the bitterness and yerba mate flavor of the total *Stevia* extract and tones down the color of cocoa, decreasing its intensity, so there is no need to purify the extracts,

- specific biological activity on cognitive impairment and neurological diseases: the extract is able to cross the blood-brain barrier and stimulates the production of Brain-Derived Neurotrophic Factor (BDNF). (Sarraf P, Parohan M, Javanbakht MH, Ranji-Burachaloo S, Djalali M. Short-term curcumin supplementation enhances serum brain-derived neurotrophic factor in adult men and women: a systematic review and dose-response meta-analysis of randomized controlled trials. Nutr Res. 2019 Sep;69:1-8. doi: 10.1016/j.nutres.2019.05.001. Epub 2019 May 9. PMID: 31279955),

- the extract is responsible for the establishment of neuronal synapses, possesses anti-amyloid-beta protein aggregation activity, potent antioxidant activity and activation of antioxidant enzymes and potent anti-inflammatory activity, prevents lipid peroxidation, promotes degradation of neurofibrillary fibrils and tangles in vitro and prevents lipid peroxidation (Franco-Robles E, Campos-Cervantes A, Murillo-Ortiz BO, Segovia J, López-Briones S, Vergara P, Pérez-Vázquez V, Solís-Ortiz MS, Ramirez-Emiliano J. Effects of curcumin on brain-derived neurotrophic factor levels and oxidative damage in obesity and diabetes. Appl Physiol Nutr Metab. 2014 Feb;39(2):211-8. doi: 10.1139/apnm-2013-0133. Epub 2013 Aug 23. PMID: 24476477),

- curcumin reduces tau protein phosphorylation and, as shown in the present research, improves learning in laboratory animals. This is because it prevents the formation of protein dimers and their oligomerization,

- the present polyphenols maintain better mental health and cognitive status, demonstrated in population studies and supported by the demonstration of decreased aggregation of Aβ peptide and hyperphosphorylated Tau protein in the hippocampus (Mazzanti G., Di Giacomo S. Curcumin and Resveratrol in the Management of Cognitive Disorders: What Is the Clinical Evidence? Molecules. 2016;21:1243. doi: 10.3390/molecules21091243),

- the extract has high content of minerals such as Na, K, Mg, Ca, Fe, Zn and contains vitamins such as C, thiamine, riboflavin, niacin, B6, B12, A, D, E, K, folate,

- activity on general health: it increases endothelial function, preventing coagulation in veins, increment of blood pressure and the onset of heart disease; it has anticarcinogenic properties by inhibiting angiogenesis, relieves or prevents rheumatoid arthritis (due to its anti-inflammatory effect). (Abad, Romina del Carmen, Vasena, Maria Catalina. 2020. Fresh turmeric: chemical-nutritional composition, use in bakery product and sensory evaluation. https://rdu.unc.edu.ar/bitstream/handle/11086/18668/tesis%20completa%201462.pd   f?sequence=1&isAllowed=y).

*Piper nigrum extract:*

- contains piperine, its main active ingredient. Studies show that this component is effective for inflammation and for the prevention of premature aging due to its amount of antioxidants,
- increases the bioavailability of curcuminoids.

*Plukenetia volubilis L. (Sacha inchi)* oil:

- functional role: in the experiments carried out, it solubilized and helped the better suspension of the curcuminoids, acted as an oily phase in the emulsions, especially very appropriate to form nano- and micro-emulsions due to its composition of fatty acids; solubilizes the liposoluble vitamins of the components, among them properties not described before and that were detected during the invention process; the results of the experimentation phase of the present invention showed that this oil enables better swallowing (the nutraceutical slips easily through the palate and along the path to the stomach), provides mechanical properties necessary for elderly people, especially easy chewability (the nutraceutical of the present invention does not need intense chewing), hardness (the nutraceutical is soft to the palate), adhesiveness, uniformity and cohesion of the components; maintains a volume of air trapped in the emulsion of 3 and 4 components and of the nutraceutical structure; provides a taste rated as "pleasant" according to the organoleptic panel scale and, surprisingly, masks the characteristic taste (bitter aftertaste) of other components *(Stevia* extract, turmeric extract),

- in different examples of execution it was demonstrated that this oil possesses specific biological activity on cognitive impairment and neurological diseases in very short term. Its polyunsaturated fatty acids, especially Omega 3 and 6 are found in large quantities; as already mentioned in the review of the state of the art, these fatty acids are easily converted in the organism into docosapentaenoic acid, docosahexaenoic acid and arachidonic acid, which intervene significantly in the Central Nervous System, in its development and in the elimination of toxic effects on neurons. In different examples of realization it was demonstrated the decrease of oxidative stress and, as demonstrated in the present invention, for the first time it is achieved the improvement of learning and cognitive improvement in the short term,

- activity on general health: several of the executed examples showed that it decreases total cholesterol and low density lipids in blood,

- the oil is known to reduce blood pressure and tissue damage.

Processed seeds of *Cucurbita moschata* and *Cucurbita pepo*:

- functional role: in an original manner in this invention: provides a solid phase that helps to set the shape or unidosis of the nutraceutical, enables the formation of a homogeneous structure in which the components do not separate (no separation of aqueous or oily phase) due to its high fat and water absorption capacity; the oil present is incorporated into the final emulsion, provides a characteristic roasted flavor accepted by the organoleptic evaluation panel, masks undesirable colors (dark brown) of turmeric extract and the bitter taste of *Stevia* extract,
- specific biological activity on cognitive impairment and neurological diseases: alpha lipoic acid is able to penetrate the blood-brain barrier; in the experiments carried out, the antioxidant and neuroprotective effect on nervous tissue was demonstrated and an anti-inflammatory effect on neuronal tissue was experimentally detected,
- a content of short and medium chain fatty acids that improve the mitochondrial function and the energy supply to the neurons was detected, which may explain the cognitive improvement found in the tests carried out as part of the research included in the present invention,
- the oil contained in the seed has cardioprotective, hypolipidemic, anti-hypertensive and hypoglycemic activity,
- activity on general health: anti-inflammatory effect; protein supply with a balance of essential amino acids with respect to non-essential ones, as a dietary supplement,
- provides tryptophan to the nutraceutical in significant concentrations, an amino acid that produces serotonin and melatonin in the body, which reduces anxiety and insomnia characteristic of patients suffering from neurological disorders and especially Alzheimer's disease.
- anti-inflammatory activity on the prostate; regulates urinary frequency, very common in elderly people, partly due to cucurbitin (-)-3-amino-3-carboxypyrrolidine),
- high zinc content that provides anti-inflammatory properties and is essential for the activity of thymic hormones that are used in anti-inflammatory processes and modulators of the immune response,
- provides iron, magnesium, phosphorus and phytosterols that act as purifiers of the circulatory system and prevent cardiovascular diseases, and provides boron, cobalt, iron, magnesium and potassium in physiological quantities to the nutraceutical, so that the ration of the same (20 g - 50 g) as a minimum daily intake, would cover by itself the physiological needs of elderly people,
- contains vitamins A, K and E, which act as antioxidants, and is a natural source of folic acid, which is described to prevent myocardial infarction and to help fix the iron provided by the other ingredients of the nutraceutical,
- provides fiber to the nutraceutical, which facilitates the transit of the active substances through the intestine, improves their absorption, prevents constipation and protects the microbiome, which was corroborated in the experiments carried out, since the experimental animals consumed more fiber than those of the control group and the feces. These fibers stabilize the microbiome, the deterioration of which, as has been shown, has a vital influence on the onset of Alzheimer's disease. (Ghosh T.S., Rampelli S., Jeffery I.B., Santoro A., Neto M., Capri M., Giampieri E., Jennings A., Candela M., Turroni S., et al. Mediterranean diet intervention alters the gut microbiome in older people reducing frailty and improving health status: The NU-AGE 1-year dietary intervention across five European countries. Gut. 2020;69:1218-1228. doi: 10.1136/gutjnl-2019-319654).
- lycopenes in the peel have antioxidant properties, being this activity the highest among all carotenoids, and unlike the other carotenoids in the composition, it does not possess pro-vitamin A properties.

Several of its qualities are described in the publication: Lemus-Mondaca, Roberto, Marin, Jessami, Rivas, Josefa, Sanhueza, Leyla, Soto, Yasna, Vera, Natalia, & Puente-Díaz, Luis. (2019). Pumpkin seeds (Cucurbita maxima). A review of functional attributes and by-products. Revista chilena de nutrición, 46(6), 783-791. https://dx.doi.org/10.4067/S0717-75182019000600783.

*Theobroma cacao:*

- functional role: it provides a smooth texture, classified as "pleasant" according to the evaluation scale of the organoleptic panel to the composition; its "pleasant" taste surprisingly masks the bitter taste of *Stevia* extract and turmeric,
- specific biological activity on cognitive impairment and neurological diseases: anti-inflammatory and antioxidant activity, rich in flavonoids that penetrate the blood-brain barrier, with potent antioxidant activity, interacts with kinases (PI3, ERK) increasing the expression of neuroprotective and neuromodulatory proteins and thus neuronal synapses, potentially prevents neuronal death, prevents Beta amyloid peptide aggregation by inhibiting beta-secretase and activating alpha-secretase in vitro, ensures neuronal synapse, cognitive enhancement, neuronal angiogenesis; in animal models it has been shown to protect against stroke and dementia, arrests cognitive decline (David L. Katz, Kim Doughty, and Ather Ali. Cocoa and Chocolate in Human Health and Disease. Antioxidants & Redox Signaling. 2011 Nov 15; 15(10): 2779-2811. doi: 10.1089/ars.2010.3697),

- activity on general health: cardiovascular protector, general antioxidant, general anti-inflammatory, reduces cholesterol and total triglyceride levels, increases high-density fatty acid levels.

Soy lecithin and egg lecithin:

- functional role: emulsifier, in the present invention it acts originally as a foaming agent, for the first time it made it possible to emulsify complex multicomponent systems of three phases (oily, aqueous, gaseous) and four phases (suspended solids contained in the droplets are incorporated into the three-phase system) with at least 5-6 components,
- specific biological activity on cognitive impairment and neurological diseases: the phosphatidylserine it contains helps to improve cognitive capacity and memory in animal models, high phosphorus content,
- activity on general health: rich in omega 3 and omega 6 essential fatty acids, reduces blood cholesterol, improves liver function,
- its combination with *Saponaria spp., Clidemia hyrta* and agavaceae extracts prevents the hemolysis of red blood cells that these extracts may possess, which ensures that it is safe to use these extracts to achieve the significant levels of air trapped in the emulsion and in the final product, which implies a softer structure to chewing.

Juice of *agavaceae:*

- contains saponins and sapogenins,
- functional role: as emulsifier and foaming agent, buffer of the composition. In the present invention it was originally used to emulsify complex multicomponent systems of three or four phases (oily, aqueous, gaseous, solid) with at least 5-6 components,
- specific biological activity on cognitive impairment and neurological diseases: antioxidant, anti-inflammatory, it provides saponins and sapogenins involved in communication between neurons and removal of acetylcholine; provides vitamins A, C, E, B1, B2, B3, B6, B12, folic acid and choline; helps reduce blood cholesterol levels as it contains sitosterol which mimics it by its structure, preventing its absorption at high levels; modulates the concentration of pro-inflammatory cytokines and improves cognitive function,
- activity on general health: it helps to alkalinize the body, absorbs toxins at the intestinal tract level, provides polysaccharides that protect the intestine from colonization by bacteria and viruses by helping the formation of macrophages, it has anti-inflammatory activity at the intestinal level and it is a vermifuge.

Extract of *Saponaria officinalis* and *Saponaria ocymoides:*

- surprising foaming capacity that allows to obtain nano and microemulsion with a high amount of soluble or suspended solids in complex three- and four-phase and multicomponent emulsions, and moreover, to integrate the emulsion to even more complex emulsions of the emulsified food type, maintaining the easy chewability and guaranteeing the maintenance of a solid structure, which does not fracture or lose shape,
- antioxidant properties due to its aglycon and apigenin content.

Egg white:

- the emulsifying and foaming capacity of egg white is known, which, in the form in which it was used in the present invention, in the concentrations evaluated and with the processing method established, made it possible to obtain very stable nano and micro emulsions, with a high percentage of gas phase, which made it possible to obtain a texture that is very easy to chew and swallow,

- significantly reduces the color intensity of the other components, such as those of *Stevia* extract, turmeric extract and chocolate powder,

- surprisingly, it provided a high emulsion stability of 100 % for 24 days and more,

- surprisingly, the inclusion in the nutraceutical composition of raw egg white did not influence the microbiological deterioration and stability, since the product preserved for more than one year under refrigeration and did not suffer deterioration by biochemical or microbiological decomposition; this is due to the original and new combination of the egg white with the other components of the emulsion and the product with high bactericidal and fungicidal power.

*Stevia rebaudiana* extract:

- useful for the treatment of type II diabetes, since it possesses glycosides,
- sweetening properties without calories (sweetening power 200-300 times higher than sugar),
- helps control blood pressure,
- hypoglycemic effect, improves glucose absorption,
- benefits fat absorption,
- antibiotic and antifungal properties,
- helps to improve the taste caused by *Curcuma longa* extract.

To the mixture of the emulsion with the mass of seeds and oils are added, in certain cases, a mass of fruits and seeds, that provide flavor and odor accepted by groups of consumers, improve the taste and odor and fulfill additional biological functions.

*Cocos nucifera* fruit and oil:

- functional role: its oils solubilize the fat-soluble vitamins of the components, and as a result of the experimental work of the authors of the present invention, it was determined that, surprisingly, it enables the better swallowing of the nutraceutical (it slips easily over the palate and along the way to the stomach and ensures this phenomenon in conjunction with the other components); it provides mechanical properties characteristic of nougats, in particular its chewiness (the nutraceutical does not require intense chewing), hardness (the nutraceutical is soft to the palate), adhesiveness, uniformity and cohesion of the components, maintains a volume of air trapped in the structure of the nutraceutical, provides a characteristic coconut flavor and masks the characteristic flavor of other components (*Stevia* extract, turmeric extract),

- specific biological activity on cognitive impairment and neurological diseases: its medium-chain fatty acids are easily converted in the liver into ketone bodies and these provide alternative energy to the brain in subjects with insulin resistance in that organ,

- activity on general health: antibacterial activity (the monolaurin derived from its enzymatic digestion inhibits pathogenic viruses, bacteria and yeasts),

- surprisingly, it made it possible to decrease the possible activity of bacteria and, after one year of preserving the product in refrigeration, it did not decompose due to contamination,

- the study of its composition by the methods used showed that it is rich in phenolic acids with known antioxidant activity (ferilic, caffeic, p-coumaric, among others, so it should have a lowering effect on plasma cholesterol and blood glucose content,

- by its structure these acids, especially ferulic acid, reduces the levels of aggregation of $A\beta$ amyloid.

*Corylus avellana* fruit:

- contains about 16 % of proteins,
- important source of hypocholesterolemic, antioxidant and anti-inflammatory substances,
- high content of vitamins such as thiamine and niacin,
- among the minerals it contains, Ca, P, Mg and K stand out,

- provides fiber, which improves digestion,
- increases the nervous connection and the tissue of the neurons,
- reduces the disintegration of platelets and contributes to reduce thrombosis,
- contains unsaturated fatty acids, Omega 3 and Omega 6.

*Pronus dulcis* fruit:

- contains proteins, fats (monounsaturated fatty acids), dietary fibers and minerals, such as calcium,
- its content in sugars and other carbohydrates is very low and does not exceed 4 %,
- contains vitamins E and C,
- contains monounsaturated fatty acids that help reduce cholesterol levels,
- antioxidant properties,
- provides a pleasant taste to the nutraceutical.

A brief review of the figures is given bellow:

Figure 1 shows the particle size measurement of the CO26 composition in zetasizer equipment. On the x-axis: size in nm; on the y-axis: intensity measured in %; V26 variant oil:water ratio = 1:1.5 v/v.

Figure 2 shows the particle size measurement of the CO27 composition in zetasizer equipment. On the x-axis: size in nm; on the y-axis: intensity measured in %; composition CO27 oil:water ratio = 1:1.5 v/v.

Figure 3 shows the effect of *Curcuma longa* extract, *Cucurbita moschata* seeds and nutraceutical in OF1 mice on oxidative lipid damage in brain homogenate. * Significant differences with respect to G1. + Significant differences with respect to G2

Figure 4 shows the effect of *Curcuma longa* extract, *Cucurbita moschata* seeds and nutraceutical in OF1 mice on total antioxidant capacity. * Significant differences with respect to G1. + Significant differences with respect to G2.

Figure 5 shows the results of the time spent in the arms in the Y-maze test with a closed arm in OF1 mice supplemented with the variant 3 component (V3). * Significant differences with respect to G1. + Significant differences between the closed arm and the others within the same group ($p<0.05$).

Figure 6 discloses the results of the latency to address the quadrant where the platform was in the Morris water maze test in male OF1 mice that were supplemented for 5 weeks with the V5 variant component (G3). G1-young control group and G2-group of aged animals that received i.g. vehicle (10 % Tween-20 in 0.9 % NaCl). * Significant differences with respect to G2.

Figure 7 shows the results of the Y-maze test of OF1 mice supplemented with the nutraceutical for 5 weeks orally. A) Number of entries. B) Percentage of alternation. ANOVA and Tukey post hoc test were applied ($p <0.05$). * Significant differences with respect to G1. + Significant differences with respect to G2.

Figure 8 shows the results of the experiment to assess the latency period in the passive avoidance test (24 h retention) of OF1 mice supplemented with the nutraceutical for 5 weeks orally. A) Acquisition phase. B) Retention phase. ANOVA and Tukey post hoc test were applied ($p <0.05$). * Significant differences with respect to G1. + Significant differences with respect to G2.

Figure 9 shows the effect of *Cocus nucifera* oils of *Plukenetia volubilis, Curcuma longa* extract and nutraceutical on serum glucose levels in male OF1 mice * Significant differences with respect to G1 and G2 groups ($p <0.05$).

Figure 10 shows the results of the experiment to determine the effect of nutraceutical and *Cocos nucifera* oil on serum cholesterol and triglyceride levels in male OF1 mice * Significant differences with respect to G2 group ($p <0.05$).

Figure 11 exposes the heat response time in the hot plate test in male OF1 male mice. a, b - Different letters means that there are differences between groups for $p < 0.05$.

The following are several examples of execution:

Example 1

The present example shows how to obtain the main components of the nutraceutical, which will be part of the different examples of execution of the final formulation. The composition and fundamental properties of the components of the emulsion and of the nutraceutical which support the essence of the invention are also shown.

Fatty acid composition tested

All the fatty acid-contributing raw materials included in the claims were evaluated for fatty acid content by gas chromatography (Tables 1, 2 and 3).
As can be seen, all the components provide the fatty acids that play a biological role in the nutraceutical and it is demonstrated that they respond to the composition shown in the claims, since they provide in their combination the medium chain fatty acids, omega 3, omega 6 and omega 9, and those that make possible the stability of the emulsion. In addition, raw materials containing phytosterols and triacylglycerols are used. These combinations of fatty acids are original and flexible, since by combining sources of at least 2 or 3 of the claimed groups, a high degree of complementarity is achieved.
As an example, one of the nutraceutical variants combines *Cocos nucifera* oil with *Cucurbita moschata* oil, soy lecithin (in the emulsion), *Curcuma longa* extract (in the emulsion), and *Sacha inchi* oil (in the emulsion).
In another variant, *Cucurbita pepo* seeds are combined with the other fatty acid sources described. A new nutraceutical variant is formed using the first combination described in this paragraph, but instead of soy lecithin, egg white is used.
Another example consists of the combination of the first variant described in the previous paragraph *(Cocos nucifera* oil with *Cucurbita moschata* oil, soy lecithin, *Curcuma longa* extract and *Sacha inchi* oil), but adding *Theobroma cacao* butter.

Table 1. Fatty acid composition of nutraceutical components

| Fatty acids | *Cocos nucifera* oil, sample 1 (%) | *Cocos nucifera* oil, sample 2 (%) | *Cocos nucifera* oil, industrial sample (%) | *Theobroma cacao* butter (%) |
|---|---|---|---|---|
| C8:0 | 6.17 ± 0.12 | 5.17 ± 0.06 | 5.57 ± 0.15 | - |
| C10:0 | 5.43 ± 0.12 | 4.87 ± 0.06 | 5.30 ± 0.0 | - |
| C12:0 | 43.47 ± 0.55 | 42.70 ± 0.46 | 44.20 ± 0.10 | - |
| C14:0 | 16.10 ± 0.36 | 17.63 ± 0.15 | 16.80 ± 0.10 | - |
| C16:0 | 8.13 ± 0.15 | 0.17 ± 0.06 | 0.47 ± 0.10 | 19.43 ± 0.21 |
| C16:1 | 0.03 ± 0.02 | 8.57 ± 0.06 | 7.93 ± 0.12 | 0.20 ± 0.00 |
| C18:0 | 2.50 ± 0.00 | 2.57 ± 0.15 | 2.30 ± 0.00 | 25.17 ± 0.42 |
| C18:1 | 6.77 ± 0.12 | 6.20 ± 0.10 | 6.50 ± 0.10 | 24.90 ± 0.56 |
| C18:2 | 1.83 ± 0.06 | 2.27 + 0.06 | 2.87 ± 0.29 | 2.07 ± 0.12 |
| C18:3 | 0.08 ± 0.04 | - | - | - |
| C20:0 | - | - | - | 0.73 ± 0.06 |
| C20:1 | - | - | - | - |
| Total | 90.50 ± 1.00 | 90.27 ± 0.91 | 91.97 ± 0.15 | 72.47 ± 1.27 |

Table 2. Fatty acid composition of nutraceutical components

| Fatty acids | Soy lecithin. (%) | *Plukenetia volubilis L* oil (*Sacha inchi*) (%) |
|---|---|---|
| C8:0 | - | - |
| C10:0 | - | - |
| C12:0 | 0.05 ± 0.05 | - |

(continued)

| Fatty acids | Soy lecithin. (%) | *Plukenetia volubilis L* oil (*Sacha inchi*) (%) |
|---|---|---|
| C14:0 | 0.13 ± 0.06 | 0.13 ± 0.06 |
| C16:0 | 11.33 ± 0.12 | 0.27 ± 0.12 |
| C16:1 | 0.08 ± 0.03 | 4.33 ± 0.06 |
| C18:0 | 3.23 ± 0.06 | 2.73 ± 0.21 |
| C18:1 | 11.57 ± 0.57 | 10.83 ± 0.23 |
| C18:2 | 35.93 ± 0.25 | 34.47 ± 0.46 |
| C18:3 | 4.17 ± 0.06 | 44.63 ± 0.12 |
| C20:0 | - | - |
| C20:1 | - | - |
| Total | 66.43 ± 0.42 | 97.43 ± 0.78 |

Table 4 shows the presence of phytosterols, triacylglycerols, monoacylglycerides, diglycerides and triglycerides determined in the raw materials.

Table 3. Fatty acid composition of nutraceutical components

| Fatty acids | Roasted *Cucurbita* seeds, (%) | Fatty acids | Roasted *Cucurbita* seeds, (%) |
|---|---|---|---|
| C8:0 | - | C17:0 | 26.44 ± 0.55 |
| C10:0 | - | C18:0 | 6.33 ± 0.99 |
| C12:0 | 0.00580 ± 0.0017 | C18:1 | 36.79 ± 0.73 |
| C13:0 | 0.04453 ± 0.0069 | C18:2 | 45.78 ± 0.83 |
| C14:0 | - | C18:3 | 0.28 ± 1.35 |
| C15:0 | 0.00486 ± 0.0010 | C19:0 | 0.00972 ± 0.00051 |
| C16:0 | 10.12 ± 0.05 | C20:0 | 0.34 ± 0.66 |
| C16:1 | 0.04376 ± 0.00239 | C20:1 | 0.03513 ± 0.0005 |
| | | C24:0 | 0.02133 ± 0.0069 |

All the examples cited above guarantee the presence of fatty acids, phytosterols and triacylglycerols described in at least three of the groups selected and described in the invention and guarantee an original combination of biological effects for the most diverse pathologies and disorders in which lipids are involved in general, highlighting the effect on neuronal diseases and disorders, including Alzheimer's Disease.

It was evidenced that *Cucurbita* seeds, even after roasting, retain a content of saturated fatty acids of 16.79 ± 2.5 %, of monounsaturated fatty acids of 37.15 ± 1.6 % and of polyunsaturated fatty acids of 46.06 ± 1.8 %. It was patented that this component provides fatty acids that complement those provided by other components of the nutraceutical, specifically the presence of pentadecanoic (C:15), heptadecanoic (C17:0), nonadecanoic (C19:0) and lignoceric (C24:0) fatty acids was determined, confirming the claims of the present invention.

Table 4 Composition of phytosterols, triacylglycerols (molecular species) and other fatty acids in the *Plukenetia volubilis L* oil and ethanolic extract of *Curcuma longa*

| Fatty acids | *Plukenetia volubilis L* oil, (%) | *Curcuma longa* ethanolic acid, (%) |
|---|---|---|
| β-sitosterol | 0.06114 | - |
| Campesterol | 00.424 | - |
| Stigmasterol | 0.02522 | - |
| Stigmastenol | 0.0183 | - |
| ECN 36 (LnLnLn) | 23.3 | - |

# EP 4 613 279 A1

(continued)

| Fatty acids | *Plukenetia volubilis L* oil, (%) | *Curcuma longa* ethanolic acid, (%) |
|---|---|---|
| ECN 38 (LnLnL) | 25.8 | - |
| ECN 40 (LnLL + LnLnP+LnLnO) | 20.1 | - |
| ECN 42 (LLL+OLLn+PLLn) | 20.3 | - |
| ECN 44 (LLO + OOLn +LLP+ POLn) | 7.6 | - |
| ECN 46 (OOL+ LnPP+ PLO) | 2.5 | - |
| ECN 48 (OOO+ POO) | 2.1 | - |
| ECN 50 (SSO) | 1.2 | - |
| Sum of triglycerides | - | 2.3 |
| Sum of diglycerides | - | 3.6 |
| Sum of monoacylglycerides | - | 1.2 |

In this example it is evident that the combination of the fatty acid sources of the present invention provides a nutraceutical composition previously undescribed for its diversity among nutraceutical products of natural origin. Specifically, *Cocos nucifera* and *Cucurbita* oils provide medium chain fatty acids; soy lecithin, *Plukenetia volubilis L* and *Cocos nucifera* oils, *Theobroma cacao* butter and *Cucurbita* seed provide longer chain fatty acids up to C18:2. Soy lecithin and *Plukenetia volubilis L* oil provide C18:2 and C18:3 fatty acids, while *Cucurbita* seed provides heavier chain fatty acids up to C24. Finally, *Plukenetia volubilis L* oil provides phytosterols and triacylglycerols.

In addition, the raw materials coconut oil and soy lecithin were determined for Fe, Cu, Zn, Mg and Ca content based on the relationship of these minerals to the development of Alzheimer's disease (Myhre O, Utkilen H, Duale N, Brunborg G, Hofer T. Metal Dyshomeostasis and Inflammation in Alzheimer's and Parkinson's Diseases: Possible Impact of Environmental Exposures. Oxidative Medicine and Cellular Longevity, 2013. ID 726954, 19 pp. doi.org/10.1155/2013/726954). The results are shown in Table 5 and confirm the contribution of micro- and macro-elements claimed in the present invention by the raw materials used.

Table 5. Mineral content in some of the raw materials of national origin

| Samples | Mineral content ($\mu$g/mL) | | | | |
|---|---|---|---|---|---|
| | Cu | Zn | Mg | Ca | Fe |
| *Cocos nucifera* oil | < 0.05 | 0.207 | 0.301 | 2.22 | 0.136 |
| Soy lecithin | < 0.05 | 1.076 | 281.3 | 11.78 | 4.56 |

The total count of microorganisms was also determined in these samples (Table 6). As can be seen, the raw materials tested meet the microbiological criteria for use in food.

Table 6. Total count of microorganisms in some of the raw materials used in the nutraceutical.

| Samples | | Indicator, UFC | | | | | |
|---|---|---|---|---|---|---|---|
| | | Total Count | *Entero-bactericiae* | *E. coli* | Heterotrophs | Yeasts | *Salmonella* spp |
| Soy lecithin | | 7.5 x 10 | <10 | <10 | 4 x10 | <10 | Absence |
| Oil | *Cocos nucifera* | 4 x 10 | <10 | <10 | <10 | <10 | Absence |

Other quality indicators of nutraceutical components, such as pH, loss on drying or total solids, are characteristic of a solid nougat or bar type product and guarantee durability (Table 7).

Table 7. Other indicators of some fatty acid-contributing raw materials

| Raw materials | pH | Loss on drying | Total Solids |
|---|---|---|---|
| Soy lecithin | 6.66 ± 0.16 | 0.76 ± 0.34 | 100 - 1.0 |
| *Cocos nucifera* | 4.35 ± 0.01 | 5.65 ± 8.74 | 95.2 - 9.41 |

It should be noted that the value of the acidity index of the sources of lipid compounds used in the present invention does not show evidence of their deterioration, which in all cases was lower than that required by the Codex Alimentarius (4 mg of KOH/g of oil) for edible fats; some examples of these values are shown below (Table 8).

Table 8. Acidity index in some of the oils used

| Raw materials | Acidity index, 4 mg KOH/g of oil |
|---|---|
| *Plukenetia volubilis L* oil | 0.78 |
| *Cocos nucifera* oil | 1.45 |

Obtaining *Stevia rubidaea* extracts.

Experiment 1:

Two hundred (200) mL of water and 20 g of previously dried *Stevia* leaves are taken, mixed and shaken at 300 1/min for 3 h at a temperature of 60 $^0$C. The extract is centrifuged to separate the solid. From the supernatant 100 mL are taken and 7.35 g of zeolite are added, shaken for 15 min and left in rest. Subsequently it is filtered through a filter plate. An extract with a refractive index of 1.343 was obtained. The *Stevia* came from the El Pitirre farm, Pinar del Rio Province, Cuba.

Experiment 2: Similar to the previous one, but activated carbon was used instead of zeolite.

Obtaining ethanolic extract of *Curcuma longa.* The reservoir root of this plant was obtained from the Medicinal Plants Farm in the municipality of Güines and from a farm in the municipality of Arroyo Naranjo, Cuba.

Experiment 1: 28 kg of *Curcuma longa* roots are taken, washed in water, drained and washed with chlorinated water (0.5 % chlorine solution), rinsed with running water and drained. The roots are ground in a knife mill and placed on trays at a rate of 2 kg each and placed in a convection drying oven at a temperature of 80 °C for 10 h; the resulting humidity is 7 %. Five (5) kg are obtained and pulverized in a high speed blade mill; 4.5 kg of turmeric powder with 10.05 % moisture content is obtained. Two hundred (200) g of the obtained powder are taken and 1,000 mL of ethyl alcohol at 95 % are added, which is shaken for 4 h at a temperature of 60 °C and the extract is obtained; this extract is centrifuged at 4300 rpm for 30 min to eliminate the precipitate and the supernatant is concentrated under vacuum at the same temperature to obtain 20 g of concentrate with 46 % of total solids. The resulting pH is 4.3.

The majority components of the extract obtained were qualitatively determined. For this purpose, the experimental extract was compared with a control commercial extract obtained from *Curcuma longa* roots from Portugal by thin layer chromatography. Chloroform:methanol (95:5) was used as mobile phase and silica as stationary phase. It was demonstrated that the three major components described for *Curcuma longa* extracts (curcumin, desmethoxycurcumin, bisdesmethoxycurcumin) were present in the extract. In addition, a standard curve of curcumin dissolved in acetone at concentrations from 1 μg/mL to 6 μg/mL was prepared and the absorbance was read at 425 nm. This curve allowed quantifying the concentration of curcuminoids in the extract obtained at laboratory scale, which was 16.81 mg/g of turmeric powder, representing 2 % of curcuminoids in the turmeric powder raw material. The literature reports that, in Indian turmeric cultures, curcuminoids represent between 2.3 % and 9.2 %. One of the components of the nutraceutical with antioxidant effect, claimed in the present invention, is *Curcuma longa* extract. The extract has a concentration of 8.43 mg/mL of curcuminoids.

Experiments 2 and 3: Similar to experiment 1; two batches of turmeric powder were obtained with humidity of 10.05 % and 6.11 %, respectively, extracts with 20.45 % and 12.9 % humidity, respectively, and pH of 4.4 and 4.3, respectively. The concentration of curcuminoids resulted in 8.43 mg/mL and 9.7 mg/mL, respectively. The reservoir roots of the plant were obtained from the Liliana Dmitrova Research Institute, Mayabeque, Cuba.

Experiment 3: a new batch of extract is prepared, according to the procedure described above. The results are shown in Table 9.

Experiment 4 on an industrial scale: 11 kg of *Curcuma longa* rhizome powder are taken and processed as described in the previous experiment. In a reactor, the powder is mixed with 110 L of 95 % ethyl alcohol, heated at 60 °C for 4 h, and vacuum is applied to evaporate the alcohol and concentrate the extract. The extract has a curcuminoid concentration of 17.51 %, including: curcumin - 9.12 %; desmethoxycurcumin - 5.21 % and bisdesmethoxycurcumin - 3.18 %. In addition, it contains 3.3 % triacylglycerides, 3.6 % diacylglycerides and 1.2 % monoacylglycerides.

Table 9. Curcuminoid extract

| Samples | Indicator | Mean | Standard Deviation |
|---|---|---|---|
| Unconcentrated extract | Average weight of liquid extract, g | 1025.32 | 84.43 |
| | Weight of liquid extract after centrifugation, g | 429.79 | 43.28 |
| | Average extraction yield with respect to the turmeric:alcohol mixture, % | 41.92 | 5.98 |
| | Average extraction yield with respect to the mass of turmeric (200 g), % | 214.90 | 21.63 |
| Concentrated extract | Weight of concentrated extract, g | 20.72 | 4.81 |
| | Average concentrate yield with respect to the original weight of the turmeric:alcohol mixture. | 2.02 | 0.38 |
| | Average concentrate yield with respect to the original weight of turmeric | 10.36 | 2.40 |
| | Moisture, % | 3.71 | 0.11 |
| | Total solids, % | 45.91 | 6.44 |
| | pH | 7.19 | 0.05 |
| | Concentration of curcuminoids in concentrated extract, mg/mL | 140 | |

Obtaining hydroalcoholic extract of *Curcuma longa*

Similar to the ethanolic extract of Experiment 4, but instead of using ethanol, a 70 % ethanolic solution in water is used and the rest of the parameters are maintained. The product is concentrated until the levels of curcuminoids described above are reached.

Obtaining emulsifying and foaming agents

The presence of air in products for oral consumption facilitates the swallowing process and contributes to form a structure with less hardness and resistance to chewing, an important aspect for a nutraceutical aimed at older adults. In this case the air forms part of the emulsion and is originated by the use of emulsifiers.

In the present invention, components never before described as emulsifiers are used, such as extracts of *Agave fourcroydes* and *Agave tequilana* for their saponin content. Intense stirring of extracts of these components not only introduces air into the emulsion, but also produces a flow in its aqueous matrix that drags protein macromolecules that may be present, for example, in egg white. If this flow is strong enough, it can unwind some of the less stable amino acidic tangles, forcing them to lose their original structure and to present the hydrophobic zones to the outside.

Given the proximity of the air bubbles, the denatured proteins and other components of the emulsions are adsorbed on the water-air interface, orienting their apolar zones towards the interior of the bubble and their polar zones towards the aqueous matrix, which drastically reduces the surface tension of the bubble, acting as an effective surfactant. Foaming is a thermodynamically adverse process that requires energy input in the form of mechanical energy supplied by the stirring. The nutraceutical components claimed in the invention incorporate air into the mixture in the final product, which improves its chewiness and gives it a softer texture.

*Agave fourcroydes:* Leaves and fruits of two henequen clones obtained from the Liliana Dmitrova Research Institute in the municipality of Quivicán, Cuba, were processed. They were washed and chopped and ground in a pilot cane grinding mill.

Twenty liters of sample were taken from each clone. It is considered that despite showing a low juice yield, an adequate foam expansion value can be obtained.

Table 10 shows a summary of the yields of each clone.

Table 10. Mass yields to obtain *Agave fourcroydes* extracts

| Parameter | Clone | |
|---|---|---|
| | CH-97 | CH 75 |
| Original weight, kg | 114 | 32.7 |
| Number of leaves | 108 | 84 |
| Average leaf weight, g | 105.55 | 0.38 |
| Fruit weight (pineapple), kg | 179 | 312.72 |

*Agave tequilana* is treated similarly to the method used for *Agave fourcroydes.*

For each 100 g of leaves, 2,000 mL of water are added, heated to 65 °C, shaken at a speed of 800 1/min for 4 h, the residue is decanted, and the supernatant is filtered. Dark, slightly opalescent extracts are obtained, with a total solids content of 1.2 % to 5 % and pH of 4.9. Foaming of the extracts is guaranteed even without very high concentrations. These values are very high considering the very low percentage of solids and guarantee that the extracts can be added at different concentrations and higher volumes in the nutraceutical, obtaining very stable foams that influence the organoleptic quality of the product, especially the chewiness, and make it possible to form 4-phase emulsions, including the gaseous phase.

Egg white as emulsifier - foaming agent

The overrun, a parameter that also characterizes emulsifiers, is calculated by the formula:

$$OV = 100 \times \frac{V_A}{V_L}$$

where: $V_A = V_F - V_L$; $V_A$ is the volume of air incorporated into the foam; $V_F$ is the volume of the foam and $V_L$ is the volume of liquid.

For an egg white:water ratio of 0.5:1 without the presence of any additional emulsifier, which is kept in agitation between one and three minutes, the value of the overrun is very high (83.3) and the value of the foam expansion is also very high (183.3 %).

Soy lecithin

The objective of the set of experiments was to determine the possibility of obtaining emulsions from *Plukenetia volubilis L (S. inchi)* oil, using soy lecithin as emulsifier.

Three emulsion compositions (1, 2 and 3) were tested in a blender using lecithin as emulsifier at different concentrations: oil, water and lecithin were placed in the blender at the desired concentration and the stirring process was carried out for 2 minutes; the blender speed was 12500 1/min. The emulsion formed was poured into a 100 mL graduated cylinder and the amount of emulsified oil and the stability of the emulsion was appreciated after 30 minutes.

- Composition 1 (Ratio water: oil: lecithin = 1:1: 0.016 V/V)
- Composition 2 (Ratio water: oil: lecithin=1:1: 0.012 V/V)
- Composition 3 (Ratio water: oil: lecithin=1:1: 0.008 V/V)

Ratio water:oil: 1:1 (30 mL of water: 30 mL of oil)

As can be seen, the foaming capacity was high for all the lecithin concentrations used, reaching 100 % for the one that included lecithin at a concentration of 1 %. The emulsion stability after 30 min was 100 % for all variants.

Egg lecithin is used in a similar way, since it has the same nature and properties as soy lecithin.

Table 11. Quality indicators of the emulsion obtained with soy lecithin as emulsifier

| Lecithin concentrations | Indicators | | |
|---|---|---|---|
| | FF (foam phase) | Foaming Capacity, % | Emulsion Stability, % |
| 0.6 % | 55.36 | 91.71 | 100 |
| 0.8 % | 55.48 | 91.73 | 100 |
| 1 % | 60.6 | 100 | 100 |

Obtaining micro-emulsion and nano-emulsion compositions.

Several oil-in-water emulsions were formed, using *Sacha inchi* oil as the oily phase, water as the aqueous phase and soy lecithin as the emulsifier.

Compositions CO24, CO25, CO28 and CO29 were made with soy lecithin, and CO26, CO27, CO30 and CO31 with egg white. Compositions CO24, CO26, CO28 and CO30, were formed using a 1:1 phase ratio; in compositions CO25, CO27, CO29 and CO31 the oily: aqueous phase ratio was 1:1.5. The stirring speed was 12500 1/min for compositions CO28, CO29, CO30 and CO31 and 15,000 1/min for the rest (CO24, CO25, CO26, CO27). The emulsion measurement times were 30, 60 and 90 min. As a result, 100 % emulsification and stability were obtained at all measured times, with the exception of composition CO30 and CO31, for which a stability of 99.15 % and 97 % was obtained after 60 min.

These experiments demonstrated that the basis for forming emulsions, according to the present invention, guarantees the formation of stable complex emulsions.

Since visual observation of the emulsion characteristics is not sufficient for its characterization, we proceeded to observe it under the optical microscope. When observing the samples of the compositions CO25, CO24 and CO28, well formed spherical droplets with a homogeneous size can be seen in general. It was appreciated that the particle size is around 1 $\mu$m and 2 $\mu$m for CO24 and CO28, respectively. For composition CO25 the particle size ranged between 3 $\mu$m and 7 $\mu$m. The size achieved is due to the fact that, at the agitation speed tested, especially in CO28, the energy consumed makes shearing possible and manages to reduce the size of the droplets to the sub-micrometer range. Homogeneity was observed in the size distribution in the field studied, which is indicative of the absence of flocculation.

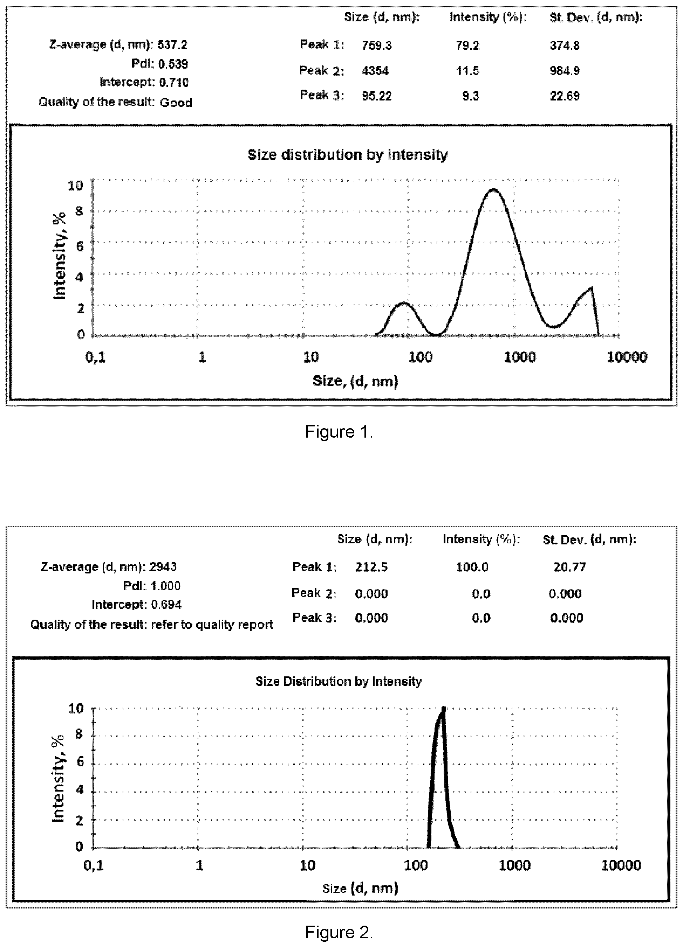

Figure 1 shows the droplet size distribution measured in a Z-ziser (laser beam scattering scanning) of the CO21 composition, in which a mean majority size of less than 1 micrometer (1 $\mu$) is observed. Figure 2 shows the size for composition CO27, with which an emulsion with droplet size in the nanometer order, between 100 and 200 nm, is achieved. These evidences indicate that the bioavailability of the components dissolved or dispersed in the oily and aqueous phases will have a higher bioavailability in the organism Already this phenomena has been widely described (Zou L.; Liu, W.; Liu, C.; Xiaob, H.; McClements D. J. (2015). Designing excipient emulsions to increase nutraceutical bioavailability: emulsifier type influences curcumin stability and bioaccessibility by altering gastrointestinal fate. The Royal Society of Chemistre .DOI: 10.1039/c5fo00606f).

Processing of *Cucurbita pepo* and *Cucurbita moschata* seeds. The seeds were acquired in the Seed Companies of Mayabeque and Artemisa provinces, Cuba.

Experiment 1:1,000 g of seeds are roasted to obtain 700 g, ground, mixed with water (280 mL), decanted, the shells that float on the surface are removed, the seeds are separated in a sieve, drained and dried in an oven at a temperature of 70 °C to 11 % humidity.

One hundred (100) g are ground in two types of mills: plastic cup and steel blades and sterilizable glass and steel blades.

Four hundred (400) mL of water were added and decanted for 2 h. The samples were sieved (250 mm) and drained for 5 min, obtaining weights of 122 g and 123 g, with no difference between the type of mill used. The sediments were dried in an oven at 70 °C for periods of 1.19 h and 1.49 h, respectively. Total grain and seed yields are 53 g/100 g and 54 g/100 g, respectively. The loss by desiccation shows values of 8.28 % and 5.97 %, respectively, a value that allows its conservation.

Table 12. Composition of whole and shelled unroasted seeds

| Raw materials | % | | | | | |
|---|---|---|---|---|---|---|
| | Loss on drying | Carbohydrates | Ashes | Chlorides | Proteins | Fat |
| Seeds in shell, mean | 8.90 | 15.04 | 4.73 | 0.24 | 30.17 | 41.35 |
| Standard deviation | 0.50 | 0.02 | 0.01 | 0.09 | 1.09 | 1.02 |
| Shelled seeds, mean | 5.7 | 18.38 | 4.18 | 0.14 | 30.77 | 41.43 |

(continued)

| Raw materials | % | | | | | |
|---|---|---|---|---|---|---|
| | Loss on drying | Carbohydrates | Ashes | Chlorides | Proteins | Fat |
| Standard deviation | 0.29 | 0.3 | 0.06 | 0 | 1.18 | 8.71 |

Experiment 2: 2,000 g of *Cucurbita pepo* seed were taken, roasted in a cast iron vessel to obtain 1952 g of roasted seed (97.6 % yield). They were ground in a plastic cup mill, mixed with 800 mL of water and left to decant for 1 h. The sediment was separated by a screen and allowed to drain. The weight of the drained wet seeds is 2083 g.

Drying was carried out in a convection oven at temperatures of 80 °C - 90 °C for 6 h. The final yield of the shell-free seeds was 1099 g for a 50 % yield. The mass was ground and passed through a 1 mm sieve, yielding 804 g of roasted, dried and ground seed (40.2 % yield with respect to the original seed mass). Desiccation loss was 2.88 %. Protein content was high (32 %).

Oil and water absorption capacity of *Cucurbita moschata* seeds.

The inclusion of the seed in the nutraceutical, not only aims to provide a content of proteins of vegetable origin and medium chain fatty acids with anti-inflammatory activity and other compounds with antioxidant activity, but also to ensure the absorption of other components such as oil emulsified in water, so it was of interest to determine the water and oil absorption capacity of this raw material.

The oil absorption capacity was determined as follows: 20 mL of oil were added to two grams of sample in 50 mL centrifuge tubes and shaken in the Vortex type shaker for one minute at room temperature. The mixture was centrifuged at 2100 g for 30 min. The results were expressed as grams of oil retained per gram of sample.

The oil absorption capacity was calculated according to the formula:

$$OAC = \frac{IMO - FMO}{MS}$$

where:

OAC - oil absorption capacity, g/g; IMOc - initial mass of oil, g
FMO - final mass of oil after centrifugation, g; MS - mass of the sample, g

Water absorption capacity

Water absorption capacity was determined in a similar manner: 20 mL of oil were added to two grams of sample in 50 mL centrifuge tubes and shaken in the Vortex type shaker for one minute at room temperature. Then the mixture was centrifuged for 30 min at 2100 g. The results were expressed as grams of water retained per gram of sample.

Water absorption capacity was calculated according to the formula:

$$WAC = \frac{IMW - FMW}{MS},$$

where:

WAC- water absorption capacity, g/g
IMW - initial mass of water, g
FMW - final mass of water after centrifugation, g
MS - mass of sample, g

Both water and oil absorption of the unshelled seeds showed adequate values (Table 13).

Table 13. Oil and water absorption capacity of unshelled seeds

| OAC (g of oil x g$^{-1}$ of sample) | | WAC (g of water x g$^{-1}$ of sample) | |
|---|---|---|---|
| $\overline{X}$ | DS | $\overline{X}$ | DS |
| 0,74 | 0,20 | 0,45 | 0,11 |

OAC: oil absorption capacity; WAC: water absorption capacity

In the case of oil absorption, the processed seed obtained a value of 0.74 g of oil x g-1 of sample and in the case of water about 50 % of its weight is absorbed. The results obtained for the seeds guarantee that they fulfill the function they are going to play in the final nutraceutical (to provide fatty acids absorbed in the mass and emulsified with the water without separation) and provided these exceptional and original characteristics already mentioned to the final product. It should be noted that this type of product, which by its action can be classified as a nutraceutical, can also be considered, due to its properties, as a type of functional food.

Amino acid composition of *Cucurbita* seeds

The composition of the seeds, according to the present invention, resulted with the presence of the essential amino acids (Table 14).

Table 14. Amino acid content of shelled, roasted and ground *Cucurbita* seeds

| Lysine | Methionine | Threonine | Phenylalanine | Histidine | Arginine |
|---|---|---|---|---|---|
| 12.24 ± 0.01 | 5.45 ± 0.013 | 7.16 ± 0.023 | 11.21 ± 0.05 | 6.75 ± 0.033 | 48.23 ± 0.04 |
| Valine | Isoleucine | Alanine | Tyrosine | Glutamic acid | Aspartic acid |
| 13.28 ± 0.03 | 10.31 ± 0.03 | 12.23 ± 0.03 | 16.02 ± 0.03 | 52.24 ± 2.11 | 28.15 ± 0.05 |
| Leucine | Glycine | Serine | Tryptophan | | |
| 23.48 ± 0.03 | 13.60 ± 0.03 | 11.27 ± 0.02 | 1.74 ± 0.05 | | |

These results indicate that technological processing did not affect its content. This example shows the content of tryptophan, an essential amino acid in many of the functions of the central nervous system.

The carbohydrate content of the seeds is low, which facilitates the assimilation of the medium chain fatty acids in the nutraceutical by the organism, converting them into ketone bodies, which are the only alternative source for the energetic support of neurons in patients with Alzheimer's disease and other disorders related to insulin resistance at the brain level. This resulted in: fructans: 4.85 % ± 0.13 %, sucrose: 3.18 % ± 0.10 %, glucose: 1.74 % ± 0.07 %.

In another variant, by adding *Apis mellifera* honey, the nutraceutical can be used in other conditions or diseases, or in general as a nutritional support with carbohydrate contribution by increasing the fructose content by 41.8 % and the glucose content by 34.6 %.

In another variant, by adding sugar, the nutraceutical can be used in other conditions or diseases, or in general as nutritional support in subjects who do not suffer from or are not at risk of diabetes with carbohydrate intake, by increasing the sucrose content by 100 g/100 g.

Table 15 shows the results of the vitamin content in the main contributor of vitamins in the nutraceutical, although not the only one, which alone guarantees a very important level of vitamins that play a fundamental role in the prevention or for the treatment of very diverse conditions, especially Alzheimer's disease and memory deficit caused by other disorders or diseases, among which B12, C, D, E, B6 and folic acid stand out.

The diversity of vitamins provided by the components of the nutraceutical allows its use to treat nutritional disorders, such as avitaminosis, fatigue, stress, among other conditions.

Table 15. Vitamin content in the shelled, roasted and ground seeds of *Cucurbita* obtained according to the present invention.

| Vitamins | UI/100 g | mg/100 g | μg/100 g |
|---|---|---|---|
| Retinol Acetate (A) | 6.1 | | |
| Cholecalciferol (D3) | 0.001 | | |
| Thiamine (B1) | | 0.5 | |

(continued)

| Vitamins | UI/100 g | mg/100 g | μg/100 g |
|---|---|---|---|
| Roboflavin (B2) | | 0.18 | |
| Nicotinamide (B3) | | 5.2 | |
| Pyridoxine (B6) | | 0.1 | |
| Biotin (B7) | | 0.002 | |
| Folic acid (B9) | | 58.5 | |
| Cyanocobalamin (B12) | | 0.03 | |
| Vitamin C | | 11.3 | |
| $\alpha$-Tocopherol (E) | | 2.2 | |
| Menadione (K3) | | | 7.3 |

Example 2

Nutraceutical variant 1 (V1)

To prepare 2800 g of nutraceutical

Preparation of the emulsion components:

To prepare the emulsion, the aqueous liquid phase consisting of 250 g of water, equivalent to 8.93 % m/m of the nutraceutical, is selected and heated to 100 °C and 780 g of sucrose (sugar) is added as sweetener (equivalent to 27.86 % m/m of the nutraceutical), stirred until completely dissolved and brought to a temperature of 40 °C. Soy lecithin is selected as emulsifying agent (5 g, representing 0.178 % m/m with respect to the total mass of the nutraceutical).

The components are stirred and mixed at stirring speeds of 15,000 1/min to obtain a microemulsion. When these components are stirred, the gas phase (air) is incorporated, which represents 19 % v/v of the emulsion and 8.8 % of the total volume of the nutraceutical. For this stage of preparation, the emulsion is kept at a temperature of 25 °C.

Subsequently, the oily phase of the emulsion is prepared with soluble solids in it, in this case ethanolic extract of *Curcuma longa.* The oily phase is composed of 250 g of *Plukenetia volubilis* oil (ratio of 8.93 % m/m with respect to the mass of the emulsion) and 15.05 g of ethanolic extract of *Curcuma longa* (0.54 % m/m with respect to the mass of the nutraceutical). In this preparation phase, the emulsion is kept at a temperature of 25 °C. In this way, a 4-phase emulsion variant is obtained (aqueous, oily, air and suspended or solubilized solid), specifically aqueous:oily:gaseous:solids suspended in the oily.

In parallel, the *Cucurbita moschata* seed mass is prepared. The seeds are processed shelled, ground and roasted. The seeds are taken out at a temperature of 80 °C for 60 min. Roasting of the seeds is carried out at a temperature of 130 °C. The seeds are milled in a blade mill and particles of a size between 10 μm to 30 μm are obtained.

The emulsion is mixed with the seed mass to form a food paste type macro-emulsion. To the mass of the shelled seeds already roasted and ground (1500 g, that represents 60 % m/m of the nutraceutical mass) the emulsion is added in quantity of 1423.05 g (48.7 % m/m with respect to the total mass of the nutraceutical). The addition is carried out under manual stirring to ensure homogeneous mixing and the least loss of entrapped air. The final emulsion composition of this V1 nutraceutical variant contains 6 components of different nature (water, air, oil, sugar, soy lecithin and *Curcuma longa* extract).

In this variant, more than one component performs various biological or functional functions, including *Plukenetia volubilis* oil, *Curcuma longa* extract and soy lecithin, as described above for each component.

The nutraceutical obtained is presented in the form of bars which are cut into individual pieces with a mass of 40 g, covered with waterproof paper and stored at temperatures of 10 °C.

Variant V1 contains 3 different sources of fatty acids, phytosterols and triacylglycerols, including *Cucurbita moschata* seeds, *Plukenetia volubilis* oil and soy lecithin. The final nutraceutical product (V1) contains three components with antioxidant activity, including *Cucurbita moschata* seeds, *Curcuma longa* extracts and soy lecithin, as part of the emulsion. The final composition of the nutraceutical includes one source of hypoglycemic compounds, specifically *Cucurbita moschata* seeds. In this variant V1, different sources of anticholesterolemic compounds are used, specifically *Plukenetia volubilis* oil and soy lecithin. To make up the nutraceutical, sources of anti-inflammatory compounds are used, including those contained in the seeds of *Cucurbita moschata.*

In the composition of the V1 variant, protein sources of vegetable origin are used, such as the seeds of *Cucurbita*

*moschata.* The formulation of the V1 nutraceutical contains components that provide vitamins, macro- and micro-elements in sufficient quantities to supply different deficiencies of them, or to maintain the necessary physiological level, specifically *Cucurbita seeds* that provide vitamins A, K, E and folic acid; soy lecithin that provides choline; ethanolic extract of *Curcuma longa* that provides Na, K, Mg, Ca, Fe. The nutraceutical composition contains substances that provide appropriate organoleptic characteristics for the subjects and patients who consume it, such as the following: *Plukenetia volubilis* oil, besides masking the flavor of the *Curcuma longa* extract, gives the nutraceutical a pleasant consistency, increases adhesiveness, makes the nutraceutical easier to swallow and facilitates chewing. On the other hand, the sucrose used provides sweetness to the composition, which eliminates or diminishes the characteristic flavor of the *Curcuma longa* extract.

In the final formulation, *Plukenetia volubilis* oil is included, which contains fatty acids of 6 to 12 carbons and from which ketone bodies are obtained, which, in the face of insulin resistance in patients with Alzheimer's disease, provides energy to the neurons.

The nutraceutical composition obtained according to V1 and by the results of Example 1, can be used for the prevention or treatment of diseases, disorders or syndromes comprising a cognitive deficit, such as Alzheimer's Disease, mental, behavioral and developmental disorders, including depressive and nervous system disorders, all of this linked to the presence of medium chain and omega fatty acids, components with antioxidant, anti-inflammatory and hypoglycemic activity and the presence of Zn, Ca and vitamins, especially A, B and C.

Likewise, the nutraceutical is useful for disorders of the digestive system, due to its vitamins, proteins, antibacterial, antifungal, vermicidal and lipid compounds. The nutraceutical is used for the treatment of diseases and disorders of the immune and endocrine systems, due to its content of Zn in certain amino acids, especially tryptophan, and the anti-inflammatory substances of its components, among other elements with biological activity. Due to the capacity of its components and the product itself to lower triglycerides and cholesterol, in addition to the aforementioned factors, it is used for disorders of the circulatory system, cardiovascular and cerebrovascular diseases.

The nutraceutical can be used in bacterial, fungal and parasitic infectious diseases, due to the antibacterial, antifungal and vermicidal compounds of its components and due to its proven stability. Due to its amino acid composition, its micro- and macro-elements, vitamins and antioxidants, among other components, it is used for the treatment of oncological and blood diseases. In general, its content in fats, carbohydrates, proteins, vitamins and minerals is highly recommended to combat tiredness, fatigue and malnutrition.

It is recommended to consume the nutraceutical twice a day, in portions of 40 g, every other day, for a period of one month, resting from 3 days to 30 days, repeating the administration cycle.

The results of different evaluations of the nutraceuticals obtained according to variant 1 are presented below.

Chemical composition of the nutraceutical

The results of the evaluation of micro- and macro-elements are shown in Table 16.

Table 16. Composition of micro- and macro elements of the nutraceutical

| Element/ sample | Ca, % | Cu, mg/kg | Zn, mg/kg | Fe, mg/kg | Mn, mg/kg | K, mg/100 g | Na, mg/100 g |
|---|---|---|---|---|---|---|---|
| Nutraceutical | 0.5 | 6.45 | 52.5 | 87 | 25 | 604.63 | 31.86 |

As can be seen, there is a mineral content that is in the range of products intended to supply the organism with sufficient doses of micro- and macro-elements, especially Zn, which is found in quantities necessary to treat Alzheimer's disease.

Nutraceutical Variant 2 (V2)

To prepare 2253 g of nutraceutical

Preparation of the emulsion components:
To prepare the emulsion, the aqueous liquid phase consisting of *Stevia rebaudiana* extract as sweetener, containing the steviosides dissolved in water (187 g of extract diluted in 63 g of water), corresponding to 11.1 % m/m of the nutraceutical) is selected and 110 g of water are added (corresponding to 4.88 % m/m of the nutraceutical). The extract is obtained as described in example 1 (experiment 2 of obtaining *Stevia* extract), which consists, in summary, of placing the previously dried leaves in water, mixing, shaking for 3 h at 60 $^0$C, centrifuging, adding activated carbon, shaking, leaving to rest and filtering.

As emulsifier, and at the same time foaming agent, 128 g of egg white (5.68 % m/m with respect to the mass of the nutraceutical) are added.

The components are stirred and mixed at a stirring speed of 15,000 1/min to obtain a microemulsion. When these components are stirred, the gaseous phase (air) is incorporated in a volume of 30 % v/v of the emulsion, which represents 10 % of the total volume of the nutraceutical. For this preparation stage, the emulsion is kept at a temperature of 25 °C.

Subsequently, the oily phase of the emulsion is prepared with soluble solids in it, in this case ethanolic extract of *Curcuma longa.* The oily phase is composed of 250 g of *Plukenetia volubilis* oil (11.1 % m/m with respect to the mass of the emulsion) and 15.05 g of ethanolic extract of *Curcuma longa* (6.64 % m/m with respect to the mass of the nutraceutical). In this preparation phase, the emulsion is kept at a temperature of 25 °C.

In this way a 4-phase emulsion variant is obtained (aqueous, oily, air and suspended or solubilized solid), specifically: aqueous:oily:gaseous:solubilized solids in the aqueous and suspended solids in the oily.

In parallel, the *Cucurbita moschata* seed mass is prepared. The seeds are processed shelled, ground and roasted. The seeds are taken out at a temperature of 80 °C for 60 min. Roasting of the seeds is carried out at a temperature of 130 °C. The seeds are milled in a blade mill and particles of a size between 10 $\mu$m to 30 $\mu$m are obtained.

The emulsion is mixed with the seed mass to form a food paste type macro-emulsion. To the mass of shelled seeds, already roasted and ground (1500 g, which represents 66.58 % m/m of the nutraceutical), 753.05 g of the emulsion are added (33.42 % m/m with respect to the total mass of the nutraceutical). The addition is carried out under manual stirring to ensure homogeneous mixing and the least loss of entrapped air. The mixing of the microemulsion with the rest of the components, to form a macroemulsion with all the components of the final formulation of the nutraceutical, is carried out manually by applying enveloping movements.

Subsequently, the emulsion mixture is cooled with the mass of seeds and oils that make up the nutraceutical obtained with all its components, initially at room temperature and later at a temperature of 8 °C, for an interval of 5 h to 24 h. Once the mass is cooled, it is cut and portioned.

The final composition of the emulsion of variant V2 contains 6 components of different nature (water, air, *Plukenetia volubilis* oil, *Stevia extract,* egg white, and *Curcuma longa* extract).

In these two variants, more than one component performs various biological or functional functions, such as *Plukenetia volubilis* oil, *Curcuma longa* extract, soy lecithin, and egg white, as described above for each component.

The nutraceutical obtained is presented in the form of bars, which is cut into individual pieces with a mass of 40 g, covered with waterproof paper and stored at temperatures of 10 °C.

In variant V2, the nutraceutical contains 3 different sources of fatty acids, phytosterols and triacylglycerols, such as *Cucurbita moschata* seeds, *Plukenetia volubilis* oil and egg white. The final nutraceutical product of variant 2 (V2) contains three components with antioxidant activity: *Cucurbita moschata* seeds, *Curcuma longa* extracts as part of the emulsion and *Stevia rebaudiana* extract, also as part of the emulsion.

A source of hypoglycemic compounds, specifically *Cucurbita moschata* seeds, is included in the final composition of the nutraceutical. In this V2 variant, different sources of anti-cholesterolemic compounds are used, specifically *Plukenetia volubilis* oil and egg white. To make up the nutraceutical, sources of anti-inflammatory compounds are used, including *Stevia rebaudiana* extract, which is part of the emulsion, and *Cucurbita moschata* seeds. In the composition of the V2 variant, protein sources of plant origin, such as *Cucurbita moschata* seeds, are used.

This V2 variant of composition contains components that provide vitamins, macro- and micro-elements in sufficient quantities to supply different deficiencies of them, or to maintain the necessary physiological level, specifically the seeds of *Cucurbita,* which provide vitamins A, K, E and folic acid; the soy lecithin that provides choline and the ethanolic extract of *Curcuma longa* that provides Na, K, Mg, Ca and Fe.

The nutraceutical composition contains substances that provide organoleptic characteristics such as easy chewing and swallowing without adhering to the upper alimentary tract for the subjects and patients who consume it, specifically *Plukenetia volubilis* oil, which besides masking the flavor of the *Curcuma longa* extract, gives the nutraceutical a pleasant consistency, increases adhesiveness, makes the nutraceutical easier to swallow and facilitates chewing. On the other hand, the *Stevia rebaudiana* extract used in V2 provides a sweetness to the composition that eliminates or diminishes the characteristic flavor of the *Curcuma longa* extract.

*Plukenetia volubilis* oil containing 6 to 12-carbon fatty acids is included in the final formulation.

The nutraceutical composition obtained according to V2 and by the results of Example 1, can also be used for the prevention or treatment of diseases, disorders or syndromes comprising a cognitive deficit, such as Alzheimer's Disease, mental, behavioral and developmental disorders, including depressive and nervous system disorders, all linked to the presence of medium chain and omega fatty acids, components with antioxidant, anti-inflammatory and hypoglycemic activity, the presence of Zn, Ca and vitamins, particularly A, B and C. It is also effective in treating digestive system disorders due to the presence of vitamins, proteins, antibacterial, antifungal and vermicidal compounds and suitable lipids.

The nutraceutical is suitable for the treatment of diseases and disorders of the immune and endocrine systems due to the Zn content in certain amino acids, especially tryptophan, and the anti-inflammatory substances of its components, among other components with biological activity. Due to the capacity of its components and the product itself to lower triglycerides and cholesterol, in addition to the aforementioned factors, it is suitable for disorders of the circulatory system; cardiovascular and cerebrovascular diseases.

It can be used in bacterial, fungal and parasitic infectious diseases due to the antibacterial, antifungal and vermicidal compounds of its components and due to its proven stability. Its amino acid composition, micro- and macroelements, vitamins and antioxidants, among other components, make it suitable for the treatment of oncological and blood diseases. In general, its content in fats, carbohydrates, proteins, vitamins and minerals is highly recommended for tiredness, fatigue and malnutrition.

It is recommended to consume the nutraceutical 2 times a day in portions of 40 g, every other day, for a period of one month, with a break of 3 days to 30 days, repeating the administration cycle.

The results of different sensory evaluations of the nutraceutical obtained according to variants V1 and V2 are presented below.

Sensory evaluation of the nutraceutical obtained.

It was performed with the help of a panel of 8 untrained members who evaluated, according to a hedonic scale (from 0 to 5), sensory characteristics such as: odor, flavor, color, consistency, chewiness, elasticity, rigidity, adhesiveness and homogeneity. The scales received the following ratings for all trials and all variants: "very pleasant" (5), "pleasant" (4), "acceptable but characteristic" (3), "not very acceptable" (2), "not acceptable" (1) and "completely unpleasant" (0).

In addition, the presence of foreign matter, oil separation, presence of lumps, slipperiness to the palate and fibrous structure were evaluated.

The odor attribute obtained a value (median) of three points corresponding to "acceptable but characteristic", in this case due to the presence of turmeric extract, which is characteristic of this spice. Flavor and color obtained the same rating of "acceptable but characteristic", also due to the presence of turmeric and a somewhat bitter aftertaste for V2, due to the aqueous extract of *Stevia*. Overall, both were "acceptable" and, according to the panel, not unpleasant.

Consistency was rated 4. There was no deformation in any of the variants. Chewiness (3.25), rigidity (3) and adhesiveness (5), parameters that characterize the mechanical properties, were rated "adequate". These characteristics were, in the opinion of the research team, the most complex to guarantee, but very relevant to consider them necessary for the elderly. In particular, easy chewability and easy swallowing, as explained above.

Homogeneity obtained a value of "acceptable" (3), which is largely due to the inclusion of most of the components in the form of emulsion (water, oil, lecithin, sucrose or *Stevia* extract, turmeric extract).

In general, the selection of the nutraceutical components, the methods used to process them, the procedures for obtaining the emulsion and the formulation of the final product, made it possible to obtain two experimental variants considered, both for their composition and their organoleptic properties, as characteristics for this type of nutraceutical in the form of nougat or solid bar, with all the design requirements for the prevention and treatment of Alzheimer's disease and other conditions.

Effect of components and nutraceutical on antioxidant activity in aged OF1 mice.

Experimental protocol

Male OF1 mice of 64 weeks of age (15 to 16 months) treated with the product and whose weight range was between 45 g and 50 g were compared to animals of the same age and young animals of 4 to 5 months of age without any treatment. During the whole experimental procedure, the animals were kept in a conventional room with a controlled environment (minimum security barrier type IV), with a temperature of 21 $\pm$ 3° C and relative humidity between 40 % and 70 %, with 18 air changes per hour, 100 % injection of outside air with 85 % filtration. In addition, the animals had 12 hours of light and 12 hours of automatically controlled darkness and free access to water and food. The animals were grouped at a rate of 5 per polypropylene box, with bedding of desmoled sugarcane bagasse. In all cases, environmental enrichment was applied to minimize aggressiveness among group members within each box (cardboard and plastic cones).

Treatment

The aged animals were distributed in 4 groups (G2 to G5) and the young animals were grouped in G1. In both control groups, aged (G2) and young (G1), a 10 % Tween 20 solution in 0.9 % NaCl was used as a vehicle intragastrically (i.g.). Group G3 received the ethanolic extract of *Curcuma longa,* as described in the present invention, at a dose of 300 mg/kg i.g. administered in a volume of 1.1 mL. Groups G4 and G5 received orally the *Cucurbita moschata* seed component and the nutraceutical of variant 1 (V1), respectively, as supplementation to the normal diet at a rate of 3 g/animal.

Regardless of the group, the treatment was administered for 5 weeks from Monday to Friday. The response to treatment was analyzed by TBARS indicators, such as lipid oxidative damage and total antioxidant capacity in brain homogenate.

Parameters evaluated

The animals were sacrificed by cervical dislocation and five animals from each group underwent brain extraction. Subsequently, brain homogenates were prepared in Tris buffer at pH 7.6 containing 0.32 M sucrose, 10 mM Tris and 1 mM EDTA and differential centrifugation was performed until the post-mitochondrial fraction was obtained, according to the method described by Uwe and Von Hagen in 2009 (Uwe, M. and J. Von Hagen (2009): Isolation of subcellular organelles and structures. Methods Enz. 463: 305-326). The supernatant was dispensed into 1 mL aliquots, which were stored at -80 °C. Total antioxidant capacity was measured in these homogenates according to the instructions of the TAS reagent kit (RANDOX, England). In addition, the quantification of oxidative damage to lipids was performed using the Thiobarbituric Acid Reactive Species (TBARS) method according to Okawa et al. in 1979 (Okawa, O., N. Ohishi and K. Yagi (1979): Assay of lipid peroxides in animal tissues by the thiobarbituric acid reaction. Anal Biochem. 95 (2): 351 - 58). The method consisted of the spectrophotometric measurement of substances reacting with thiobarbituric acid (TBA), which is based on the stoichiometric reaction of two molecules of TBA with one molecule of malondialdehyde (MDA), which is one of the products of the chain reaction called lipid peroxidation. The reaction of MDA with TBA forms a pink compound which has its absorbance maximum (ABS) at a wavelength of 532 nm in acidic solution. TBARS were determined from the molar extinction coefficient of MDA ($\xi$ = 155 mM-1cm-1) and the protein concentration in the tissue homogenate which was determined by the modified Lowry's method for biological tissues according to Upreti et al. in 1988 (Upreti, G.C., R.A. Ratcliff and Riches P.C. (1988): Protein Estimation in Tissues Containing High Levels of Lipid: Modifications to Lowry's Method of Protein Determination. Analytical Biochemestry. 168: 421-427).

Results

Table 17 shows that the oral formulation of the nutraceutical, at a rate of 3 g/animal administered for 5 weeks from Monday to Friday, was able to reduce the oxidative damage that appears in the brain of the aged mice; even a significantly lower value was obtained than in the young controls. In the G2 group, a significant increase in lipid damage in the brain was obtained with respect to the G1 group. In the case of the groups supplemented with *Curcuma longa* extract and *Cucurbita moschata* seed, there was a significant decrease in damage with respect to the aged controls and no differences were found with respect to the young control animals.

The decrease in oxidative damage to lipids in the brain homogenate found in groups G3 to G5 can be considered a beneficial effect of the treatment, since the literature describes this damage as a physiological event associated with the aging process and neurodegenerative diseases reported by Myhre et al, in 2013 (Myhre, O., H. Utkilen, N. Duale, G. Brunborg and T. Hofer (2013): Metal dyshomeostasis and Inflammation in Alzheimer's and Parkinson's diseases: Possible impact of environmental exposure. http://dx.doi.org/10.1155/2013/726954; accessed: 8-07-2016) and therefore decreasing it constitutes a benefit. Although it should be noted that the group supplemented with the nutraceutical showed the best effects in this regard.

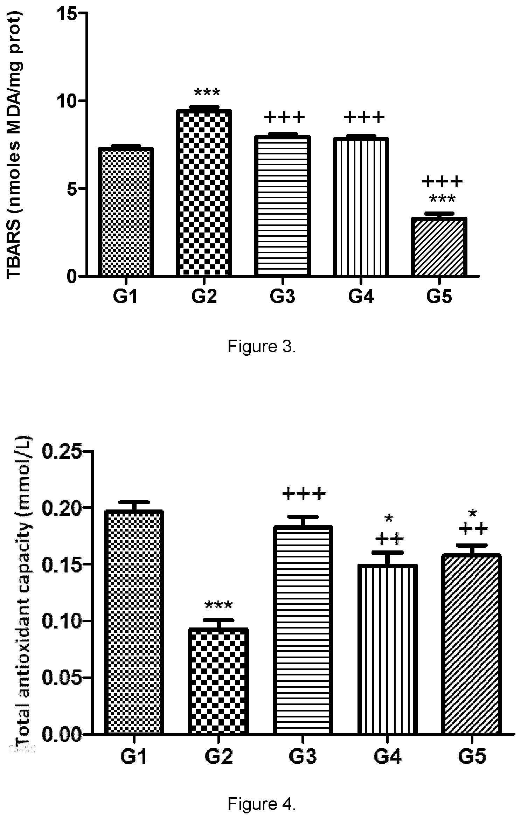

The determination of total antioxidant capacity in brain homogenate showed that the G3 group, which received supplementation with *Curcuma longa* extract, was the only one that did not show significant differences with the group of young animals that received vehicle (G1) (Figure 3). In the case of the G4 and G5 groups, they had a significantly higher antioxidant capacity than the aged controls. This result corroborates the antioxidant effect reported for extracts of similar composition to *Curcuma longa* extract.

Although the best results were obtained for the G3 group with respect to this variable, the treatment with the nutraceutical had more effects with respect to overall antioxidant activity, including both variables analyzed.

These results demonstrate that it is not obvious that the combination of all sources of natural components of natural origin, but of different chemical nature, overcomes the antioxidant effect of the separate components, which has already been described in the available scientific literature. The combination of these substances together with the other components, such as micro-macro-elements and vitamins, in a very original way, form a previously undescribed solution. Surprisingly, a lower oxidative damage to lipids in brain homogenate was evidenced in the group of aged mice treated with the nutraceutical than that found in the young animals.

With respect to the total antioxidant activity, it was also possible to demonstrate a statistically significant improvement of the activity in the group of mice treated with the nutraceutical with respect to the untreated aged mice. This other effect has not been previously described in animal models aged on a product that conjugates the components described in the present invention.

From these first two variants, once the protective and therapeutic action of the nutraceutical has been demonstrated, new variants of composition and changes in the process parameters are designed, which in principle did not significantly vary the original design concept of the nutraceutical, since components were chosen that have common compositions and biological activities. Thus, egg lecithin can be used to replace soy lecithin as an emulsifier and to form foams, since their chemical composition and biological properties are similar.

According to the results of the evaluation of the biological activity in experiments in animal models, the product can be applied for the prevention or treatment of diseases in which oxidative stress plays a relevant role, including Alzheimer's disease, other mental disorders such as Parkinson's, cardiovascular, cerebrovascular, oncological and blood diseases.

Table 17. Effect of ethanolic extract of *Curcuma longa, Cucurbita moschata* seeds and nutraceutical on antioxidant activity

| Oxidative damage | G1 (vehicle) young | G2 (vehicle) aged | G3 (*Curcuma longa* extract; dose: 300 mg/kg i.g.) | G4 (*Cucurbita moschata* seed; 3g/animal; oral route) | G5 (nutraceutical, 3g/animal, oral route) |
|---|---|---|---|---|---|
| TBARS, nmoles MDA/mg prot | 7.244 ± 0.465 | 9.389 ± 0.750*** | 7.921 ± 0.538 +++ | 7.813 ± 0.4640 +++ | 3.265 ± 0.896 ++ +,*** |
| Total antioxidant capacity, mmoles/L | 0.196 ± 0.021 | 0.093 ± 0.018*** | 0.182 ± 0.023 +++ | 0.149 ± 0.026 * ++ , | 0.158 ± 0.023 * + + , |
| * Significant differences with respect to G1. + Significant differences with respect to G2* | | | | | |

Example 3

Nutraceutical Variant 3 (V3)

Preparation of the emulsion components.

To prepare the emulsion, the aqueous liquid phase consisting of 250 g of water, equivalent to 26.82 % m/m of the nutraceutical, is selected, heated to 100 °C and 218 g of sucrose (sugar) is added as sweetener (equivalent to 23.39 % m/m of the nutraceutical). The sugar-in-water syrup is prepared by heating the mixture in sugar:water ratio without stirring, at a temperature of 80 °C, until complete dissolution of the sugar, then cooled to a temperature of 40 °C.

Egg white is selected as an emulsifying agent and an amount of 32 g is added (ratio of 3.43 % m/m with respect to the total mass of the nutraceutical) and 15.05 mL of *Curcuma longa* aqueous extract is added (ratio of 1.61 % m/m with respect to the total mass of the nutraceutical).

The components are stirred and mixed at a stirring speed of 10,000 1/min to obtain an emulsion. The oily phase is added with 80 g of *Plukenetia volubilis* oil (ratio of 8.58 % m/m with respect to the mass of the nutraceutical). No oil-soluble substances are added to the *Plukenetia volubilis* oil phase beforehand. When these components are agitated, the gaseous phase (air) is incorporated, which represents 50 % of the emulsion volume (31.92 % of the total volume of the nutraceutical). For this emulsion preparation stage, a temperature of 25 °C is maintained.

The final composition of the emulsion of variant 3 (V3) contains five components of different nature (water, air, oil, sugar, egg white).

In these two variants, more than one component plays various biological or functional roles, described in the description of the invention dedicated to the role of each component and its biological activity. At this stage the emulsion is kept at a temperature of 25 °C. In this way a 4-phase emulsion variant is obtained: aqueous:oily:gaseous:solids solubilized in the aqueous.

In parallel, the mass of *Cucurbita pepo* seeds is prepared, which are processed shelled, ground and roasted, as described in the experiment of Example 1. In summary, 2,000 g of seeds are taken, roasted in a cast iron vessel, ground in a plastic cup mill, mixed with water and left to decant for 1 h. The sediment is separated by a grid, and the seeds are separated by a sieve from the sediment. The sediment is separated by grid and left to drain. The seed mass is dried in a convection oven at a temperature of 80 °C - 90 °C for 6 hours. The mass is ground and sieved through a 1 mm sieve to obtain a roasted, dried and ground seed mass with a protein content of 32 %.

The emulsion is mixed with the seeds to form a food paste type macro-emulsion: to the mass of 500 g of shelled, ground and roasted seeds (53.6 % m/m with respect to the mass of the nutraceutical) 432 g of the emulsion obtained are added, equivalent to 46.4 % m/m with respect to the total mass of the nutraceutical. The addition is carried out under manual stirring to ensure homogeneous mixing and the least loss of entrapped air. The mixing of the emulsion with the seeds to form a macroemulsion with all the components of the final formulation of the nutraceutical is carried out manually by applying enveloping movements.

Subsequently, the macroemulsion of the nutraceutical macroemulsion obtained with all the components is cooled, initially at room temperature and later at a temperature of 4 °C, for an interval of 24 h. Once the mass is cooled, it is cut and portioned. The nutraceutical obtained is presented in the form of bars that are covered with impermeable paper and stored at temperatures of 4 °C.

In variant V3, the nutraceutical contains three different sources of fatty acids, phytosterols and triacylglycerols, including *Cucurbita pepo* seeds, *Plukenetia volubilis* oil and egg white. The final nutraceutical product according to variant 3 (V3) contains two components with antioxidant activity, which are *Cucurbita pepo* seeds and *Plukenetia volubilis* oil. The final nutraceutical composition, according to V3, includes a source of hypoglycemic compounds, specifically *Cucurbita pepo* seeds. In this variant, a source of anticholesterolemic compounds is used, specifically *Plukenetia volubilis* oil. A source of anti-inflammatory compounds, specifically *Cucurbita pepo* seeds, is used to make up the nutraceutical.

In the composition of the V3 variant, protein sources from different origins are used, including *Cucurbita pepo* seeds, of vegetable origin, and egg white, of animal origin. The formulation of nutraceutical V3 contains components that provide vitamins, macro- and micro-elements in sufficient quantities to supply different deficiencies of them or to maintain the necessary physiological level, specifically the seeds of *Cucurbita pepo* provide vitamins A, K, E and folic acid.

The nutraceutical composition contains substances that provide organoleptic characteristics required by the subjects and patients who consume it, specifically, *Plukenetia volubilis* oil provides the nutraceutical with a softer consistency, not so rigid to the palate, increases the intrinsic adhesiveness of the product but makes it more digestible and improves chewability, requiring much less effort in chewing. The egg white traps a very significant amount of air (high overrun) that makes the consistency of the nutraceutical softer to the palate and causes the intensity of the color to decrease. On the other hand, the *Stevia rebaudiana* extract used in V4 provides sweetness to the composition, appropriate to the palate.

The final formulation includes *Plukenetia volubilis* oil containing 6 to 12 carbon fatty acids.

The organoleptic evaluation based on a maximum of 5 points, carried out by a panel of 14 evaluators familiar with the product, yielded the following results: "pleasant" smell (4,07); "pleasant" taste, with a slight oil taste (3,78); "adequate" consistency (4,07); "good elasticity" (4,36); "easy masticability", but with particles that are more solid (3,36); "creamy" consistency (4); adhesiveness "adequate", practically does not adhere to the mouth (4.43); homogeneity: "homogeneous" (4,86).

Nutraceutical variant 4 (V4)

Preparation of the components of the emulsion

To prepare the emulsion, the aqueous liquid phase composed of the *Stevia rebaudiana* extract, containing the steviosides dissolved in water (187 g of extract diluted in 63 g of water, corresponding to 25.33 % m/m of the nutraceutical) is selected as the sweetener and 110 g of water are added (corresponding to 11.14 % m/m of the nutraceutical).

As an emulsifier, and at the same time foaming, 32 g of egg white are added (32.42 % m/m with respect to the mass of the nutraceutical) and 15.05 mL of the aqueous extract of *Curcuma longa* are added (ratio of 1.52 % m/m with respect to the total mass of the nutraceutical).

The components are stirred and mixed at a stirring speed of 10,000 1/min to obtain an emulsion. The oily phase is added with 80 g of *Plukenetia volubilis* oil (ratio of 8.10 % m/m with respect to the mass of the nutraceutical). No oil-soluble substances are previously added to the oil phase of *Plukenetia volubilis.*

When these components are stirred, the gaseous phase (air) is incorporated, which represents 50 % of the volume of the emulsion (24.67 % of the total volume of the nutraceutical). For this emulsion preparation step the temperature of 25 °C is maintained.

The final composition of the emulsion of Variant 4 (V4) contains five components of different nature (water, air, oil, sugar, egg white). In this way a four-phase emulsion variant is obtained: aqueous:oily:gaseous: solids solubilized in the aqueous.

In parallel, the mass of *Cucurbita pepo* seeds is prepared, which are processed shelled, ground and roasted. The emulsion is mixed with the seeds to form a macro-emulsion type food paste: to the mass of 500 g of the shelled, ground and roasted seeds (50.66 % m/m with respect to the mass of the nutraceutical) is added 487.05 g of the emulsion obtained, equivalent to 49.34 % m/m with respect to the total mass of the nutraceutical. The addition is carried out under manual agitation to ensure homogeneous mixing and the least loss of trapped air. The mixing of the emulsion with the seeds to form a macroemulsion with all the components of the final formulation of the nutraceutical is executed manually by applying enveloping movements.

Subsequently, the macroemulsion of the nutraceutical obtained with all the components is cooled, initially at room temperature and subsequently at a temperature of 4 °C, for an interval of 24 h. Once the mass is cooled, the cutting and portioning proceed. The obtained nutraceutical is presented in the form of bars that are covered with waterproof paper and stored at a temperature of 4 °C.

In the V4 variant, the nutraceutical contains several different sources of fatty acids, phytosterols and triacylglycerols, including *Cucurbita pepo* seeds and *Plukenetia volubilis* oil (which contains fatty acids of 6 to 12 carbons, capable of generating ketone bodies) and egg white. In this variant, more than one component performs several biological or functional functions, described in the part of the description devoted to the role of each component and its biological activity.

The final nutraceutical product (V4) contains three components with antioxidant activity, including *Cucurbita pepo* seeds, *Plukenetia volubilis* oil and *Stevia rebaudiana* extract, as part of the emulsion. A source of hypoglycemic compounds, specifically *Cucurbita pepo* seeds, is included in the final composition of the nutraceutical. A source of anticholesterolemic compounds is used, specifically *Plukenetia volubilis* oil. To make up the nutraceutical, two sources of anti-inflammatory compounds are used, which are *Stevia rebaudiana* extract, forming part of the V4 emulsion, and *Cucurbita pepo* seeds. The nutraceutical of the V4 variant contains components that provide vitamins, macro- and micro-elements in sufficient quantities to supply different deficiencies of them, or to maintain the necessary physiological level, specifically *Cucurbita pepo* seeds provide vitamins A, K, E and folic acid.

The nutraceutical composition contains substances that provide organoleptic characteristics typical of nougat and bars, appropriate for the subjects and patients who consume it; specifically, *Plukenetia volubilis* oil provides the nutraceutical with a softer consistency, lower hardness, increases adhesiveness, makes it more digestible and improves chewability. The egg white traps a very significant amount of air (high overrun) that makes the consistency of the nutraceutical softer to the palate and causes the intensity of the color to decrease. On the other hand, the *Stevia rebaudiana* extract used in V4 provides sweetness to the composition, appropriate to the palate.

The nutraceutical composition obtained, according to V4, can be used, by the biological properties of its components, in the prevention or treatment of diseases, disorders or syndromes accompanying cognitive impairment, Alzheimer's disease, developmental disorders, disorders of the digestive, endocrine, circulatory, cardiovascular and cerebrovascular systems, in infectious and parasitic diseases, as well as to treat tiredness, fatigue and malnutrition.

The nutraceutical, according to composition V4, is recommended to be consumed two to three times a day, in portions of 40 g, daily frequency, for one month, rest from 3 to 30 days, repeating the cycle of administration.

Effect of the components of the nutraceutical according to the present invention on cognitive improvement in aged OF1 mice.

Experimental protocol

Male OF1 mice, 7 to 8 months old, whose weight range was between 45 g and 55 g were used. In addition, young mice 1 to 2 months old and 20 g to 24 g in weight were used. During the whole experimental procedure, the animals were kept in a conventional room with a controlled environment (minimum security type IV barrier), with a temperature of $21 \pm 3$ °C and relative humidity between 40 % and 70 %, performing 18 air changes per hour, 100 % injection of outdoor air with 85 % filtration. In addition, with automatically controlled lighting of 12 hours of light and 12 hours of darkness and with free access to water and food. The animals were grouped at a rate of five per polypropylene box with bedding of desmoled sugarcane bagasse.

Treatment

The animals were divided into three groups: one group with young animals (1 to 2 months old), and the rest of the groups with animals from 7 to 8 months old.

Both the group of young animals (G1) and one of the groups of 7- to 8-month-old animals (G2) were administered 10 % Tween 80 in 0.9 % NaCl as a vehicle via i.g. While the remaining group of animals from 7 to 8 months of age was supplemented via i.g with the oil of *Plukenetia volubilis* (of the V3 variant) at a dose of 10 mL/kg which was administered in a volume of 0.5 mL. The treatment was administered on a daily basis for 30 days. To evaluate the effect on cognitive improvement, the Y-maze test was applied with a closed arm on days 29 and 30 of the study.

Parameters evaluated

Y-maze test with a closed arm: An addition of three arms of equal length (30 cm), interconnected with each other at an angle of 120°, was used. Each arm was identified with a different letter (A, B, C).

One of the three arms of the Y-maze was canceled. With the gloved hand the animal was held by the tail to extract it from the cage and check the identification number. The animal was then placed on the palm of the hand to move it from the plateau to the center of the Y-maze. Seven (7) minutes were marked on the digital clock and, when the start button was activated, the animal's tail was released so that it was free and the exploration of the maze could begin. During that time of 7 minutes the experimenter was placed in the same position, in silence. After the 1 h period, the animal was returned to the maze, but with all three arms open. Then the digital clock was dialed back to 7 minutes and in that interval the experimenter was counting with a stopwatch the total time during which the animal explored the novel arm. The percentage variable that represented the scanning time of the novel arm of the total test time was measured using the formula % = exploration time/240 s * 100.

As a criterion of validity it was considered that, if the animal did not manifest locomotor activity during the first 4 minutes, the test would not be considered as valid.

Results

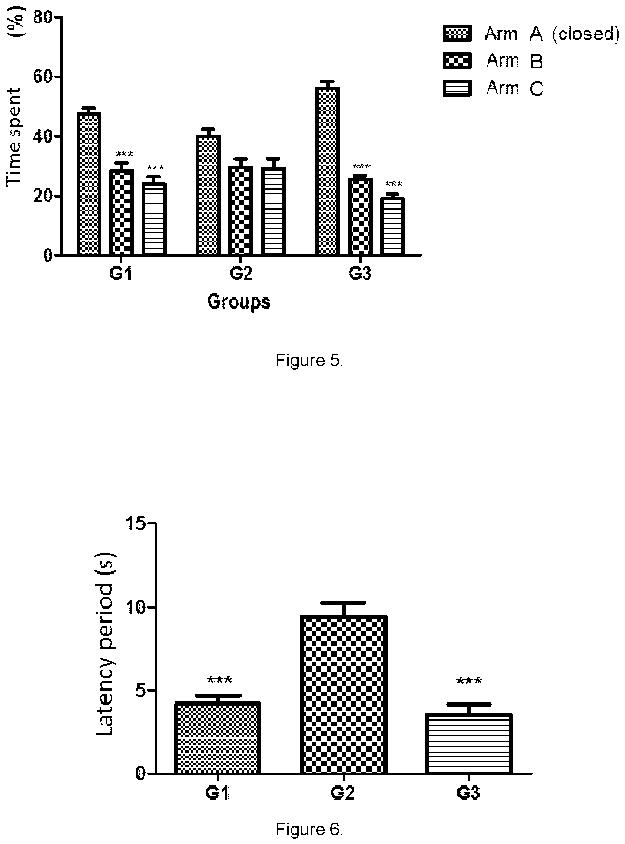

In Figure 5 it is observed that in the Y-maze test with a closed arm it was obtained that, in groups G1 and G3, the percentage of the time spent in the arms that were open was significantly lower than in the closed arm. In the G2 group there were no significant differences between the arms.

These results first demonstrated the validity of the animal model used, because in young animals (G1) the time spent in the closed arm (A) was significantly longer, while in animals that did not receive supplementation with the V3 variant component, no significant differences were obtained between the time spent in the different arms, that is, the animals presented cognitive impairment.

On the other hand, in this work it was demonstrated that supplementation with the product evaluated for 30 days and daily frequency, at the dose of 10 mg/kg, improved the cognitive function of OF1 male mice, since in this group the time spent in arm A was significantly longer than in the rest of the arms. In addition, this time was also longer than in the two control groups of the experiment (G1 and G2). The cognitive improvement that occurred in this group allows us to affirm that supplementation with omega-3 and omega-6 fatty acids present stimulates the fluidity of the cell membrane of nerve cells, the adhesion of nerve cells, dendritic formation, and the speed of neurotransmission, which as a whole favors the cellular processes of learning and memory (Hering, H., C. C. Lin and M. Sheng, (2003): Lipid rafts in the maintenance of synapses, dendritic spins, and surface AMPA receiver stability. J Neurosci, 23 (8): 62-71).

In this way, it is demonstrated that the product can be used for the prevention and treatment of cognitive deficit, Alzheimer's disease, mental, behavioral and developmental disorders, including depressive, nervous system and cerebrovascular disorders.

Example 4

Preparation of the nutraceutical composition of Variant 5 (V5)

Preparation of the emulsion components:
A suspension of solids in oil phase is prepared consisting of the addition of 8 g of turmeric alcoholic extract concentrate (ratio of 1.09 % m/m with respect to the total mass of the nutraceutical) obtained according to Example 1, diluted in 90 g of *Plukenetia volubilis* oil (ratio of 12.23 % m/m with respect to the total mass of the nutraceutical).

The aqueous liquid phase consisting of 19 g of *Stevia rubidaea* extract concentrate (2.58 % m/m with respect to the mass of the nutraceutical) is selected to be dissolved in 70 g of water (9.51 % m/m with respect to the total mass of the nutraceutical), stirring until complete dissolution to form the aqueous phase with soluble solids in it.

Egg white is selected as the emulsifying agent and 32 g of it are added (in a ratio of 4.35 % m/m with respect to the total mass of the nutraceutical). The emulsifying agent is added to the aqueous phase under agitation at 18,000 1/min and the oily phase with the suspended solids is incorporated. By stirring these components, the gas phase (air) is incorporated, which represents 40 % with respect to the volume of the emulsion (11.93 % of the nutraceutical volume). The emulsion is kept at room temperature. In this way a 4-phase emulsion variant is obtained (aqueous, oily, air and suspended and solubilized solids: aqueous:oily:gaseous:suspended solids in the oily and solubilized solids in the aqueous.

The seeds of *Cucurbita moschata*, roasted at 80 °C and shelled, were ground in a blade mill with a stainless steel cup at a speed of 12,000 1/min for 5 min until a fine paste was obtained. The emulsion mixture was then mixed with the seed and oil mass, i.e., a macro-emulsion of the type of food mass. Five hundred (500) g of the mass of the shelled seeds already roasted and ground (67.9 % m/m with respect to the total mass of the nutraceutical) are taken and 219 g of the emulsion are added (29.76 % m/m with respect to the total mass of the nutraceutical) is added and mixed at low speed with slow movement so as not to let the trapped air escape and 10 g of *Plukenetia volubilis* oil (1.36 % m/m with respect to the mass of the nutraceutical) are added to form an emulsion mixture with the mass of oil and seed.

To the emulsion mixture and the seed and oil mass, 7 g of *Theobroma cacao* powder (0.95 % m/m with respect to the mass of the nutraceutical) are added and the mixing is continued until the components are homogeneous. The product is placed in a rectangular mold and refrigerated at a temperature of 2 °C for 12 hours. It is then stored at room temperature.

The final composition of the V5 emulsion contains 4 phases, 6 components of different nature (water, air, oils, *Stevia* extract, egg white and *Curcuma longa* alcoholic extract). *Plukenetia volubilis* oil and cocoa powder are also added to the final composition of the product. In this variant, more than one component performs various biological or functional functions, which were described above for each component, and to these is added the cocoa powder, which acts as an antioxidant and anti-inflammatory, provides flavor and masks flavors, odors, color of the *Curcuma extract* and the bitter aftertaste characteristic of the *Stevia* extract. Other beneficial properties of cocoa powder were described above in the description of the role of each nutraceutical component.

In this V5 variant the nutraceutical contains four different sources of fatty acids, phytosterols and triacylglycerols which are seeds of *Cucurbita moschata, Plukenetia volubilis* oil, *Theobroma cacao* powder and egg white. The final nutraceutical product of the V5 variant contains four components with antioxidant activity, which are *Cucurbita moschata* seeds, *Curcuma longa* extracts, *Stevia rebaudiana* extract and *Theobroma* cacao. One source of hypoglycemic compounds, specifically *Cucurbita moschata* seeds, is included. In addition, three different sources of anti-cholesterolemic compounds are used, specifically *Plukenetia volubilis* oil, egg lecithin and *Theobroma cacao.* The anti-inflammatory compounds are represented by *Stevia rebaudiana* extract as part of the emulsion, *Cucurbita moschata* seeds and *Theobroma cacao* in the final formulation of the product.

Sources of proteins of vegetable origin are used, such as the seeds of *Cucurbita moschata* and contain components that provide vitamins, macro- and micro-elements in sufficient quantities to supply different deficiencies of them, or to maintain the necessary physiological level, specifically the seeds of *Cucurbita, Theobroma cacao* powder and the ethanolic extract of *Curcuma longa,* whose contributions have already been described above. To improve swallowing and chewiness, obtaining a better score in the organoleptic evaluation, *Plukenetia volubilis* oil and *Stevia rebaudiana* extract are used, whose role was described in Example 2, and *Theobroma cacao* powder, which in addition to providing a pleasant and characteristic flavor, masks the flavor and color of the turmeric extract, bringing it closer to a traditional food product.

The nutraceutical composition can be used for the prevention or treatment of diseases, disorders or syndromes comprising a cognitive deficit, such as Alzheimer's Disease, mental, behavioral and developmental disorders, including depressive disorders and disorders of the nervous, digestive, immune, endocrine and circulatory systems; cardiovascular, cerebrovascular, infectious, parasitic, oncological and blood diseases; as well as treating tiredness, fatigue and malnutrition, due to the causes already described in the above examples.

The nutraceutical is recommended to be consumed once a day in portions of 40 g, daily frequency, for a period of one month, resting from 3 days to 30 days, repeating the administration cycle.

The sensory evaluation of the nutraceutical showed that the taste, color and smell are more "pleasant" to the palate, according to the consumers, than all the previous variants. Consistency is smoother and swallowing is easier. There is no separation or breakage of the structure.

Effect of the nutraceutical component on cognitive improvement in aged OF1 mice

Experimental protocol

The effect on cognitive impairment was evaluated in the presence of *Curcuma longa* extract at the dose 300 mg/kg, administered via i.g, in a volume of 1.1 mL, in aged male OF1 mice. In this experiment a similar design to experiment 1 was used, but 3 treatment groups were formed: two of the groups (young control and aged control) received a 10 % Tween 20 solution in 0.9 % NaCl and the G3 group was supplemented with the V5 variant component tested from Monday to Friday for 5 weeks. Response to treatment was analyzed by the Morris water maze test.

Parameters evaluated

Morris water maze test: it was performed in a 120 cm diameter plastic tank inside which a plastic platform was placed, the upper base of which is square. The test consists of 2 phases: a training phase lasting four days with 4 trials from the same position per day per animal, and a holding phase on the 5th day without a platform. The platform was kept in a fixed location during the 4 days of training and the animal was placed with its back to the platform, i.e., with its face facing the tank wall. In each training section, the animal was allowed to swim for 90 s until it found the platform. The animal was allowed to stay on the platform for a period between 10 s and 30 s. On the 5th day of the test, the platform was removed from the tank and all animals were placed in the same position to swim in the tank for 60 s. With the help of the video player, the time that each animal spent in each of the 4 quadrants and the time each animal took to go for the first time to quadrant 1, where the platform was located (latency period), was counted.

Results

At the end of 5 weeks of treatment, the Morris water maze test, which evaluates cognitive improvement, was applied. In this test, the aged control mice (G2) took longer to find the platform quadrant than the rest of the animals (Figure 6). This result demonstrates the cognitive improvement in the supplemented group, G3, in terms of the latency period during the acquisition phase. These animals took less time to visit the quadrant for the first time, even though there were no significant differences with respect to the young animals. The fact that aged animals had the worst performance with respect to this variable has been described in the literature and R. D'Hooge and P.P. De De Deyn in 2001 (R. D'Hooge, P.P. De De Deyn. Applications of the Morris water maze in the study of learning and memory. Brain Research Reviews 36 (2001) 60 -90) argued that as animals age they show a decline in cognitive functions and have a decrease in the efficiency of the performance of this behavioral test. All this because in these animals the abilities for swimming, locomotion and exploration change. It has been suggested that this has coincided with structural and physiological modifications in some brain structures, specifically in the hippocampus, related to spatial learning.

As can be seen, the use of *Curcuma longa* extract in the composition of the nutraceutical causes a cognitive state in aged mice that is not statistically different from untreated young mice (although a shorter latency period is noted). This result was not expected, as an improvement of the treated aged animals with respect to the untreated group of aged mice was assumed.

Similar to the effect of the nutraceutical described above, the product formulated with this component can be used for the prevention and treatment of cognitive deficits, Alzheimer's disease, mental, behavioral and developmental disorders, including depressive, nervous system and cerebrovascular disorders.

Example 5

Effect of nutraceutical on cognitive improvement in aged OF1 mice

Experimental protocol

The effect of the nutraceutical according to the V1 Variant of Example 2 was evaluated as supplementation to the normal diet at a rate of 3 g/animal in aged male OF1 mice. In this experiment, a design was used in which 3 treatment groups were formed, two of the groups (young control and aged control) received a solution of 10 % Tween 20 in 0.9 % NaCl and the G3 group was supplemented with the nutraceutical from Monday to Friday for 5 weeks.

During the last week of treatment, the Y-maze test (spontaneous alternation) and the passive avoidance test were applied to the three groups.

Parameters evaluated

Y-maze: An addition of three arms of equal length (30 cm) was used, interconnected with each other at an angle of 120°. Each arm was identified with a different letter (A, B, C). The test was run for 7 min and at the beginning of the test the animal was placed in the center of the maze. An entry was considered when all four legs were inside one of the arms. An alternation was considered when the animal visited the three arms of the maze consecutively, without repeating the entrance to the arm it visited previously. In addition, the number of inputs to the arms was used as an indicator of locomotor activity. From the number of alternations the variable alternation percentage was calculated by the following expression:

$$Alternation\ (\%) = \frac{Alternations}{Total\ posible\ alternations}\ X\ 100$$

Where: Total Possible alternations = Total entries - 2

Passive Avoidance: The animals were transferred from the usual accommodation area to the experimental one 24 hours earlier to get them to get acquainted with that premises. To carry out this test, a box with two compartments, one light and one dark, was used. In the dark compartment there is a closed circuit that produces small electric shocks. It is intended that the mouse learns to avoid the dark compartment where the shock is applied to it.

The test began with a training that consisted of placing the mouse in the illuminated compartment. At 25 s the door of the dark behavior was opened until the mouse spontaneously went to it.

The moment the mouse entered the dark compartment with all four paws, the door was closed and a 0.3 mA electric shock was given to it for a period of 5 s. After 10 s after the application of the shock, the mouse was returned to its cage.

The long-term memory was evaluated by allowing 24 h to elapse to return the mouse to the illuminated compartment with free access to the dark compartment, i.e. the dark compartment with the door open. The animal was left in the passive avoidance box for 5 min and at that time the time it takes the animal to visit the camera obscura for the first time (latency period) was measured.

Results

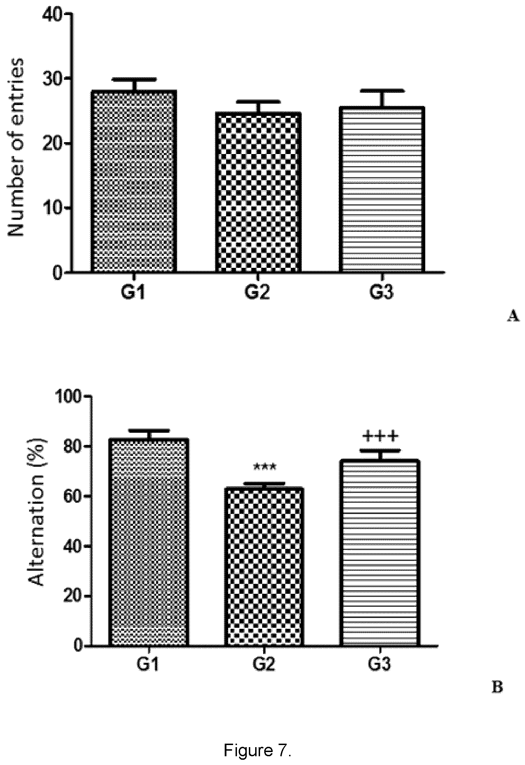

At the end of 5 weeks of treatment with the nutraceutical, the Y-maze test was applied to evaluate the cognitive improvement of the animals that received this supplement orally. Although no significant differences were found between the two groups with respect to the variable number of entrances to the Y-maze arms (Figure 7A), this was not the case with the percentage of alternation. In this sense, the alternation of the animals of the group supplemented with the nutraceutical with respect to the aged control group was significantly higher (Figure 7B).

Table 18 shows that the nutraceutical administered as a dietary supplement from Monday to Friday, managed to improve cognitive function in the mice that had deficiency. The results obtained in the Y-maze test in this experimental procedure significantly demonstrated the superiority of the supplemented group (G3) in the percentage of alternation, in addition to presenting a similar behavior to the young controls (G1) without showing significant differences. These results suggest that the supplemented mice remember the last arm visited better than the aged controls, orient themselves better in space and also suggest that, based on the memory mechanism that is evaluated in the test, it can be affirmed that, with the treatment, a cognitive improvement was obtained and, therefore, greater efficacy of the treatment.

As demonstrated, the nutraceutical provides a technical superiority over other previously reported solutions by achieving that the cognitive state of the animals that received treatment with the nutraceutical, not only exceeded that of the aged animals, but also did not show significant differences with respect to that of the young animals.

Table 18. Effect of the nutraceutical orally for 5 weeks and a frequency of 5 times a week.

| Group and treatment conditions | Results of the spontaneous alternation test | |
|---|---|---|
| | Alternation percentage | Number of entries |
| **G1** (vehicle, 10 % Tween 20 in 0.9 % NaCl) young | 82.81 $\pm$ 11.00 | 28.00 $\pm$ 5.68 |
| **G2** (vehicle, 10 % Tween 20 in 0.9 % NaCl) aged | 62.81 $\pm$ 6.65 | 24.60 $\pm$ 5.68 |
| **G3** (nutraceutical) 3 g/animal orally | 74.03 $\pm$ 12.94 | 25.50 $\pm$ 7.31 |

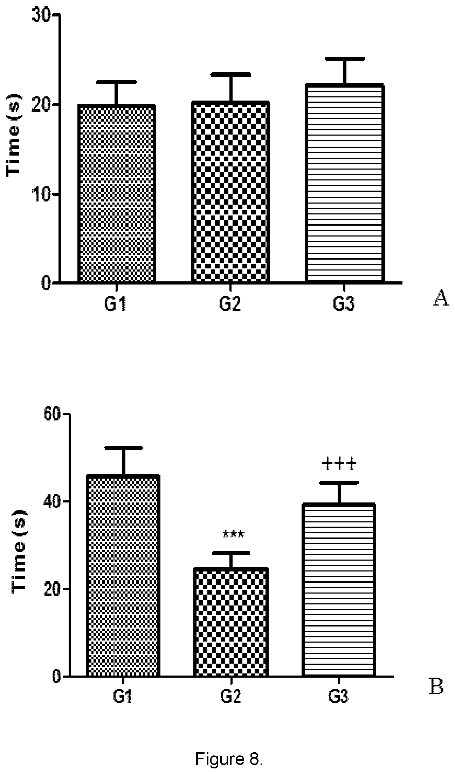

At the same experimental time, the passive avoidance test was also applied to the experimental animals. Both the animals that received the treatment with the nutraceutical and those that received placebo had no significant differences regarding the time of entry into the dark compartment in the acquisition phase of the test (Figure 8A). This allows us to affirm that both groups of animals started from the same locomotor conditions and that aging did not influence the speed with which they passed to the dark compartment. On the other hand, after receiving the electrical stimulus, an improvement in cognitive function was evidenced in the animals of the group supplemented with nutraceutical with respect to the aged control (Figure 8B). The passive avoidance test is traditionally used as a quick and easy way to explore short-term and long-term memory. In this case it was used to measure long-term memory and the presence of cognitive impairment can be affirmed in the aged control animals since they took less time to return to the dark compartment where they received the aversive stimulus.

On the other hand, it can be taken as a positive effect of the treatment the fact that the animals that received the nutraceutical had a longer latency period than the animals of the G2 group, indicating that they remembered the stimulus received, behaving similarly to the G1 group.

This may be linked to the presence in the nutraceutical composition of omega fatty acid sources in *Sacha inchi* oil, which is an ingredient with a high content of polyunsaturated fatty acids, such as Linoleic acid (18:2) known as Omega 6 and Linolenic (18:3) which is known as Omega 3. These fatty acids are considered essential for all animals. Omega-3 fatty acid is essential for the development and growth of the nervous system. Added to this is the presence of cocoa, which provides health benefits that have been widely documented.

This other example reinforces the claim of nutraceutical application in the prevention and treatment of cognitive deficits, Alzheimer's disease, mental, behavioral and developmental disorders, including depressive, cerebrovascular and nervous system disorders.

Example 6

Hypoglycemic effect of the nutraceutical and the components *Cocos nucifera* oil, *Plukenetia volubilis* oil and *Curcuma longa* extract in OF1 mice.

Experimental protocol

Treatment

The same design as Example 2 was used, but 5 groups of animals from 7 to 8 months of age and one group of young animals were formed. Groups G1 to G2 received the same treatment described in Example 3, while groups G3 and G4 received the *Cocos nucifera* oil and *Plukenetia volubilis* oil components at the same dose, frequency and route of administration of the *Plukenetia volubilis* oil component in Example 3. In addition, group G5 received *Curcuma longa* extract at the same dose and route of administration as described for this component in example 3, while group G6 received the nutraceutical orally at a rate of 3 g/day per animal on average. To evaluate the hypoglycemic effect, the serum glucose concentration of the animals that were bled at the end of the supplementation period was measured. For this purpose, the Glucotest reagent kit (HELFA Diagnósticos, Cuba) was used.

Results

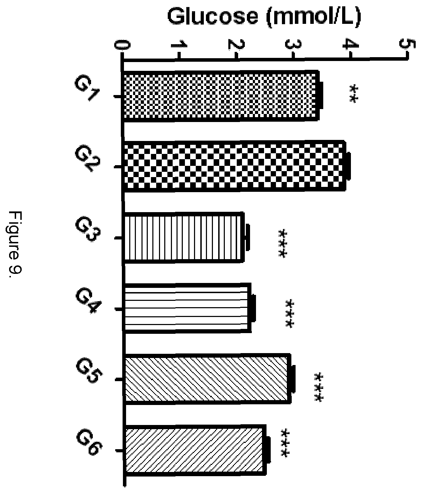

Glucose concentration decreased significantly in groups G3 to G6 with respect to animals of the same age that did not receive supplementation (G2, placebo). In addition, the comparison between them showed that there were no significant differences between the group supplemented with nutraceutical and those that received *Cocos nucifera* oil and *Plukenetia volubilis* oil. Meanwhile, glucose levels for the case of *Curcuma longa* extract were significantly higher (p < 0.05) than for the group that was supplemented with the nutraceutical (Figure 9). The decrease in glucose levels constitutes a positive effect of the evaluated oils, *Curcuma* extract and nutraceutical, as it has been shown that, in humans, lower brain glucose metabolism is present before the onset of clinically measurable cognitive impairment in groups of people at risk for Alzheimer's disease. Furthermore, in vitro and animal studies have shown that brain hypometabolism may precede and contribute to the neuropathological cascade that causes cognitive impairment in Alzheimer's disease. On the other hand, this hypometabolism of glucose in the brain may be due to defects in glucose transport at the blood-brain barrier, in glycolysis and/or in mitochondrial function, whereby this physiological situation leads to elevated serum levels of this molecule during aging (Cunnane SC, Nugent S, Roy M, Courchesne-Loyer A, Croteau E, et al. Brain fuel metabolism, aging and Alzheimer's disease. Nutrition. 2011 27(1): 3-20. doi:10.1016/j.nut.2010.07.021).

Unexpectedly and surprisingly, administration of the nutraceutical and several of its active components resulted in a reduction in blood glucose levels relative to both young and aged untreated animals.

This effect was not obvious, as the result of the combination of the three components mentioned above, although it did not yield significantly different results between groups 3 to 6, showed a difference between the group treated with the nutraceutical with respect to the groups treated with the components separately.

The results of the tests in animal models showed that the nutraceutical can be used in the prevention or treatment of certain diseases, disorders, or as nutritional support, specifically in those linked to glucose metabolism disorders, including: cognitive deficits, Alzheimer's disease, mental, behavioral and developmental disorders, including depressive disorders; nervous system disorders; cerebrovascular diseases, tiredness and fatigue.

Example 7

Hypocholesterolemic and hypotriglyceridemic effect of nutraceutical and *Cocos nucifera* oil component in OF1 mice

Experimental protocol

The same design as Example 3 was used

Treatment

The same design of example 3 was used, but 3 groups of animals from 7 to 8 months of age were formed. Groups G1 to G3 received the same treatment described in example 2, while group G4 received the nutraceutical component at the same dose, frequency and route of administration described in the previous example. To evaluate the hypocholesterolemic and hypotriglyceridemic effect, serum triglyceride and cholesterol concentrations were measured. The Monotrigli test and Colestest reagent set (HELFA Diagnósticos, Cuba) was used for this purpose.

Results

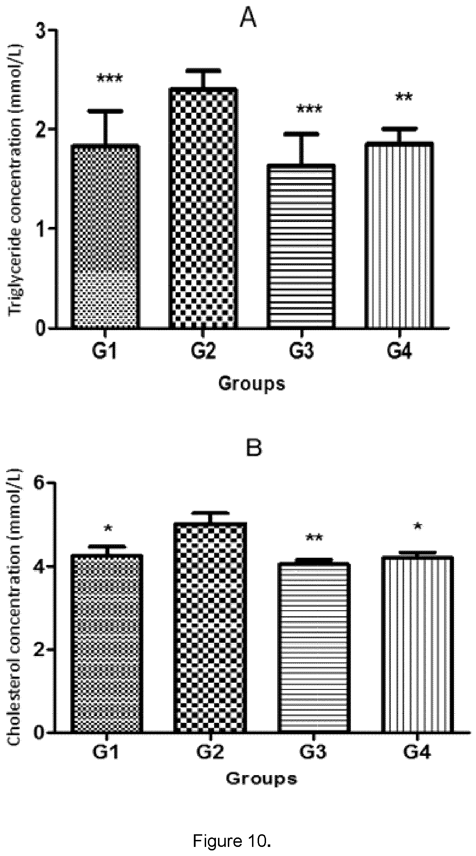

Analysis of the serum samples collected revealed that, in the G3 and G4 groups, triglyceride and cholesterol levels decreased significantly with respect to the aged control animals that received placebo (G2). In addition, the levels achieved for both biochemical parameters related to lipid metabolism showed no significant differences with respect to the young animals (G1) (Figure 10). These results constitute a favorable effect of these components, as increased serum cholesterol and triglyceride levels are known to increase the risk of developing dementias, including Alzheimer's Disease (Proitsi P, Lupton MK., Velayudhan L, Newhouse S, Fogh I, Tsolaki M, et al. Genetic Predisposition to Increased Blood Cholesterol and Triglyceride Lipid Levels and Risk of Alzheimer Disease: A Mendelian Randomization Analysis. PLOS Medicine, 2014, 11 ( 9): e1001713).

In the experimental models, it was established that the nutraceutical, due to its hypocholesterolemic properties and its ability to decrease triglyceride content, can prevent or be employed for the treatment of cognitive deficit, Alzheimer's Disease, mental, behavioral and developmental disorders, including depressive disorders and disorders of the nervous, digestive, endocrine and circulatory systems, cardiovascular, cerebrovascular, oncological and blood diseases, tiredness, fatigue, and malnutrition.

Example 8

Anti-inflammatory effect of nutraceutical and ethanolic extract of *Curcuma longa* in OF1 mice

Experimental protocol

The same design as in Example 2 was used

Treatment

Aged animals were distributed into 3 groups (G2 to G4) and young animals were grouped into G1. The animals were supplemented orally. Both control groups, the aged animals (G2) and the young animals (G1), were supplemented with a placebo feed made from soybean oil, wheat flour, egg and refined sugar. Groups G3 and G4 received *Curcuma* extract and the nutraceutical formulation, respectively. In all groups, the feed was administered after the animals had been deprived of their normal diet for 4 hours (fasting). The feed was administered ad libitum and an average consumption of 3 g/animal was measured.

To evaluate the anti-inflammatory effect of supplementation, the behavioral test of pain sensitivity to hot iron was used. The mice were placed on a stainless steel surface maintained at $50 \pm 1$ °C. Animals remained on the hot iron until they performed either of two behaviors considered indicative of nociception: jumping or shaking/licking a hind paw (Mogil, J.S. Wilson, S.G.; Bon, K.; Lee, S.E.; Chung, K.; Raber, P.; Pieper, J.O.; Hain, H.S.; Belknap, J.K.; Hubert, L.; et al. Heritability of nociception i: Responses of 11 inbred mouse strains on 12 measures of nociception. Pain 1999, 80, 67-82). The experimenter recorded the time during which the animal showed one of these pain behaviors. Mice that did not show any of the expected pain behaviors during the first 60 s were rejected.

Results

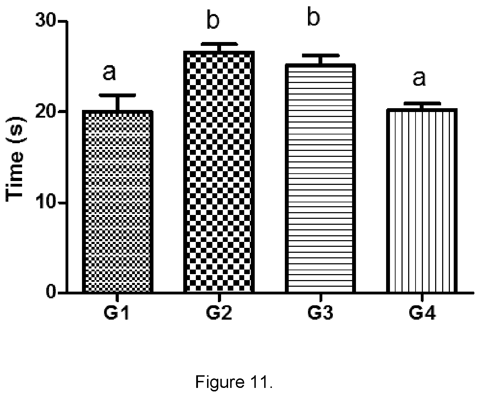

It was obtained that the G4 group (supplemented with nutraceutical) showed a sensitivity to heat pain similar to that of the young animals (G1). In addition, no significant differences were obtained between groups G2 and G3 and it was significantly higher than that of groups G1 and G4 (Figure 11). These results demonstrate that the nutraceutical decreases the inflammation that occurs during the natural aging process and affects nociception (King-Himmelreich TS., Möser CV., Wolters M., Olbrich K, Geisslinger G, Niederberger E. Age-Dependent Changes in the Inflammatory Nociceptive Behavior of Mice. Int. J. Mol. Sci. 2015, 16, 27508-27519; doi:10.3390/ijms161126041).

The product composition obtained according to the present invention can be used as a nutraceutical for the prevention or treatment of diseases or disorders having as a cause or consequence inflammation and, as a basis, changes in the immune response and defense of the organism, such as: Alzheimer's disease, mental, behavioral and developmental disorders, including depressive disorders and disorders of the nervous, digestive, endocrine and circulatory systems, cardiovascular, cerebrovascular, oncological and blood diseases, tiredness, fatigue, and malnutrition.

Example 9

A new nutraceutical variant (V6) was prepared

Preparation of the emulsion components

An oil-phase suspension of solids is prepared consisting of the addition of 5.03 g of turmeric hydroalcoholic extract (ratio of 0.52 % m/m to the total mass of the nutraceutical) obtained according to Example 1 and dissolved in 80 g of *Plukenetia volubilis* oil (ratio of 8.28 % m/m to the total mass of the nutraceutical). For this, the mixture is stirred for 4 h in a temperature-controlled bath at 50 °C. It is centrifuged at 4,300 1/min.

The aqueous liquid phase consisting of 80 g of water (8.28 % m/m with respect to the total mass of the nutraceutical) is selected to dissolve 250 g of sugar (25.88 % m/m with respect to the total mass of the nutraceutical), heated until boiling and reaching syrupy point, without stirring, to form the aqueous phase with soluble solids in it and allowed to cool to 40 °C.

Soy lecithin is selected as emulsifying agent of which 1.6 g is added (in a ratio of 0.17 % m/m to the total mass of the nutraceutical). The emulsifying agent is added to the aqueous phase by stirring it with an ultra agitator and the oily phase of *Plukenetia volubilis* with the turmeric extract already included is added. The emulsion is kept at room temperature.

On the other hand, 32 g of egg white are taken and beaten until foam is formed as a foaming agent, but at the same time emulsifier (3.31 % m/m with respect to the mass of the nutraceutical) and incorporated into the emulsion. When these components are stirred, the gas phase (air) is incorporated, representing 40 % with respect to the volume of the emulsion (18.58 % of the volume of the nutraceutical).

In this way, a 4-phase emulsion variant is obtained (aqueous, oily, air and suspended and solubilized solids: aqueous:oily:gaseous:solubilized solids in the oily and solubilized solids in the aqueous.

Roasted at 80 °C and shelled seeds of *Cucurbita moschata* are ground in a blade mill with a stainless steel cup at a speed of 12,000 1/min for 5 min until a fine paste is obtained. The emulsion is then mixed with the seed and oil mass, i.e., a macro-emulsion of the type of food mass. Five hundred (500) g of the mass of shelled, roasted and ground seeds (51.78 % m/m with respect to the total mass of the nutraceutical) are taken, 448.63 g of the emulsion are added to it (46,46 % m/m with respect to the total mass of the nutraceutical) and mixed at low speed with slow movement so as not to let the trapped air escape, and 10 g of *Plukenetia volubilis* oil (1.03 % m/m with respect to the mass of the nutraceutical) are added to form an emulsion mixture with the oil and seed mass.

To the emulsion mixture and the seed and oil mass, 7 g of *Theobroma cacao* powder (0.73 % m/m with respect to the nutraceutical mass) are added and the mixing is continued until the components are homogeneous. The product is placed in a rectangular mold and refrigerated at a temperature of 2 °C for 12 hours. Subsequently, it is stored at room temperature.

In this variant it is demonstrated the obtaining of an emulsion of 4 phases type: aqueous:oily:solubilized solids in the oily and solubilized solids in the aqueous. The chemical composition is shown in Table 19.

Table 19. Chemical composition of nutraceutical variant V6

| Total solids, % | Proteins, % | Total fats, % | Ashes, % | Chlorides, % |
|---|---|---|---|---|
| 89.01 | 35 | 38.86 | 3.2 | 0.45 |

The high content of total solids, among which fats are the majority, guarantees the high stability and preservation of the product, since the product was kept at $8\,^0C$ for 1 year without loss of its main attributes, such as flavor, color and odor, although an increase in hardness when cut was observed.

In this Variant 6 (V6), more than one component performs several biological or functional functions, which were described above for each component, and to these is added the cocoa powder that serves as an antioxidant and anti-inflammatory, provides flavor and masks flavors, odors and colors of the *Curcuma* extract, and the bitter aftertaste characteristic of the *Stevia* extract. Other beneficial properties of cocoa powder were described above in the description of the role of each nutraceutical component.

In this V6 variant, the nutraceutical contains four different sources of fatty acids, phytosterols and triacylglycerols, which are *Cucurbita moschata* seeds, *Plukenetia volubilis* oil, *Theobroma cacao,* egg white and soy lecithin. The final nutraceutical product of variant V6 contains 3 components with antioxidant activity, which are the seeds of *Cucurbita moschata* and the extracts of *Curcuma longa* and *Theobroma cacao.* A source of hypoglycemic compounds is included, specifically *Cucurbita moschata* seeds. In addition, four different sources of anti-cholesterolemic compounds are used, specifically *Plukenetia volubilis* oil, lecithin present in egg white, soy lecithin and *Theobroma cacao.*

The anti-inflammatory compounds are represented in the final formulation of the product by the seeds of *Cucurbita moschata* and *Theobroma cacao.*

Sources of proteins of vegetable origin are used, such as *Cucurbita moschata* seeds, and it contains components that provide vitamins, macro- and micro-elements in sufficient quantities to supply different deficiencies of them, or to maintain the necessary physiological level, specifically the seeds of *Cucurbita, Theobroma cacao* powder and the ethanolic extract of *Curcuma longa,* whose contributions have already been described above.

Example 10

To prepare new nutraceutical variants.

Preparation of the emulsion components according to Variants 7 (V7), 8 (V8) and 9 (V9)

To prepare the base emulsion, the aqueous liquid phase consisting of 250 g of water is selected, heated to 100 °C and 780 g of sucrose (sugar) is added as sweetener, stirred until completely dissolved and brought to a temperature of 40 °C.

The emulsifying agent soy lecithin (5 g) is selected. The components are stirred and mixed at a stirring speed of 15,000 1/min to obtain a microemulsion. While stirring these components, the gas phase (air) is incorporated. For this preparation stage, the emulsion is kept at a temperature of 25 °C.

Subsequently, the oily phase of the emulsion is prepared with soluble solids in it, in this case ethanolic extract of *Curcuma longa*. The oily phase is composed of 250 g of *Plukenetia volubilis* oil and 15.05 g of ethanolic extract of *Curcuma longa*. In this preparation phase the emulsion is kept at a temperature of 25 °C. In this way a 4-phase emulsion variant is obtained (aqueous, oily, air and suspended or solubilized solid), specifically aqueous:oily:gaseous:solids suspended in the oily.

In parallel, the *Cucurbita moschata* seed mass is prepared. The seeds are processed shelled, ground and roasted. The seeds are dried at a temperature of 80 °C for 60 min. Roasting of the seeds is carried out at a temperature of 130 °C. The grinding of the seeds is executed in a blade mill and particles of size between 10 $\mu$m to 30 $\mu$m are obtained. 1500 g of seed are taken and mixed with the emulsion with manual agitation so as not to lose the contained air. Different mixtures of the emulsion with the mass of seeds and oils and other components are made according to the variants V7, V8 or V9.

For V7, 75 g of the mixture of the emulsion with the seeds are taken, so that in this mass of 75 g of emulsion and seeds it is possible to calculate how their components are found in quantities and, in brackets, the relation to the total mass of the nutraceutical of this variant V7 is indicated:

water - 6.70 g (7.88 % m/m in relation to the total mass of the nutraceutical)
sucrose - 20.89 g (24.58 % m/m with respect to the total mass of the nutraceutical)
soy lecithin - 0.13 g (0.15 % m/m with respect to the total mass of the nutraceutical)
*Plukenetia volubilis* oil - 6.70 g (7.88 % m/m with respect to the total mass of the nutraceutical)
ethanolic extract of *Curcuma longa* - 0.40 g (0.47 % m/m with respect to the total mass of the nutraceutical)
*Cucurbita seeds* - 40.19 g (47.28 % m/m with respect to the total mass of the nutraceutical).

To these 75 g, 10 g of *Plukenetia volubilis* oil are added (11.76 % m/m with respect to the mass of the nutraceutical).

The mixing is continued until the homogeneity of the components is achieved. The product is placed in a rectangular mold and refrigerated at a temperature of 2 °C for 12 hours. It is then stored at room temperature.

For V8, 65 g of the mixture of the emulsion with the seeds is taken, so in this mass of emulsion and seeds it can be calculated how its components are in quantities and, in parentheses, the relation to the total mass of the nutraceutical of this variant V8:

water - 5.80 g (7.88 % m/m in relation to the total mass of the nutraceutical)
sucrose - 18.10 g (24.59 % m/m with respect to the total mass of the nutraceutical)
soy lecithin - 0.12 g (0.16 % m/m with respect to the total mass of the nutraceutical)
*Plukenetia volubilis* oil - 6.70 g (9.10 % m/m with respect to the total mass of the nutraceutical)
ethanolic extract of *Curcuma longa* - 0.35 g (0.48 % m/m with respect to the total mass of the nutraceutical)
*Cucurbita seeds* - 34.82 g (47.31 % m/m with respect to the total mass of the nutraceutical).

8.6 g of *Plukenetia volubilis* oil (11.68 % m/m with respect to the mass of the nutraceutical) is added.

For Variant 9 (V9), 41 g of the emulsion mixture with the seed mass (macroemulsion) were taken and 6.47 g of *Cocos nucifera* oil (13.63 % of the macroemulsion mass) were added.

In this mass of emulsion and seeds it can be calculated how its components are in quantities and, in parentheses, its relation to the total mass of the nutraceutical of this variant 9 V9:

water - 3.66 g (7.71 % m/m in relation to the total mass of the nutraceutical)
sucrose - 11.42 g (24.06 % m/m with respect to the total mass of the nutraceutical)
soy lecithin - 0.07 g (0.15 % m/m with respect to the total mass of the nutraceutical)
*Plukenetia volubilis* oil - 3.66 g (7.71 % m/m with respect to the total mass of the nutraceutical)
ethanolic extract of *Curcuma longa* - 0.22 g (0.46 % m/m with respect to the total mass of the nutraceutical)
*Cucurbita seeds* - 21.97 g (46.28 % m/m with respect to the total mass of the nutraceutical).

The chemical composition is shown in Table 20.

Table 20. Chemical composition of nutraceutical variant V9

| Total solids, % | Total fat, % | Carbohydrates, % | pH |
|---|---|---|---|
| 95.06 | 46.53 | 19.55 | 6.48 |

Sensory evaluation of the nutraceutical

It was carried out with the help of a panel of 11 members, familiar with the product, evaluating the sensory characteristics according to a hedonic scale (from 0 to 5):
The odor attribute obtained a value of 2.9 points corresponding to "acceptable but not very marked". The taste "acceptable and characteristic" (2.2) and the color, also "acceptable" (2.9); the consistency "pleasant", "the components are not separated" (3.9). "Easy to chew" (chewiness, 3); "adequate rigidity" and "keeps its shape" (adhesiveness, 4.6) and homogeneity (3.8). The product was "acceptable" and "pleasant" for all panelists.

The most marked difference for V8 and V9 were the attributes homogeneity and adhesiveness, which were slightly improved.

This demonstrates that the addition of a quantity of *Cocos nucifera* oil to the emulsion mixture with the nutraceutical seed mass does not impair the organoleptic quality of the product, and, on the contrary, benefits the organoleptic characteristics of the final nutraceutical and provides an important content of medium chain fatty acids with a neuroprotective function.

Variants V7 and V8 contain 3 different sources of fatty acids, phytosterols and triacylglycerols, including *Cucurbita moschata* seeds, *Plukenetia volubilis* oil and soy lecithin, while V9 contains 4, due to the addition of *Cocos nucifera* oil. The final nutraceutical product of variants V7, V8 and V9 contains 3 components with antioxidant activity, including *Cucurbita moschata* seeds, *Curcuma longa* extracts as part of the emulsion, soy lecithin as part of the emulsion. In the final composition of the nutraceutical according to variants V7, V8 and V9 1 source of hypoglycemic compounds is included, specifically the seeds of *Cucurbita moschata.*

In these variants V7, V8 and V9, different sources of anticholesterolemic compounds are used, specifically *Plukenetia volubilis* oil and soy lecithin. To form the nutraceutical according to variants V7, V8 and V9, sources of anti-inflammatory compounds are used, including those contained in the seeds of *Cucurbita moschata.* In the composition of all three variants (V7, V8 and V9), sources of proteins of plant origin, such as seeds of *Cucurbita moschata,* are used.

The nutraceutical formulation executed according to variants V7, V8 and V9 contains components that provide vitamins, macro- and micro-elements in sufficient quantities to supply different deficiencies of them or to maintain the necessary physiological level, specifically Cucurbita seeds, which provide vitamins A, K, E and folic acid; soy lecithin, which provides choline; ethanolic extract of *Curcuma longa,* which provides Na, K, Mg, Ca, Fe. The nutraceutical compositions of this example (V7, V8 and V9) contain substances that provide organoleptic characteristics required by the people who consume it, specifically *Plukenetia volubilis* oil, which, besides masking the flavor of the *Curcuma longa* extract, gives the nutraceutical a softer consistency on the palate, increases adhesiveness, makes the nutraceutical easier to swallow and facilitates chewing. On the other hand, the sucrose used provides sweetness to the composition, which eliminates or reduces the bitter taste characteristic of *Curcuma longa* extract.

In the final formulation of these three variants, *Plukenetia volubilis* oil is included, which contains fatty acids of 6 to 12 carbons, and from which ketone bodies are obtained, which, in the face of insulin resistance in patients with Alzheimer's disease, provide energy to the neurons. The nutraceutical composition obtained according to variants V7, V8 and V9 and by the results of Example 1, the nutraceutical can be used for the prevention or treatment of diseases, disorders or syndromes comprising a cognitive deficit, such as Alzheimer's Disease, mental, behavioral and developmental disorders, including depressive and nervous system disorders. These actions are linked to the presence of medium-chain and omega fatty acids, to the components with antioxidant, anti-inflammatory and hypoglycemic activity, to the presence of Zn, Ca and vitamins, especially A, B and C.

Likewise, the nutraceutical is used for disorders of the digestive system, due to the vitamins, proteins, antibacterial, antifungal, vermicidal compounds and suitable lipids. It is used for the treatment of diseases and disorders of the immune and endocrine systems, due to the Zn content in certain amino acids, especially tryptophan and the anti-inflammatory substances of its components, among other elements with biological activity. Due to the capacity of its components and the product itself to lower triglycerides and cholesterol, in addition to the aforementioned factors, it is used for disorders of the circulatory system, cardiovascular and cerebrovascular diseases. It can also be used in bacterial, fungal and parasitic infectious diseases, due to the antibacterial, antifungal and vermicidal compounds of its components and due to its proven stability.

The amino acid composition, its micro-macro-elements, vitamins and antioxidants, among other components, allow it to be used for the treatment of oncological and blood diseases and, in general, due to its content in fats, carbohydrates, proteins, vitamins and minerals, it is highly recommended to treat tiredness, fatigue and malnutrition.

Example 11

Nutraceutical composition according to new variants V10 and V11

Variant 10 (V10)

To prepare the emulsion, the aqueous liquid phase consisting of *Stevia rebaudiana* extract containing steviosides dissolved in water (2 g of extract, corresponding to 1.99 % m/m of the nutraceutical mass) is selected as sweetener and 18 g of water (corresponding to 17.92 % m/m of the nutraceutical) are added. As an emulsifier and foaming agent, 0.4 g of soy lecithin is added (0.4 % m/m with respect to the mass of the nutraceutical).

The components are stirred and mixed at a stirring speed of 4,000 1/min to obtain nanometer-sized droplets of the aqueous phase with the oily phase, but particles larger than 200 nm of the solid phase of the Curcuma longa extract remain suspended.

When these components are agitated, the gas phase (air) is incorporated in a volume of 30 % v/v of the emulsion, which represents 12 % of the total volume of the nutraceutical. For this preparation stage, the emulsion is kept at a temperature of 25 °C.

Subsequently, the oily phase of the emulsion is prepared with soluble solids in it, in this case ethanolic extract of *Curcuma longa.* The oily phase is composed of 18 g of *Plukenetia volubilis* oil (17.93 % m/m with respect to the mass of the nutraceutical) and 2 g of ethanolic extract of *Curcuma longa* (1.99 % m/m with respect to the mass of the nutraceutical). At this stage of preparation, the emulsion is kept at a temperature of 25 °C.

In this way a 4-phase emulsion variant is obtained (aqueous, oily, air and suspended or solubilized solid), specifically: aqueous:oily:gaseous:solubilized solids in the aqueous phase and suspended solids in the oily phase.

In parallel, the *Cucurbita pepo* seed mass is prepared. The seeds are processed shelled, ground and roasted. The seeds are dried at a temperature of 80 °C for 60 min. Roasting of the seeds is carried out at a temperature of 130 °C. The seeds are milled in a blade mill and particles of a size between 10 $\mu$m to 30 $\mu$m are obtained.

The emulsion is mixed with the seed mass to form a food paste type macro-emulsion. To the mass of shelled, roasted and ground seeds (54 g, equivalent to 53.79 % m/m with respect to the mass of the nutraceutical), 40.4 g of the emulsion (40.24 % m/m with respect to the total mass of the nutraceutical) are added. The mixing of the microemulsion with the seed mass to form a macroemulsion with these components of the formulation is carried out manually by applying enveloping movements. To the mixture of seeds and emulsion, 6 g of *Theobroma cacao* powder (ratio 5.98 % m/m with respect to the nutraceutical mass) is added. The mixing with all the components of the final formulation of the nutraceutical is carried out manually applying enveloping movements.

Subsequently, the emulsion mixture is cooled with the mass of seeds and oils that make up the nutraceutical obtained, with all its components, initially at room temperature and subsequently at a temperature of 8 °C, for an interval of 5 h to 24 h. Once the dough is cooled, it is cut and portioned.

Variant 11 (V11)

As the aqueous liquid phase is selected and 2 g of *Stevia rebaudiana* extract are added as a sweetener, containing the steviosides dissolved in water (corresponding to 1.99 % m/m of the mass of the nutraceutical) and 18 g of water are added (corresponding to 17.92 % m/m of the nutraceutical). As an emulsifier, and at the same time foaming agent, 0.4 g of commercial *Saponaria officinalis* root extract is added (0.4 % m/m with respect to the mass of the nutraceutical). The components are stirred and mixed at a speed of 4,000 1/min.

When these components are stirred, the gaseous phase (air) is incorporated by volume of 30 % v/v of the emulsion and this represents 12.12 % with respect to the total volume of the nutraceutical. For this preparation step, the emulsion is kept at a temperature of 25 °C.

Subsequently, the oil phase of the emulsion is prepared with soluble solids in it, in this case of ethanolic extract of *Curcuma longa.* The oily phase is composed of 18 g of *Plukenetia volubilis* oil (17.93 % m/m with respect to the mass of the nutraceutical) and 2 g of ethanolic extract of *Curcuma longa* (1.99 % m/m with respect to the mass of the nutraceutical). In this preparation phase the emulsion is kept at a temperature of 25 °C.

In this way a 4-phase emulsion variant is obtained (aqueous, oily, air and suspended or solubilized solid), specifically: aqueous:oily:gaseous:solubilized solids in the aqueous phase and suspended solids in the oily phase.

In parallel, the mass of the *Cucurbita pepo* seed is prepared. The seeds are processed shelled, ground and roasted. The seeds are dried at a temperature of 80 °C for 60 min. The roasting of the seeds is carried out at a temperature of 130 °C. The milling of the seeds is carried out in a blade mill and particles of size between 10 $\mu$m and 30 $\mu$m are obtained. The emulsion mixture is formed with the seed mass forming a macro-emulsion type food paste. To the mass of the shelled, roasted and ground seeds (54 g, equivalent to 53.79 % m/m with respect to the mass of the nutraceutical) 40.4 g of the emulsion is added (40.23 % m/m with respect to the total mass of the nutraceutical).

The mixing of the microemulsion with the seed mass to form a macroemulsion with these components of the formulation is executed manually, applying enveloping movements. To the mixture of the seeds and the emulsion are incorporated 6 g of *Theobroma cocoa* powder (ratio 5.98 % m/m with respect to the mass of the nutraceutical). The mixture with all the components of the final formulation of the nutraceutical is executed manually by applying enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the mass is cooled, cutting and portioning proceed.

The final composition of the emulsion of the V10 variant contains 7 components of different nature (water, air, *Plukenetia volubilis* oil, *Stevia extract,* soy lecithin, *Curcuma longa* extract and *Theobroma cocoa* powder) and the V11 also contains 7 components, but differs from V10 in that the extract of *Saponaria officinalis* is used as an emulsifier.

In these two variants, more than one component performs various biological or functional functions, such as *Plukenetia volubilis* oil, *Curcuma longa* extract, soy lecithin and egg white, as described above for each component.

In the V10 variants the nutraceutical contains four different sources of fatty acids, phytosterols and triacylglycerols, which are *Cucurbita moschata* seeds, *Plukenetia volubilis* oil, soy lecithin and *Theobroma cocoa* powder, while the V11 contains three sources, because lecithin is absent.

The final nutraceutical product of variants 10 and 11 (V10 and V11) contains three components with antioxidant activity, which are *Cucurbita moschata* seeds, *Curcuma longa* extracts, as part of the emulsion, and *Stevia rebaudiana* extract, as part of the emulsion. The final composition of the two variants of the nutraceutical includes a source of hypoglycemic compounds, specifically *Cucurbita moschata* seeds, while a source of anticholesterolemic compounds is used, specifically *Plukenetia volubilis* oil.

To make up the nutraceutical, sources of anti-inflammatory compounds are used, including *Stevia rebaudiana* extract, forming part of the emulsion, and *Cucurbita moschata* seeds. In both compositions, V10 and V11, protein sources of vegetable origin are used, such as *Cucurbita moschata* seeds. Both variants show compositions that contain components that provide vitamins, macro- and micro-elements in sufficient quantities to supply different deficiencies of them, or to maintain the necessary physiological level, specifically *Cucurbita seeds,* which provide vitamins A, K, E and folic acid, soy lecithin, which provides choline and *Curcuma longa* extract, which provides Na, K, Mg, Ca and Fe.

The nutraceutical composition obtained according to both variants contains substances that provide organoleptic characteristics such as odor, color. taste, chewability and swallowing, with values higher than 3 points on a scale of 0 to 5, during the organoleptic evaluation that is considered to satisfy the needs of the subjects and patients who consume it. Specifically, *Plukenetia volubilis* oil, in addition to masking the taste of *Curcuma longa* extract, gives the nutraceutical a smoother consistency, increases adhesiveness, makes the nutraceutical more easily swallowable and facilitates chewability. On the other hand, the *Stevia rebaudiana* extract used in V2 provides sweetness to the composition, which eliminates or diminishes the characteristic flavor of the *Curcuma longa* extract.

The final formulation includes *Plukenetia volubilis* oil containing 6 to 12 carbon fatty acids.

As in previous examples, the nutraceutical composition obtained according to variants V10 and V11, can also be used for the prevention or treatment of diseases, disorders or syndromes, comprising a cognitive deficit, such as Alzheimer's Disease, mental, behavioral and developmental disorders, including depressive disorders and disorders of the nervous system, all this uses linked to the presence of medium-chain and omega fatty acids, to the components with antioxidant, anti-inflammatory and hypoglycemic activity, the presence of Zn, Ca and vitamins, especially A, B and C. Likewise, the nutraceutical is useful to treat disorders of the digestive system, due to its content of vitamins, proteins, antibacterial, antifungal and vermicidal compounds and suitable lipids, as well as for the treatment of diseases and disorders of the immune and endocrine systems thanks to the content of Zn in certain amino acids, especially tryptophan and by the anti-inflammatory substances of its components, among other elements with biological activity.

Due to the ability of its components and the product itself to lower triglycerides and cholesterol, in addition to the aforementioned factors, it is suitable for disorders of the circulatory system, cardiovascular and cerebrovascular diseases, and can be used in bacterial, fungal and parasitic infections due to the antibacterial, antifungal and vermicidal compounds of its components and the stability demonstrated.

The aminoacid composition, its micro and macro-elements, vitamins and antioxidants, among other components, is suitable for the treatment of oncological and blood diseases.

In general, its content in fats, carbohydrates, proteins, vitamins and minerals is highly recommended for tiredness, fatigue and malnutrition. The organoleptic evaluation showed a marked turmeric flavor and a somewhat bitter aftertaste characteristic of *Stevia,* which can be assimilated by subjects accustomed to the consumption of turmeric-based products.

The chemical composition is shown in Table 21.

Table 21. Chemical composition of the V10 variant of nutraceutical

| Total solids, % | Proteins, % | Total Fats, % | Ashes, % | Chlorides, % |
|---|---|---|---|---|
| 89.19 | 10.63 | 47.81 | 4.6 | 0.14 |

Example 12

Variant 12 (V12)

To prepare the emulsion, the aqueous liquid phase consisting of 27 g sucrose as sweetener (corresponding to 15.49 % m/m of the nutraceutical mass) is selected and 13 g water (7.46 % m/m of the nutraceutical) is added. It is dissolved by heating and cooled to 40 °C.

As emulsifier and at the same time foaming agent, 0.3 g of soy lecithin (0.17 % m/m with respect to the mass of the nutraceutical) is added.

The components are stirred and mixed at a stirring speed of 20,000 1/min to obtain millimeter-sized droplets of the aqueous phase with the oily phase, but particles of more than 200 nm of the solid phase of the *Curcuma longa* extract, which was not dissolved but suspended in the oily phase, remain suspended.

By stirring these components, the gas phase (air) is incorporated in volume of 30 % v/v of the emulsion and reaches 16.3 % with respect to the total volume of the nutraceutical. For this preparation stage, the emulsion is kept at a temperature of 25 °C. Subsequently, the oily phase of the emulsion is prepared with soluble solids in it, in this case ethanolic extract of *Curcuma longa.* The oily phase is composed of 13 g of *Plukenetia volubilis* oil (7.46 % m/m with respect to the mass of the nutraceutical) and 1 g of ethanolic extract of *Curcuma longa* (0.58 % m/m with respect to the mass of the nutraceutical). In this preparation phase, the emulsion is kept at a temperature of 25 °C.

In this way a 4-phase emulsion variant is obtained (aqueous, oily, air and suspended or solubilized solid), specifically: aqueous:oily:gaseous:solubilized solids in the aqueous and suspended solids in the oily.

In parallel, the mass of whole *Cucurbita pepo* seeds, with their shells, is prepared, roasted and ground to incorporate 54 g to the emulsion, and mixed with 54 g of shelled *Cucurbita moschata* seeds (61.96 % of the emulsion mass with respect to that of the nutraceutical).

The emulsion is mixed with the seed mass to form a food paste-like macro-emulsion. To the mass of shelled, roasted and ground seeds, 54.3 g of the emulsion are added (31.15 % m/m with respect to the total mass of the nutraceutical). The mixing of the microemulsion with the mass of seeds to form a macroemulsion with these components of the formulation is carried out manually by applying enveloping movements. To the mixture of seeds and emulsion, 12 g of *Theobroma cacao* powder (6.88 % m/m with respect to the mass of the nutraceutical) is added. The mixing with all the components of the final formulation of the nutraceutical is carried out manually applying enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the mass is cooled, it is cut and portioned.

The chemical composition is shown in Table 22.

Table 22. Chemical composition of nutraceutical variant V12

| Total solids, % | Proteins, % | Total Fats, % | Ashes, % | Chlorides, % |
|---|---|---|---|---|
| 89.19 | 18.75 | 40.47 | 4.0 | 0.12 |

Sensory evaluation of the nutraceutical

The sensory evaluation was carried out with the help of a panel of 7 members familiar with the product, the results of which are shown below:

The odor attribute obtained a value of 2.71 points corresponding to "acceptable, but not very marked". The taste "acceptable, but characteristic", although somewhat bitter (3.9); the color, also "acceptable" (3); the consistency "pleasant", the components are not separated (3.9). "Easy to chew" (chewiness, 3); rigidity "adequate" and maintains its shape (4.43); adhesiveness "adequate" (4.14) and homogeneity (3.71). The product was "acceptable" and pleasant for all panelists.

This example demonstrates that whole and shelled processed seeds can be combined and with different ratios between components, both of the initial emulsion and of the nutraceutical macroemulsion.

Example 13

Preparation of the nutraceutical with a new variant 13 (V13)

To prepare the emulsion, the aqueous liquid phase consisting of sucrose as sweetener (57 g, 26.6 % m/m of the nutraceutical mass) is selected and 18 g of water (8.4 % m/m of the nutraceutical) is added. It is dissolved by heating and cooled to 40 °C.

As emulsifier and at the same time foaming agent, 0.3 g of soy lecithin (0.14 % m/m with respect to the mass of the nutraceutical) is added.

The components are stirred and mixed at a stirring speed of 20,000 1/min to obtain millimeter-sized droplets of the charged phase with the oily phase, but particles of more than 200 nm of the solid phase of the *Curcuma longa* extract, which was not dissolved but suspended in the oily phase, remain suspended.

By stirring these components, the gas phase (air) is incorporated in a volume of 30 % v/v of the emulsion and reaches 13.2 % with respect to the total volume of the nutraceutical. For this preparation stage, the emulsion is kept at a temperature of 25 °C.

Subsequently, the oily phase of the emulsion is prepared with soluble solids in it, in this case ethanolic extract of *Curcuma longa.* The oily phase is composed of 18 g of *Plukenetia volubilis* oil (8.4 % m/m with respect to the mass of the nutraceutical) and 1 g of ethanolic extract of *Curcuma longa* (0.47 % m/m with respect to the mass of the nutraceutical). In this preparation phase, the emulsion is kept at a temperature of 25 °C.

In parallel, the mass of whole seeds of *Cucurbita pepo* is prepared, soaked in water at a temperature of 60 °C for 30 min, drained for 2 h and dried in an oven at 80 °C. They are ground and roasted in a metal container at a temperature above 90 °C. 108 g of whole seeds (50.4 % m/m with respect to the mass of the nutraceutical) with shells, dried, ground and roasted, are obtained for incorporation into the emulsion. The emulsion is mixed with the seed mass to form a food paste type macro-emulsion. To the mass of shelled, roasted and ground seeds, 94.3 g of the emulsion are added (44 % m/m with respect to the total mass of the nutraceutical).

The mixing of the microemulsion with the mass of seeds to form a macroemulsion with these components of the formulation is carried out manually by applying enveloping movements. To the mixture of seeds and emulsion, 12 g of *Theobroma cacao* powder (5.6 % m/m with respect to the mass of the nutraceutical) is added. The mixing with all the components of the final formulation of the nutraceutical is carried out manually applying enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the mass is cooled, it is cut and portioned.

The chemical composition is shown in Table 23.

Table 23. Chemical composition of the V13 nutraceutical variant

| Total solids, % | Proteins, % | Total fats, % | Carbohidratos, % | Ashes, % | Chlorides, % |
|---|---|---|---|---|---|
| 88.61 | 10.63 | 27.22 | 20.68 | 3.7 | 0.18 |

Sensory evaluation of the nutraceutical

A panel of 7 members familiar with the product evaluated the product, with the following results: the odor attribute obtained a value of 3.42 points, which corresponds to "adequate". The taste "acceptable and characteristic" (3.3) and the color equally "acceptable" (2.85); the consistency "pleasant, the components do not separate" (3). The product was "easy to chew" (chewiness 3.14); rigidity was "adequate and maintains its shape" (3.85); adhesiveness (3.42) and homogeneity (3.57). The product was "acceptable" and pleasant for all panelists.

Example 14

Variant 14 (V14) is prepared.

To prepare the emulsion, the aqueous liquid phase consisting of *Apis mielifera* honey as sweetener (40 g, 21.34 % m/m of the nutraceutical mass) is selected to form the emulsion (ratio of oily phase with suspended solids:aqueous with soluble solids) and 18 g of water (9.61 % m/m of the nutraceutical) are added. As emulsifier and at the same time foaming agent, 0.4 g of soy lecithin (0.21 % m/m with respect to the mass of the nutraceutical) is added.

The components are stirred and mixed at a stirring speed of 20,000 1/min. When these components are stirred, the gaseous phase (air) is incorporated in a volume of 30 % v/v of the emulsion, which reaches 12.4 % with respect to the total volume of the nutraceutical. For this preparation stage, the emulsion is kept at a temperature of 25 °C.

Subsequently, the oily phase of the emulsion is prepared with soluble solids in it, in this case ethanolic extract of *Curcuma longa.* The oily phase is composed of 18 g of *Plukenetia volubilis* oil (9.61 % m/m with respect to the mass of the nutraceutical) and 1 g of ethanolic extract of *Curcuma longa* (0.53 % m/m with respect to the mass of the nutraceutical). In this preparation phase, the emulsion is kept at a temperature of 25 °C.

In parallel, the mass of *Cucurbita pepo* seeds without shells, ground, roasted and ground again is prepared. 104 g of seeds are used for incorporation into the emulsion (55.5 % m/m with respect to the nutraceutical mass) and mixed with the emulsion. To the mass of the shelled, roasted and ground seeds, 77.4 g of the emulsion are added (39.7 % m/m with respect to the total mass of the nutraceutical). The mixing of the microemulsion with the mass of seeds to form a macroemulsion with these components of the formulation is carried out manually by applying enveloping movements. To the mixture of seeds and emulsion, 6 g of *Theobroma cacao* powder (3.2 % m/m with respect to the nutraceutical mass) are added. The mixing with all the components of the final formulation of the nutraceutical is carried out manually applying enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the dough is cooled, it is cut and portioned. The addition of honey provides a pleasant flavor to the nutraceutical that helps to diminish the characteristic flavor of the *Curcuma* extract. The combination with cocoa powder improves the organoleptics of the product which was accepted by the tasters.

The chemical composition is shown in Table 24.

Table 24. Chemical composition of nutraceutical variant V14.

| Total solids, % | Proteins, % | Total fats, % | Carbohidratos, % | Ashes, % | Chlorides, % |
|---|---|---|---|---|---|
| 92.72 | 16.88 | 35.20 | 12.20 | 4.8 | 0.18 |

Example 15

Variant 15 (V15) is prepared.

To prepare the emulsion, the aqueous liquid phase consisting of *Apis mielifera* honey as sweetener (40 g, 21.82 % m/m of the mass of the nutraceutical) is selected to form the emulsion (ratio of oily phase with suspended solids:aqueous with soluble solids) and 18 g of water (10.72 % m/m of the nutraceutical) are added.

As emulsifier and at the same time foaming agent, 0.4 g of soy lecithin (0.24 % m/m with respect to the mass of the nutraceutical) is added.

The components are stirred and mixed at a stirring speed of 20,000 1/min. While stirring these components, the gas phase (air) is incorporated in a volume of 30 % v/v of the emulsion, which reaches 13.92 % with respect to the total volume of the nutraceutical. For this preparation stage, the emulsion is kept at a temperature of 25 °C. Subsequently, the oily phase of the emulsion is prepared with soluble solids in it, in this case ethanolic extract of *Curcuma longa.* The oily phase is composed of 18 g of *Plukenetia volubilis* oil (10.72 % m/m with respect to the mass of the nutraceutical) and 1.5 g of ethanolic extract of *Curcuma longa* (0.89 % m/m with respect to the mass of the nutraceutical). In this preparation phase, the emulsion is kept at a temperature of 25 °C.

In parallel, the mass of shelled, roasted and ground *Cucurbita pepo* seeds is prepared. 84 g of seed are used for incorporation into the emulsion (50.03 % m/m with respect to the nutraceutical mass) and mixed with the emulsion. To the mass of the shelled, roasted and ground seeds, 77.9 g of the emulsion is added (46.40 % m/m with respect to the total mass of the nutraceutical).

The mixing of the microemulsion with the mass of seeds to form a macroemulsion with these components of the formulation is carried out manually by applying enveloping movements. To the mixture of seeds and emulsion, 6 g of *Theobroma cacao* powder (3.57 % m/m with respect to the mass of the nutraceutical) is added. The mixing with all the components of the final formulation of the nutraceutical is carried out manually applying enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the mass is cooled, it is cut and portioned.

The chemical composition is shown in Table 25.

Table 25. Chemical composition of the nutraceutical variant V15

| Total solids, % | Proteins, % | Total fats, % | Carbohidratos, % | Ashes, % | Chlorides, % |
|---|---|---|---|---|---|
| 92.94 | 13.75 | 40.32 | 20.68 | 3.7 | 0.17 |

Example 16

Variant 16 (V16) is prepared.

To prepare the emulsion, the integrated aqueous liquid phase consisting of a fructooligosaccharide is selected as sweetener (50 g, 23.54 % m/m mass of the nutraceutical) to form the emulsion (ratio oil phase with suspended solids:aqueous with soluble solids) and 18 g of water (8.47 % m/m of the nutraceutical) are added.

As emulsifier and at the same time foaming agent, 0.4 g of soy lecithin (0.19 % m/m with respect to the mass of the nutraceutical) was added.

The components were stirred and mixed at a stirring speed of 20,000 1/min. While stirring these components, the gas phase (air) is incorporated in a volume of 30 % v/v of the emulsion, which reaches 12.35 % with respect to the total volume of the nutraceutical. For this preparation stage, the emulsion is kept at a temperature of 25 °C. Subsequently, the oily phase of the emulsion is prepared with soluble solids in it, in this case ethanolic extract of *Curcuma longa.* The oily phase is composed of 18 g of *Plukenetia volubilis* oil (8.47 % m/m with respect to the mass of the nutraceutical) and 1.0 g of ethanolic extract of *Curcuma longa* (0.47 % m/m with respect to the mass of the nutraceutical). In this preparation phase, the emulsion is kept at a temperature of 25 °C.

In parallel, the mass of *Cucurbita pepo* seeds shelled, roasted and ground, is prepared. 104 g of seeds are used for incorporation into the emulsion (48.96 % m/m with respect to the nutraceutical mass) and mixed with the emulsion. To the mass of the shelled, roasted and ground seeds, 87.4 g of the emulsion are added (41.15 % m/m with respect to the total mass of the nutraceutical). The mixing of the microemulsion with the mass of seeds to form a macroemulsion with these components of the formulation is carried out manually by applying enveloping movements. To the mixture of seeds and emulsion are added 6 g of *Theobroma cacao* powder (2.82 % m/m with respect to the mass of the nutraceutical), 4 g of *Plukenetia volubilis* (1.88 % m/m with respect to the mass of the nutraceutical) and 11 g of shelled, roasted and ground *Pronus dulcis* fruits (5.18 % with respect to the total mass of the nutraceutical). Mixing with all the components of the final nutraceutical formulation is carried out manually using enveloping movements.

Subsequently, the emulsion mixture is cooled with the mass of seeds and oils that make up the nutraceutical obtained with all its components, initially at room temperature and subsequently at a temperature of 8 °C, for an interval of 5 h to 24 h. Once the mass is cooled, it is cut and portioned.

The chemical composition is shown in Table 26.

Table 26. Chemical composition of nutraceutical variant V16

| Total solids, % | Proteins, % | Total fats, % | Carbohidratos, % | Ashes, % | Chlorides, % |
|---|---|---|---|---|---|
| 91.80 | 19.38 | 35.16 | 18.87 | 3.85 | 0.18 |

The taste of this variant was pleasant to the palate due to the addition of *Pronus dulcis* and no separation was observed; on the contrary, the fruit mass integrated very well with the nutraceutical mass.

The fruits of *Pronus dulci,* in addition to their role as a component to improve flavor and odor, provide monounsaturated fatty acids, calcium, vitamins E and C, and are a source of antioxidants, in addition to those already included in other examples.

Example 17

Variant 17 (V17) is prepared.

To prepare the emulsion, the aqueous liquid phase consisting of *Apis mielifera* honey as sweetener (40 g, corresponding to 20.30 % m/m of the mass of the nutraceutical) is selected to form the emulsion (ratio of oily phase with suspended solids: aqueous with soluble solids) and 18 g of water (corresponding to 9.13 % m/m of the nutraceutical) are added.

As an emulsifier and foaming agent, 0.4 g of soy lecithin is added (0.2 % m/m with respect to the mass of the nutraceutical). The components are agitated and mixed at an agitation speed of 20,000 1/min. When these components are stirred, the gas phase (air) is incorporated in a volume of 30 % v/v of the emulsion, which represents 11.73 % with respect to the total volume of the nutraceutical. For this preparation stage, the emulsion is kept at a temperature of 25 °C.

Subsequently, the oily phase of the emulsion is prepared with soluble solids in it, in this case ethanolic extract of *Curcuma longa.* The oily phase is composed of 18 g of *Plukenetia volubilis* oil (9.13 % m/m with respect to the mass of the nutraceutical) and 1.0 g of ethanolic extract of *Curcuma longa* (0.51 % m/m with respect to the mass of the nutraceutical). In this preparation phase, the emulsion is kept at a of temperature 25 °C.

In parallel, the mass of *Cucurbita pepo* seeds, shelled, roasted and ground, is prepared. One hundred and four (104) g of seed are used for incorporation into the emulsion (52.78 % m/m with respect to the nutraceutical mass) and mixed with the emulsion. To the mass of the deshelled seeds, already roasted and ground, 77.04 g of the emulsion is added (39.10 % m/m with respect to the total mass of the nutraceutical).

The mixing of the microemulsion with the mass of seeds is carried out manually by applying enveloping movements to form a macroemulsion with these components of the formulation. To the mixture of seeds and emulsion, 6 g of *Theobroma cacao* powder (ratio 3.05 % m/m with respect to the nutraceutical mass), 4 g of *Plukenetia volubilis* (ratio 2.03 % m/m with respect to the nutraceutical mass) and 6 g of *Moringa oleifera* leaf powder (3.05 % with respect to the total nutraceutical mass) are added.

Mixing with all the components of the final nutraceutical formulation is carried out manually using enveloping movements. Subsequently, the emulsion mixture is cooled with the mass of seeds and oils that make up the nutraceutical obtained with all its components, initially at room temperature and then at a temperature of 8 °C, for an interval of 5 to 24 hours. Once the mass is cooled, it is cut and portioned.

Example 18

Variant 18 (V18) is prepared from variant V17 and is highly similar to it.

*Apis mielifera* honey is selected as sweetener (40 g), 18 g water is added. As emulsifier, and at the same time foaming agent, 0.4 g of *Agave fourcroydes* leaf extract is added. The components that trap the air and keep it in the composition are stirred and mixed and proceed as in V17. The oil phase of the emulsion is composed of 18 g of *Plukenetia volubilis* oil and 1.0 g of *Curcuma longa* ethanolic extract. The mass of shelled, roasted and ground *Cucurbita pepo* seeds (104 g) is prepared and mixed with the emulsion (77.04 g). Six (6) g of *Theobroma cacao* powder and 4 g of *Plukenetia volubilis* are added. The process is continued as in variant V 17 until the final product is obtained.

Example 19

Variant 19 (V19) is prepared from variant V17 and is highly similar to it.

*Apis mielifera* honey is selected as sweetener (40 g), 18 g of water are added. As emulsifier, and at the same time foaming agent, 0.4 g of soy lecithin is added. The components are stirred and mixed and proceed as in V17. The oil phase of the emulsion is composed of 18 g of *Plukenetia volubilis* oil and 0.8 g of *Curcuma longa* ethanolic extract and 0.2 g of *Pipper nigrum* extract. In addition, it contains air as part of the emulsion. The mass of the shelled, roasted and ground *Cucurbita pepo* seeds (104 g) is prepared and mixed with the emulsion (77.04 g). Six (6) g of *Theobroma cacao* powder and 4 g of *Plukenetia volubilis* are added. The process is continued as in variant V17 until the final product is obtained.

Example 20

Variant 2 (V2) is prepared from variant V17 and is highly similar to it.

*Apis mielifera* honey is selected as sweetener (40 g), 18 g water is added. As emulsifier, 0.4 g of soy lecithin is added. The components are stirred and mixed and proceed as in V17. The oil phase of the emulsion is composed of 18 g of *Plukenetia volubilis* oil and 1.0 g of *Curcuma longa* ethanolic extract. The emulsion contains the air trapped during the process. The mass of the shelled, roasted and ground *Cucurbita pepo* seeds (104 g) is prepared and mixed with the emulsion (77.04 g). The process is continued according to variant V17; 6 g of *Theobroma cacao* powder, 4 g of *Plukenetia volubilis* and 6 g of *Coccus nucifera* fruit mass are added. The process is continued as in variant V17 until the final product is obtained.

Example 21

Variant 21 (V21) is prepared from variant V17 and is highly similar to it.

The juice of the fruit of the *Agave tequilana* is selected as sweetener (40 g), 18 g of water are added. As emulsifier, 0.4 g of soy lecithin is added. The components are stirred and mixed and proceed as in V17. The oil phase of the emulsion is composed of 18 g of *Plukenetia volubilis* oil and 1.0 g of *Curcuma longa* ethanolic extract. The emulsion contains the air trapped during the process. The mass of the shelled, roasted and ground *Cucurbita pepo* seeds (104 g) is prepared and mixed with the emulsion (77.04 g). The process is continued according to variant V17; 6 g of *Theobroma cacao* powder and 4 g of *Plukenetia volubilis* are added. The process is continued as in variant V17 until the final product is obtained.

Example 22

Variant 22 (V22) is prepared from variant V17 and is highly similar to it.

*Apis mielifera* is selected as sweetener (40 g), 18 g of water are added. As emulsifier, 0.3 g of soy lecithin and 0.1 g of pectin are added. The components are stirred and mixed and proceed as in V17. The oil phase of the emulsion is composed of 18 g of *Plukenetia volubilis* oil and 1.0 g of *Curcuma longa* ethanolic extract. The emulsion contains the air trapped during the process. The mass is prepared from the shelled, roasted and ground *Cucurbita pepo* seeds (104 g) and mixed with the emulsion (77.04 g). The process is continued according to variant V17; 6 g of *Theobroma cacao* powder and 4 g of *Plukenetia volubilis* are added. The process is continued as in variant V17 until the final product is obtained.

Example 23

Variant 23 (V23) is prepared from variant V17 and is highly similar to it.

*Apis mielifera* is selected as sweetener (40 g), 18 g of water are added. As emulsifier, 0.4 g of soy lecithin is added. The components are stirred and mixed and proceed as in V17. The oil phase of the emulsion is composed of 18 g of *Plukenetia volubilis* oil, 0.9 g of *Curcuma longa* ethanolic extract and 0.1 g of *Theobroma cacao* extract. The emulsion contains the air trapped during the process. The mass of the shelled, roasted and ground *Cucurbita pepo* seeds (104 g) is prepared and mixed with the emulsion (77.04 g). The process is continued according to variant V17; 6 g of *Theobroma cacao* powder and 4 g of *Plukenetia volubilis* are added. The process is continued as in variant V17 until the final product is obtained.

Example 24

Variant 24 (V24) is prepared from variant V17 and is highly similar to it.

*Apis mielifera* is selected as sweetener (40 g), 18 g of water are added. As emulsifier, 0.4 g of *Agave* leaf extract is added. The components are stirred and mixed and proceed as in V17. The oil phase of the emulsion is composed of 18 g of *Plukenetia volubilis* oil, 0.9 g of *Curcuma longa* ethanolic extract and 0.1 g of Thebroma cacao extract. The emulsion contains the air trapped during the process. The mass of the shelled, roasted and ground *Cucurbita pepo* seeds (104 g) is prepared and mixed with the emulsion (77.04 g). The process is continued according to variant V17; 6 g of *Theobroma cacao* powder and 4 g of *Plukenetia volubilis* are added. The process is continued as in variant V17 until the final product is obtained.

Example 25

Variant 25 (V25) is prepared from variant V17 and is highly similar to it.

*Apis mielifera* is selected as sweetener (40 g), 18 g of water are added. As emulsifier, 0.4 g of soy lecithin was added. The components are stirred and mixed and proceed as in V17. The oil phase of the emulsion is composed of 18 g of *Plukenetia volubilis* oil, 0.9 g of ethanolic extract of *Curcuma longa* and 0.1 g of *Theobroma cacao* extract. The emulsion contains the air trapped during the process. The mass of the shelled, roasted and ground *Cucurbita pepo* seeds (104 g) is prepared and mixed with the emulsion (77.04 g). The process is continued according to variant V17; 6 g of *Theobroma cacao* powder, 4 g of *Plukenetia volubilis* and 6 g of whey protein are added to the mixture of seeds and emulsion. The process is continued as in variant V17 until the final product is obtained.

Other obvious advantages of the present invention are described below:

- the emulsions of the nanometric (5-200 nm) and millimetric (more than 200 nm) order obtained according to the present invention make it possible for the components incorporated in them to lose their color intensity and opacity, providing pleasant and attractive colors to the eye, which can be appreciated in the organoleptic evaluation of the different variants. The emulsions achieved with sizes smaller than 100 nm are more colorless and more translucent. In addition, the interfacial area is significantly increased, which in turn makes it possible to solubilize the biologically active substances of the composition soluble at the interface and increases the bioavailability of all the components,

- these achieved emulsions do not depend on lipolysis for digestion and for absorption in the intestine of the bioactive components of the composition, which leads to a faster release of the active ingredients,

- the curcuminoids contained in the product can pass the blood-brain barrier in greater quantities compared to previous solutions because they are accompanied by lipid combinations, microbiome protective and vermifuge substances, and anti-inflammatory compounds, which guarantee their adequate absorption at the intestinal level,

- a novel and beneficial effect achieved is that the natural emulsifiers used to achieve the nano or microemulsions are used in much smaller quantities than those described for surfactants and co-surfactants previously used, since it is reported that a disadvantage of microemulsions is that they require a relatively high amount of these substances with respect to the volume of oil,

- emulsions with 100 % emulsifying capacity and 100 % stability for more than 24 h are achieved. The overrun is 119 to 150 %, due to the capacity to trap the gas phase, which guarantees a smooth structure. We did not find such overrun percentages in nutraceuticals in the previous state of the art,

- the advantage of using components derived from plants with saponin content that guarantee the inclusion of large volumes of air in emulsions together with oils, especially soy lecithin, lies in the fact that they eliminate the hemolytic effect caused by the high volumes of saponins commonly used in emulsions, which have a hemolytic effect on red blood cells,

- the use of coconut oil to obtain the final mixture of the emulsion with the mass of seeds and oil (food emulsion), guarantees the presence of long chain triglycerides, since they provide a great stability to the food type emulsion with a tendency to avoid the aggregation of droplets and their increase in size due to the Ostwald phenomenon. This phenomenon, in the present invention does not follow the tendency described in previous solutions, characterized by preventing the obtaining of translucent or transparent nano- and micro-emulsions. On the other hand, the medium chain triglycerides present in *Plukenetia volubilis* oil and *Cocos nucifera* oil guarantee a high solubility of curcuminoids and other biological compounds of organic nature,

- like no other nutraceutical, the invention contains components involved in almost all the processes attributed to the origin of Alzheimer's disease and other neurological conditions (anti-inflammatory, hypoglycemic, cholesterol-lowering, antioxidant, alternative source of energy to counteract insulin resistance at the neuronal level, antibacterial, antifungal, vermifuge, anti-aggregation of beta-amyloid peptide, anti neurofibrillary tangle formation and promoter of neuronal synapses), as well as antihypertensive, antiviral, antitoxic, antiplatelet aggregation, cardiovascular protector and stabilizer of the intestinal microbiome and the content of minerals such as Zn,

- the main components are provided by several different substances, e.g.: fatty acids - from 3 to 5 sources of different nature and includes medium chain and long chain, antioxidants - from 3 to 8 sources of different chemical nature, anti-inflammatory - from 1 to 4 sources of different chemical structure, from 1 to 3 sources of hypoglycemic compounds, from 1 to 4 sources of anti-cholesterol compounds, so they employ different metabolic routes to achieve their effect and, if the subjects present some difficulty or concomitant disease that prevents the action of a compound, the other two routes can be used for their assimilation and action,

- the nutraceutical also contains a set of essential nutritional substances that support the body, as proteins of high nutritional value, essential amino acids, vital vitamins for neuronal functions such as E and those of group B; macro- and micro-elements such as Zn, Ca, Fe, Mg, Mn, P, K, which makes it an independent food. It especially contains significant levels of Zn and Mg, which are known to play a decisive role in cognitive processes,

- for nutritional balance, the presence of all the elements required by the body, including proteins, fats, carbohydrates, vitamins, micro- and macro-elements, among others, can be consumed several times a day with daily frequency for prolonged periods of time,

- the product has a significant content of tryptophan, an amino acid that produces serotonin and melatonin in the body, which, in turn, reduces anxiety and insomnia, characteristic of patients suffering from neurological disorders, especially Alzheimer's disease,

- all the components are of natural origin, especially of vegetable origin and in most of the variants do not contain components of animal origin, with the exception of egg white in some of its variants, or if required for very justified cases, whey proteins are incorporated, which makes them suitable for vegetarians or people who consume vegan or halal diets. In those cases in which it is necessary, animal proteins are added in appropriate proportions to meet the special requirements, without deterioration of the product's qualities,

- the invention is a new type of nutraceutical. There are few products with this presentation classified in the range of nutraceuticals; it has a food presentation (nutraceuticals are usually tablets, other types of tablets, liquids, capsules) and thus eliminates the perception that you are consuming a drug and stimulates the neuropsychological state, perceiving that you are not consuming drugs to treat any disease and can also be considered a functional food,

- the invention is most appropriate for elderly people, especially those with feeding coordination disorders, as it does not require chewing or minimal effort and is easy to swallow,

- the product contains quantities of the sources of the active substances in magnitudes that exceed those of common nutraceuticals, such as capsules and tablets, as it can be consumed from a few grams to 80 grams per intake or more than 200 g per day,

- the administration of the active components can be adapted through the amount of product to be consumed per unit intake or per daily intake, according to the purpose (preventive, support for sick people) and according to the stage of the disease (mild, moderate, severe),

- the product can be presented in various flavors to suit different tastes,
- the product is stable at room temperature and refrigerated for months,
- the production technology exists in many countries and there are factories ranging from artisanal to highly mechanized and automated,
- the raw materials are readily available on the world market and are in all cases derived from natural sources,
- the technological processes do not involve in any case toxic or harmful substances to the organism, do not generate waste that pollutes the environment,
- the inclusion of the selected seeds, with the compositions specified in the present invention and processed by the methods described herein guarantees a high fat absorption capacity (up to 50 %) and prevents further separation during processing and conservation of the nutraceutical,
- similarly, the inclusion of the seeds selected for the nutraceutical with the compositions specified in the present invention and processed by the methods described herein guarantees a high water absorption capacity (up to 50 %) and water retention, due to the presence of proteins and carbohydrates with hydrophilic properties, which helps not to decrease neither during the technological processing, nor during the conservation, maintaining the nutraceutical its mechanical and organoleptic properties, even at high temperatures,
- the preparation of the oil-in-water emulsion reinforces the effect described in the two previous paragraphs, since emulsions, and even more so micro- and nano-emulsions, prevent the separation of the oils and the aqueous phase,
- no other product having so many components (minimum 7) of such dissimilar nature (seeds, oils, powders, emulsions, liposoluble and hydrosoluble substances, emulsifiers, natural flavorings) shows a homogeneity, integrity and stability of its structure as that of the present invention,
- the drying and roasting parameters of the seeds cause the appearance of "pleasant" odors and flavors derived from the changes in the proteins due to the action of the melanoidinosis process and, in addition, preserve the internal gel of the seed, and the fine grinding favors that no significant phase separation process and loss of oil occurs,
- like no other nutraceutical, its preventive, supportive or therapeutic application covers cognitive deficit, Alzheimer's disease, mental, behavioral and developmental disorders, depressive disorders and disorders of the nervous, digestive, immune, endocrine, circulatory and cardiovascular systems; cerebrovascular, infectious, parasitic, oncological and blood diseases; fatigue and malnutrition,
- the use of ground or roasted and ground pumpkin seeds, walnuts, hazelnuts, or other fruits, avoids that small pieces of these are absorbed in the respiratory tract, or cause swallowing disorders and choking, among other phenomena, in elderly people with cognitive deficits or problems with swallowing,
- the product does not offer any danger for children and other subjects that may, due to carelessness or ignorance, consume them; on the contrary, they are totally innocuous formulations for human beings,
- the process of elaboration of the nutraceutical guarantees the conservation of nutrients, especially vitamins and proteins,
- the processing technology is simple and very flexible, it does not require complex or special equipment. The product can be prepared at home, in artisan workshops and in industry,
- it was possible to demonstrate - something not previously achieved - less oxidative lipid damage in the brain homogenate in the group of aged mice treated with the nutraceutical than that found in the young animals,
- it was possible to demonstrate a statistically significant improvement of the antioxidant activity in the group of mice treated with the nutraceutical with respect to the untreated aged mice. This other effect has not been previously described in aged animal models in a product that conjugates the components described in the present invention,
- the nutraceutical provides a technical superiority over other previously reported solutions by achieving that the cognitive status of the animals that received treatment with the nutraceutical, not only surpassed that of the aged animals, but did not show significant differences with respect to that of the young animals,
- the administration of the nutraceutical and several of its active components resulted in a reduction of blood glucose levels with respect to both young and aged animals that did not receive treatment, an aspect that surpasses the previous solutions described in the state of the art using the analytical methodology described.

## Claims

1. A solid nutraceutical composition for the prevention and treatment of cognitive impairment **characterized by** a mixture of:

    (i) an emulsion in amounts of 20 to 60 % m/m with respect to the total mass of the nutraceutical comprising:

        a. *Plukenetia volubilis* oil, from 5 % m/m to 20 % m/m
        b. aqueous, ethanolic or hydroalcoholic extracts of *Curcuma longa,* from 0.1 % m/m to 10 % m/m

c. water or an aqueous solution of one of the following components from 3 to 30 % m/m

  i. *Piper nigrum* extract
  ii. *Theobroma cacao* extract

d. emulsifying agents in amounts of 0.1 % m/m to 35 % m/m selected from among:

  i. lecithin, specifically soybean, or egg lecithin, from 0.1 % m/m to 1 % m/m
  ii. an extract of the genus *Saponaria,* more specifically of the species *Saponaria officinalis* or *Saponaria ocymoides,* 0.1 % m/m to 10 % m/m
  iii. egg white, from 3 % m/m to 35 % m/m
  iv. an extract of the genus *Agave,* specifically of *Agave americana, Agave tequilana, Agave fourcroydes* from 0.1 % m/m to 10 % m/m

e. selected sweetening agents in amounts of 1 % m/m to 30 % m/m selected among:

  i. fructooligosaccharide, in amounts from 1 % m/m to 30 % m/m
  ii. *Stevia rebaudiana* extract, in amounts of 1 % m/m to 30 % m/m
  iii. *Apis mellifera* honey, in amounts of 15 % m/m to 30 % m/m
  iv. sucrose, in quantities of 10 % m/m to 30 % m/m
  v. liquor or extract of the genus *Agave,* specifically *Agave* species, preferably *Agave tequilana,* in quantities of 10 % m/m to 30 % m/m;

f. air of 0.2 % v/v to 50 % v/v with respect to the volume of the emulsion

(ii) a mass of seeds and oils in amount of 40 %, m/m to 70 %, m/m with respect to the total mass of the nutraceutical, the components of which are selected from among:

  a. seeds of the genus *Cucurbita,* specifically *Cucurbita moschata* or *Cucurbita pepo,* in amounts of 40 %, m/m to 65 % m/m
  b. *Plukenetia volubilis* oil in quantities of 1 %, m/m to 20 % m/m

2. Nutraceutical composition according to claim 1, **characterized in that** to the mixture of the emulsion with the mass of seeds and oils *Cocos nucifera* oil is added in quantities of 5 %, m/m to 20 % m/m with respect to the total mass of the nutraceutical.

3. Nutraceutical composition according to claim 1, **characterized in that** to the mixture of the emulsion with the mass of seeds and oils *Theobroma cacao* powder is added in amounts of 0.5 %, m/m to 9 % m/m with respect to the total mass of the nutraceutical.

4. Nutraceutical composition according to claim 1, **characterized in that** to the mixture of the emulsion with the mass of seeds and oils is added a mass of fruits or other seeds, preferably *Cocos nucifera* in amounts of 1 %, m/m to 10 % m/m with respect to the total mass of the nutraceutical.

5. Nutraceutical composition according to claim 1, **characterized in that** to the mixture of the emulsion with the mass of the seeds and oils are added natural juices or fruit concentrates in quantities of 1 %, m/m to 5 % m/m with respect to the total mass of the nutraceutical.

6. Nutraceutical composition according to claims 1, **characterized in that** to the mixture of the emulsion with the mass of the seeds and oils are added protein sources in the form of isolates, concentrates, extracts or integral powders preferably from *Moringa oleifera* or whey in amounts of 1 %, m/m to 10 % m/m with respect to the total mass of the nutraceutical.

Figure 1.

Figure 2.

Figure 3.

Figure 4.

Figure 5.

Figure 6.

Figure 7.

Figure 8.

Figure 9.

Figure 10.

Figure 11.

# INTERNATIONAL SEARCH REPORT

| International application No. |
|---|
| PCT/CU2023/050003 |

**A. CLASSIFICATION OF SUBJECT MATTER**

*A61K36/00* (2006.01)
*A23L33/00* (2016.01)
According to International Patent Classification (IPC) or to both national classification and IPC

**B. FIELDS SEARCHED**

Minimum documentation searched (classification system followed by classification symbols)
A61K, A23L

Documentation searched other than minimum documentation to the extent that such documents are included in the fields searched

Electronic data base consulted during the international search (name of data base and, where practicable, search terms used)

EPODOC, INVENES, WPI, INTERNET

**C. DOCUMENTS CONSIDERED TO BE RELEVANT**

| Category* | Citation of document, with indication, where appropriate, of the relevant passages | Relevant to claim No. |
|---|---|---|
| A | WO 2006121985 A1 (BRAINSAVERS LLC) 16/11/2006, page 5, line 9 - page 9, line 5. | 1-6 |
| A | CN 109771518 A (TUNTUNMAI INC) 21/05/2019, abstract. | 1-6 |
| A | ES 2413497 B1 (WESTMAN WORLWIDE ACTIVITIES SL) 16/07/2013, abstract. | 1-6 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 16/02/2024 | **(19/02/2024)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | J. López Nieto |
| | Telephone No. 913498426 |

Form PCT/ISA/210 (second sheet) (July 2022)

## INTERNATIONAL SEARCH REPORT

Information on patent family members

International application No.

PCT/CU2023/050003

| Patent document cited in the search report | Publication date | Patent family member(s) | Publication date |
|---|---|---|---|
| WO2006121985 A1 | 16.11.2006 | US2009215885 A1 | 27.08.2009 |
| CN109771518 A | 21.05.2019 | NONE | |
| ES2413497 A1 | 16.07.2013 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6914071 B
- US 6426362 B
- DE 4303214 A1
- ES 2209539 T3
- US 5985936 A
- WO 20170065660 A
- ES 2323940
- US 6579544 B

- US 6140304 A
- US 5834044 A
- WO 2019018445 A
- US 20180325842 A1
- US 4451488 A
- WO 2006053379 A1
- WO 2006121985 A1

**Non-patent literature cited in the description**

- **C. PATTERSON**. Cognitive Impairment in the Elderly: Case Finding in Family Practice. *Can. Fam. Physician.*, March 1989, vol. 35, 621-624
- **LARSON EB** ; **KUKULL WA** ; **KATZMAN RL**. Cognitive impairment: dementia and Alzheimer's disease. *Annu Rev Public Health.*, 1992, vol. 13, 431-49
- **LARSON EB** ; **KUKULL WA** ; **KATZMAN RL**. Alzheimer's Disease and Mild Cognitive Impairment. *Neurol. Clin.*, August 2007, vol. 25 (3), 577-v
- **HAASS, C.** ; **KAETHER, C.** ; **THINAKARAN, G. S.** Sisodia Trafficking and proteolytic processing of APP. *Cold Spring Harb Perspect Med.*, 2012, 2
- **MUCKE, L.** ; **SELKOED.J.** Neurotoxicity of amyloid β-protein: Synaptic and network dysfunction. *Cold Spring Harb Perspect Med.*, 2012, 2
- **CASTELLO, M.A.** ; **SORIANO, S.** On the origin of Alzheimer's disease. Trials and tribulations of the amyloid hypothesis. *Ageing Res Rev*, 2014, vol. 13, 10-12
- On the origin of Alzheimer's disease. Trials and tribulations of the amyloid hypothesis. *Ageing Res Rev*, vol. 13, 10-12
- **DRACHMAN, D. A.** The amyloid hypothesis, time to move on: Amyloid is the downstream result, not cause, of Alzheimer's disease. *Alzheimers Dement*, 2014, vol. 10, 372-380
- **HONG-QI, Y.** ; **ZHI-KUN, S.** ; **SHENG-DI, C.** Current advances in the treatment of Alzheimer's disease: focused on considerations targeting Aβ and tau. *Trans. Neurodeg.*, 2012, vol. 1, 1-21
- **SELKOE, D. J.** ; **HARDY, J.** The amyloid hypothesis of Alzheimer's disease at 25 years. *EMBO molecular medicine*, 2016, vol. 8, 595-608

- **SERRANO M.** ; **CASCALES, M.** Resistencia a la insulina. *inflamación y obesidad*, 2015, vol. 1, 377-399
- **JAGUA, A.** ; **AVILA, A.** Insulina y enfermedad de Alzheimer: ¿una diabetes tipo 3?. *Rev Med Univ Nac Colomb.*, 2007, vol. 55 (1), 6-70
- **HU, I.** ; **ORTÍ, J. E. R.** ; **SABATER, P. S.** ; **CASTILLO S. S.** ; **ROCHINA, M. J.** Aceite de coco: tratamiento alternativo no farmacológico frente a la enfermedad de Alzheimer. Universidad Católica de Valencia. *España. Nutr Hosp.*, 2015, vol. 32 (6), ISSN 0212-1611, 2822-2827
- **FELICE F. D.** ; **LOURENCO M** ; **FERREIRA S.** How does brain insulin resistance develop in Alzheimer's disease?. *Alzheimers Dement*, 2014, vol. 10, 26-32
- **BOADA, M.** ; **RAMOS-FERNÁNDEZ, E.** ; **GUIVERNAU, B.** ; **MUÑOZ, F. J.** ; **COSTA, M.** ; **ORTIZ, A. M.** Tratamiento de la enfermedad de Alzheimer mediante terapia combinada de aféresis terapéutica y hemoféresis con albúmina e inmunoglobulina intravenosa: fundamentos y aproximación terapéutica al estudio AMBAR (Alzheimer Management by Albumin Replacement). Sociedad Española de Neurología, 2016, vol. 31, 473-481
- **WARREN E.B.** ; **MORROW E.M.** Mitochondrial Function in 22q11 Deletion Syndrome. *Neuron.*, 19 June 2019, vol. 102 (6), 1089-1091
- **JARA C.** ; **TORRES A.K.** ; **OLESEN M.A.** ; **TAPIA-ROJAS C**. *Mitochondrial Dysfunction as a Key Event during Aging: From Synaptic Failure to Memory Loss*, https://www.intechopen.com/predownload/68488

- **MANCUSO M.** ; **CALSOLARO V.** ; **ORSUCCI D.** ; **CARLESI C.** ; **CHOUB A.** ; **PIAZZA S.** ; **SICILIANO G.** Mitochondria, Cognitive Impairment, and Alzheimer's Disease. *Int J Alzheimers Dis.*, 06 July 2009, vol. 2009, 951548
- **HASSELBALCH S. G.** ; **MADSEN P. L.** ; **HAGEMAN L. P.** ; **OLSEN K. S.** ; **JUSTESEN N.** ; **HOLM S.** ; **PAULSON O. B.** Changes in cerebral blood flow and carbohydrate metabolism during acute hyperketonemia.. *American Journal of Physiology-Endocrinology and Metabolism*, vol. 270 (5)
- **VENEMAN T.** ; **MITRAKOU A.** ; **CRYER P** ; **GERICH J**. Effect of Hyperketonemia and Hyperlacticacidemia on Symptoms, Cognitive Dysfunction, and Counterregulatory Hormone Responses During Hypoglycemia in Normal Humans. *Diabetes*, November 1994, vol. 43 (11), 1311-1317
- **SLOAN, E.** The top 10 trends to watch and work on. *Food Technol.*, 1996, vol. 50 (7), 55-71
- **FIALLO, V. R. F.** ; **CASTRO, L. P. L.** Apuntes para la flora económica de Cuba III. Plantas condimenticias. *Revista del Jardín Botánico Nacional*, 2000, vol. 21 (1), 47-70, http://www.jstor.org/stable/42597069
- **JORGE A. PINO** ; **JUAN AGÜERO** ; **VICTOR FUENTES**. Chemical Composition of the Aerial Parts of Piper nigrum L. from Cuba. *Journal of Essential Oil Research*, 2003, vol. 15 (3), 209-210
- **DAMARIS DE LA C. SALGADO** ; **MAX MONAN**. Pharmacognostic and Physicochemical Studies of Theobroma cacao bean husk in Cuba. *International Invention of Scientific Journal*, 2018, vol. 2 (07), http://iisj.in/index.php/iisj/article/view/54
- **FAJARDO ROSABAL, L.** ; **FIGUEREDO DELGADO, Y.** ; **ROSABAL CORDOVÍ, U.** ; **GUARDIA PUEBLA, Y.** ; **RODRIGUEZ PÉREZ, S.** ; **SILVA-PUPO, J.** ; **VIERA TAMAYO, Y.** Contenido de polifenoles totales en callos de Theobroma cacao L. clon 'UF-650. *Biotecnologia Vegetal*, 2020, vol. 20 (1), 63-72, https://revista.ibp.co.cu/index.php/BV/article/view/656
- Escalado de la reacción de biosíntesis de fructooligosacáridos, a partir de sacarosa, en biorreactores tipo tanque agitado. **PÉREZ-CRUZ, E. R.** ; **MARTINEZ-GARCIA, D.** ; **RODRIGUEZ-RICO, I.** ; **SOBRINO-LEGÓN, A.** ; **HERNÁNDEZ, L.** Tecnologia Química. Centro de Ingenieria Genética y Biotecnología. Enzimas Industriales, 2011, vol. XXXI, 19-25
- **RODRIGUEZ GONZÁLEZ** ; **HORACIO, ACOSTA DE LA LUZ** ; **LÉRIDA L** ; **HECHEVARRÍA SOSA** ; **ISABEL, RIVERA AMITA** ; **MARIA MAGDALENA** ; **RODRIGUEZ FERRADÁ** ; **CARLOS ALBERTO** ; **SÁNCHEZ GOVÍN, ESTER** ; **MILANÉS FIGUEREDO, MASGLOIRIS**. Comportamiento del cultivo de Stevia rebaudiana (Bertoni) Bertoni en Cuba. *Revista Cubana de Plantas Medicinales*, 2007, vol. 12 (4), http://scielo.sld.cu/scielo. php?script=sci_arttext&pid=S 1028-47962007000400004&lng=es&tlng=es
- **ULLOA TRUJILLO L.** Proyecto avileño de investigación valida uso de la Stevia como edulcorante. Agencia Cubana de Noticias, 20 December 2021
- **PANEQUE ESCALONA, T.** ; **POLANCO ARIAS, M.** ; **JIMÉNEZ NOGUERAS, C.** ; **PIQUERA PALOMINO, Y.** Estudio etnofarmacológico de algunas especies endémicas de agave utilizados en la medicina tradicional. *Revista Científica y Tecnológica UPSE*, 2019, vol. 6 (2), 57-66
- **MAIDELYS et al.** Effect of diet supplementation with meal of Agave tequilana stems on hematological indicators and blood biochemistry of fattening rabbits. *Cuban Journal of Agricultural Science, [S.I.*, December 2019, vol. 53 (4), ISSN 2079-3480, http://www.cjascience.com/index.php/CJAS/article/-view/920/913
- **ESPINOSA REYES, Á.** ; **SILVA PUPO, J.** ; **BORGES GARCIA, M.** ; **FAJARDO ROSABAL, L.** ; **PÉREZ PÉREZ, J.** ; **GONZÁLEZ PANEQUE, O.** ; **SARIEGO FRÓMETA, S.** Establecimiento y multiplicación in vitro de Curcuma longa L.. *Biotecnologia Vegetal*, 2009, vol. 9 (1), https://revista.ibp.co.cu/index.php/BV/article/view/308
- **FERNÁNDEZ IZQUIERDO A.** *Cúrcuma: una reina, para Cuba y el mundo*, 16 June 2021, http://artemisadiario.cu/2021/06/curcuma-una-reina-para-cuba-y-el-mundo
- **BEATÓN RUIZ B.** *Trabajadores*, 24 April 2020, https://www.trabajadores.cu/20200424/procesadora-de-soya-de-santiago-de-cuba-estable-en-su-quehacer-productivo
- **LEMUS RODRIGUEZ** ; **MARTHA ZOE** ; **CHONG QUESADA, AMAURY** ; **BOSCH ESCOBAR, JORGE**. Tableta masticable de lecitina de soya: de subproducto a producto farmacéutico. *MEDISAN*, 2017, vol. 21 (5), 556-563, http://scielo.sld.cu/scielo.php?script=sci_arttext&pid=S1029-30192017000500007&lng=es&tlng=es
- **DE ZAYAS, A. Á.** Los agaves de Cuba Occidental. *Revista Del Jardín Botánico Nacional*, 1984, vol. 5 (3), 3-16, http://www.jstor.org/stable/42596752
- **ACEVEDO-RODRÍGUEZ, P.** ; **STRONG, M.T.** Catalogue of seed plants of the West Indies. *Smithsonian Contributions to Botany*, 2012, vol. 98, 1-1192

- **VINENT SERRANO E.** ; **FAJARDO GUTIÉRREZ O**. Estrategia para el mejoramiento genético de Agaves en Cuba. Instituto de Investigaciones Horticolas ''Liliana Dimitrova'' (IIHLD)

- **VALDIVIA, A.** ; **RUBIO FONTANILLS, Y.** ; **HERNÁNDEZ ÁLVAREZ, L.** ; **JIMÉNEZ RABELO, J.** ; **PÉREZ HERNÁNDEZ, Y.** ; **PORTILLA TUNDIDOR, Y.** Propiedades fitoquímicas y antibacterianas de los extractos de las hojas de Agave fourcroydes Lem. (henequén). *Revista Cubana de Plantas Medicinales*, 2018, vol. 23 (2), https:/Irevplantasmedicinales.sld.cu/index.php/pla/article/view/452/302

- **GONZÁLEZ DE LA TORRE, L** ; **RODRIGUEZ LEYES E.A.** ; **VICENTE MURILLO R.** ; **GONZÁLEZ CANAVACIOLO V.L** ; **DIAZ RIVERA Y. YURISLEY**. Caracterización preliminar del aceite de Plukenetia volubilis L. (sacha inchi) cultivada en Cuba. *Revista Cubana de Plantas Medicinales*, 2022, vol. 27 (1), e1227

- **ALVARADO-RUFFO K.** ; **BLANCO-IMBERT A.** ; **M. DE LA NOVAL-PONS B** ; **MARTIN-ALONSO G.M.** Nursery propagation of Cocos nucifera L. Case study: Baracoa. *Tropical Crops.*, 2018, vol. 39 (4), https://ediciones.inca.edu.cu/index.php/ediciones/article/view/1487/html

- **FIALLO, V. R. F.** Apuntes para la flora económica de Cuba VII. Especies Frutales. *Revista Del Jardín Botánico Naciona*, 2003, vol. 24 (1/2), 177-217, http://www.istor.org/stable/42597201

- **MARTÍN, C. et al.** Potential applications of Moringa oleifera. A critical review. *Pastures and Forages, [S.I.*, September 2013, vol. 36 (2), ISSN 2078-8452, https://payfo.ihatuey.cu/index.php?journal=pasto&page=article&op=view&path%5B%5D =1539

- **SARRAF P** ; **PAROHAN M** ; **JAVANBAKHT MH** ; **RANJI-BURACHALOO S** ; **DJALALI M**. Short-term curcumin supplementation enhances serum brain-derived neurotrophic factor in adult men and women: a systematic review and dose-response meta-analysis of randomized controlled trials. *Nutr Res.*, 09 May 2019, vol. 69, 1-8

- **FRANCO-ROBLES E** ; **CAMPOS-CERVANTES A** ; **MURILLO-ORTIZ BO** ; **SEGOVIA J** ; **LÓPEZ-BRIONES S** ; **VERGARA P** ; **PÉREZ-VÁZQUEZ V** ; **SOLÍS-ORTIZ MS** ; **RAMIREZ-EMILIANO J**. Effects of curcumin on brain-derived neurotrophic factor levels and oxidative damage in obesity and diabetes.. *Appl Physiol Nutr Metab.*, 23 August 2013, vol. 39 (2), 211-8

- **MAZZANTI G.** ; **DI GIACOMO S**. Curcumin and Resveratrol in the Management of Cognitive Disorders: What Is the Clinical Evidence?. *Molecules*, 2016, vol. 21, 1243

- **ABAD, ROMINA DEL CARMEN** ; **VASENA, MARIA CATALINA**. *Fresh turmeric: chemical-nutritional composition, use in bakery product and sensory evaluation*, 2020, https://rdu.unc.edu.ar/bitstream/-handle/11086/18668/tesis%20completa%201462. pd f?sequence=1&isAllowed=y

- **GHOSH T.S.** ; **RAMPELLI S.** ; **JEFFERY I.B.** ; **SANTORO A.** ; **NETO M.** ; **CAPRI M.** ; **GIAMPIERI E.** ; **JENNINGS A.** ; **CANDELA M.** ; **TURRONI S. et al.** Mediterranean diet intervention alters the gut microbiome in older people reducing frailty and improving health status: The NU-AGE 1-year dietary intervention across five European countries. *Gut.*, 2020, vol. 69, 1218-1228

- **LEMUS-MONDACA, ROBERTO** ; **MARIN, JESSAMI** ; **RIVAS, JOSEFA** ; **SANHUEZA, LEYLA** ; **SOTO, YASNA** ; **VERA, NATALIA** ; **PUENTE-DÍAZ, LUIS**. Pumpkin seeds (Cucurbita maxima). A review of functional attributes and by-products. *Revista chilena de nutrición*, 2019, vol. 46 (6), 783-791, https://dx.doi.org/10.4067/S0717-75182019000600783

- **DAVID L. KATZ** ; **KIM DOUGHTY** ; **ATHER ALI**. Cocoa and Chocolate in Human Health and Disease. *Antioxidants & Redox Signaling.*, 15 November 2011, vol. 15 (10), 2779-2811

- **MYHRE O** ; **UTKILEN H** ; **DUALE N** ; **BRUNBORG G** ; **HOFER T**. Metal Dyshomeostasis and Inflammation in Alzheimer's and Parkinson's Diseases: Possible Impact of Environmental Exposures. *Oxidative Medicine and Cellular Longevity*, 2013

- **ZOU L.** ; **LIU, W.** ; **LIU, C.** ; **XIAOB, H.** ; **MCCLEMENTS D. J.** Designing excipient emulsions to increase nutraceutical bioavailability: emulsifier type influences curcumin stability and bioaccessibility by altering gastrointestinal fate. The Royal Society of Chemistre, 2015

- **UWE, M.** ; **J. VON HAGEN**. Isolation of subcellular organelles and structures. *Methods Enz.*, 2009, vol. 463, 305-326

- **OKAWA, O.** ; **N. OHISHI** ; **K. YAGI**. Assay of lipid peroxides in animal tissues by the thiobarbituric acid reaction. *Anal Biochem.*, 1979, vol. 95 (2), 351-58

- **UPRETI, G.C.** ; **R.A. RATCLIFF** ; **RICHES P.C.** Protein Estimation in Tissues Containing High Levels of Lipid: Modifications to Lowry's Method of Protein Determination. *Analytical Biochemestry.*, 1988, vol. 168, 421-427

- **MYHRE, O.** ; **H. UTKILEN** ; **N. DUALE** ; **G. BRUNBORG** ; **T. HOFER**. *Metal dyshomeostasis and Inflammation in Alzheimer's and Parkinson's diseases: Possible impact of environmental exposure*, 2013, http://dx.doi.org/10.1155/2013/726954

- **HERING, H.** ; **C. C. LIN** ; **M. SHENG**. Lipid rafts in the maintenance of synapses, dendritic spins, and surface AMPA receiver stability. *J Neurosci*, 2003, vol. 23 (8), 62-71

- **R. D'HOOGE** ; **P.P. DE DE DEYN**. Applications of the Morris water maze in the study of learning and memory. *Brain Research Reviews*, 2001, vol. 36, 60-90
- **CUNNANE SC** ; **NUGENT S** ; **ROY M** ; **COURCH-ESNE-LOYER A** ; **CROTEAU E et al.** Brain fuel metabolism, aging and Alzheimer's disease. *Nutrition.*, 2011, vol. 27 (1), 3-20
- **PROITSI P** ; **LUPTON MK.** ; **VELAYUDHAN L** ; **NEWHOUSE S** ; **FOGH I** ; **TSOLAKI M et al.** Genetic Predisposition to Increased Blood Cholesterol and Triglyceride Lipid Levels and Risk of Alzheimer Disease: A Mendelian Randomization Analysis. *PLOS Medicine*, 2014, vol. 11 (9), e1001713
- **MOGIL, J.S.** ; **WILSON, S.G.** ; **BON, K.** ; **LEE, S.E.** ; **CHUNG, K.** ; **RABER, P.** ; **PIEPER, J.O.** ; **HAIN, H.S.** ; **BELKNAP, J.K.** ; **HUBERT, L. et al.** Heritability of nociception i: Responses of 11 inbred mouse strains on 12 measures of nociception. *Pain*, 1999, vol. 80, 67-82
- **KING-HIMMELREICH TS.** ; **MÖSER CV.** ; **WOLTERS M.** ; **OLBRICH K** ; **GEISSLINGER G** ; **NIEDERBERGER E**. Age-Dependent Changes in the Inflammatory Nociceptive Behavior of Mice. *Int. J. Mol. Sci.*, 2015, vol. 16, 27508-27519